# EUROPEAN PATENT SPECIFICATION

(11) **EP 1 169 642 B1**
(45) Date of publication and mention of the grant of the patent: **04.01.2006**
(21) Application number: 00923174.7
(22) Date of filing: 07.04.2000
(51) Int. Cl.: G01N 33/53, C12N 15/90, C12N 5/12, C07K 16/18, C07K 14/51

(54) **COMPLEX FORMED BY N-LINKED GLYCOPROTEINS (SIBLINGS) AND FACTOR H**
KOMPLEX AUS N-GLYKOLISIERTEN PROTEINEN (SIBLINGS) UND FAKTOR H
COMPLEXE FORME PAR DES GLYCOPROTEINES (SOEURS) A LIAISON N, ET LE FACTEUR H

(30) Priority: 09.04.1999 US 128468 P
(43) Date of publication of application: 09.01.2002
(73) Proprietor: THE GOVERNMENT OF THE UNITED STATES OF AMERICA, as represented by THE SECRETARY, DEPARTMENT OF HEALTH AND HUMAN SERVICES, Rockville, MD 20852 (US)
(72) Inventor: FISHER, Larry, W., Derwood, MD 20855 (US); FEDARKO, Neal, S., Columbia, MD 21045 (US); YOUNG, Marian, F., Silver Spring, MD 20910 (US)
(74) Representative: Gowshall, Jonathan Vallance
(86) International application number: PCT/US2000/009349
(87) International publication number: WO 2000/062065

(56) References cited:
- US-A- 5 340 934
- BELLAHCENE ET AL: "Expression of bone matrix proteins in human breast cancer: Potential roles in microcalcification formation and in th genesis of bone metastases" BULLETIN DU CANCER,XX,XX, vol. 84, no. 1, January 1997 (1997-01), pages 17-24, XP002107803
- PRITCHARD ET AL: "Is the expression of osteopontin and bone sialoprotein greater in breast cancer bone metastases compared to other metastatic sites" EUROPEAN SYMPOSIUM ON CALCIFIED TISSUES,XX,XX, vol. 20, no. 4S, 25 April 1997 (1997-04-25), page 63S XP002107804
- PATENT ABSTRACTS OF JAPAN vol. 1997, no. 09, 30 September 1997 (1997-09-30) & JP 09 138213 A (SANGI CO LTD), 27 May 1997 (1997-05-27)
- FISHER, L.W. ET AL.: "Antisera and cDNA probes to human and certain animal model bone matrix noncollagenous proteins" ACTA ORTHOPAEDICA SCANDINAVICA, vol. 266, 1995, pages 61-65, XP000937776
- FEDARKO, NEAL S. ET AL: "Factor H binding to bone sialoprotein and osteopontin enables tumor cell evasion of complement-mediated attack" J. BIOL. CHEM., 275(22), 16666-16672 , 2 June 2000 (2000-06-02), XP000938681
- FEDARKO, N. S. (1) ET AL: "Integrins and factor H mediate bone sialoprotein 's and osteopontin's protective properties in complement attack on susceptible cancer cells." JOURNAL OF BONE AND MINERAL RESEARCH, (SEPT., 1999) VOL. 14, NO. SUPPL. 1 PP. S187. MEETING INFO.: TWENTY-FIRST ANNUAL MEETING OF THE AMERICAN SOCIETY FOR BONE AND MINERAL RESEARCH ST. LOUIS, MISSOURI, USA SEPTEMBER 30-OCTOBER 4 1999 AMERICAN SOCIETY, XP000937682
- SPRINGER SEMINARS IN IMMUNOPATHOLOGY, vol. 15, 1994, pages 417-431,

## Description

### FIELD OF THE INVENTION

The present invention relates to methods of detecting and utilizing small integrin-binding ligand, N-linked glycoproteins (SIBLINGS), as well as the production and use of recombinant SIBLINGS proteins that can be used as controls for such detection methods.

### BACKGROUND OF THE INVENTION

### A. Factor H

Factor H, a 150-kDa protein, is a key regulatory braking mechanism in normal and alternate complement-mediated cell lysis. It dissociates and thereby inactivates the assembled C3 convertase, serves as an essential accelerator of Factor I-mediated cleavage of C3b to iC3b, and sterically inhibits C5 binding to C3b (a prerequisite step for terminal pathway activation). The salient structural features of Factor H include 20 short consensus repeats (SCRs) that contain four cysteine residues forming two disulfide bonds per repeat. In addition, each SCR contains at least one conserved tryptophan residue per repeat, and Factor H is known to interact with several sialic acid-containing proteins.

### B. Molecular Characterization of BSP, A Member of the SIBLINGS Protein Family

Bone sialoprotein (BSP), also briefly known as BSPII, is a phosphorylated and sulfated glycoprotein that is associated with most normal and many pathological mineralized matrices. It is a small (approximate Mr=75,000) integrin-binding protein that supports cell attachment *in vitro* through both RGD-dependent and RGD-independent mechanisms and has a high affinity for hydroxyapatite. BSP is a member of the family of small integrin-binding ligand, N-linked glycoproteins (SIBLINGS) family that also includes osteopontin (OPN), dentin matrix protein (DMP1) and dentin sialophosphoprotein (DSPP). All have similar gene structures and are clustered on human chromosome 4.

Bone sialoprotein (BSP) constitutes about 10-15% of the non-collagenous proteins found in the mineralized compartment of young bone (Fisher et el., *J*. *Biol. Chem.* **258**:12723-727, 1983). Immunolocalization and *in situ* studies have shown BSP to be produced by osteoblasts, osteocytes, and osteoclasts (the multinucleated cells that resorb bone) (Bianco et al., *Calcif. Tissue Int.* **49**:421-426, 1991). The areas richest in BSP are the collagen-poor matrix found between areas of new bone, where bone is developing or turning over. Outside of bone, BSP has been found in other mineralized tissues, such as dentin (Fisher et al., *J Biol. Chem.* **258**:12723-727. 1983), cementum (MacNeil et al., *J. Bone Min. Res.* **9**:1597-1606, 1994), and calcifying cartilage of the growth plate (Bianco et al., *Calcif. Tissue Int.* **49**:421-426, 1991). Trophoblasts of the developing placenta also express high levels of BSP (Bianco et al., *Calcified Tissue International,* **49**:421-426, 1991). While placental tissue is not usually considered to be a mineralized tissue, late term human placentas have hydroxyapatite crystals associated with the aging trophoblasts.

Using human BSP as a model (Fisher et al., *J. Biol. Chem*. **265**:2347-2351, 1990), the protein is first made as a 317 amino acid, 35,000 Da protein. A 16 amino acid leader peptide is removed during synthesis. BSP has no disulfide bonds and it is nearly uniformly hydrophilic along its length, indicating that the protein is likely to be an extended rod in solution. There are three regions particularly rich in glutamic acids residues ("polyglutamic acid domains") that have long been thought to govern the high affinity of this protein for hydroxyapatite. Recent work with recombinant fragments, however, shows that BSP's ability to bind strongly to apatite is found throughout its length (Stubbs et al., *Bone Miner. Res.* **12**:1210-1222, 1997).

Human BSP contains four consensus sequences for N-linked oligosaccharides, three of which are conserved for all mammalian species known to date. These N-linked and the many O-linked oligosaccharides make up approximately 50% of the mass of BSP as it is secreted into the human bone matrix (Fisher et al., *J Biol. Chem.* **258**:12723-727, 1983). Tyrosine sulfation and serine/threonine phosphorylation make up the remainder of the known post translational modifications. There are three tyrosine-rich domains in BSP, the last two of which flank the RGD domain and are subject to sulfation. The presence or absence of the sulfate groups does not appear to change the ability of fibroblasts to attach in a simple *in vitro* assay (Mintz et al., *J. Biol. Chem*. **269**:4845-4852, 1994).

The cDNA BSP sequences for rat (Oldberg et al., *J. Biol. Chem*. **263**:19430-19432, 1988), human (Fisher et al., *J. Biol. Chem*. **265**:2347-2351, 1990), mouse (Young et al. , *Mamm. Genome* **5**:108-111, 1994), cow (Chenu et al., *J. Bone Miner. Res.* **9**:417-421, 1994), hamster (Sasaguri et al., Direct submission to GenBank, Accession number U65889, 1996) and chicken (Yang et al., *J. Bone Miner. Res.* **10**:632-640, 1995) have been published. The human (Kerr, J.M., Fisher, L.W., Termine, J.D., Wang, M.G., McBride, O.W. and Young, M.F. *Genomics* **17**:408-415, 1993) and chicken (Yang, R. and Gerstenfeld, L.C., *J. Cell. Biochem.* **64**:77-93, 1997) genes have also been published. The human IBSP gene maps very close to two other members of this family, within 340 kb of SPPI (osteopontin), and within 150 kb of DMP1 with the order being: cen-DMPl-IBSP-SPPI-tel on chromosome 4 (Aplin et al., *Genomics* **30**:347-349, 1995; Crosby et al., *Genome* **7**:149-151, 1996). Mouse Ibsp is on the homologous region of chromosome 5 at 56.0 (Young et al. , *Mamm. Genome* **5**:108-111, 1994). Other members of the family are also encoded on chromosome 4, for example dentin phosphoprotein and dentin sialoprotein (DSPP) are cleavage products expressed from a single transcript coded by a gene on human chromosome 4 (MacDougall et al., J Biol. Chem. 272(2):835, 1997).

### C. Detection of Human BSP

The detection of human BSP in biological samples has been accomplished using polyclonal antibodies directed towards denatured whole BSP, non-denatured whole BSP, or synthetic fragments of BSP. Certain tumors have been found to ectopically express BSP. For example, BSP has been found to be expressed by breast cancer tissue, prostate cancer tissue, lung cancer tissue and thyroid cancer tissue (Bellahcene et al., *Cancer Research* **54**:823-826, 1994; Bellahcene et al., *Calcif. Tissue Int.* **61**:183-188, 1998; Bellahcene et al., *Calcif. Tissue Int.* **61**:183-188, 1997; and Bellahcene et al., *Thyroid* **8**:637-641, 1998 respectively). Additional studies have shown that BSP mRNA levels are increased in human breast cancer tissue as well as cell lines derived from breast cancer tissue (Bellachcene et al., *Laboratory Investigation* **75**:203-210, 1996).

The detection of BSP in various tissue samples described above has been accomplished through the use of polyclonal antibodies directed to either whole human BSP (LF-6) or synthetic fragments of human BSP, such as the synthetic fragment comprising amino acids 277-294 (LF-83).

An increased level of BSP in serum has been correlated with the presence of hyperparathyroidism, Paget's disease, multiple myeloma and breast cancer (Seibel, et al., *J. Clinical Endocrinology and Metabolism*, **81**:3289-294, 1996). Elevated levels of serum BSP have also been detected in subjects suffering from rheumatoid arthritis (Mansson et al., *J. Clin. Invest.* **95**:1071-1077, 1995).

US-A-5 340 934 relates to a sequence for human BSP. Fedarko et al, Journal of Bone and Mineral Research, 14, Suppl No 1,88187 (1999) relates to use of BSP to protect tumor cells from the lytic pathway of complement.

### SUMMARY OF THE INVENTION

The present invention stems from the discovery that members of the SIBLINGS family of proteins bind to Factor H and that these proteins can confer resistance to complement mediated lysis. This discovery also allows for the creation of assays that more accurately determine the total quantity of SIBLINGS proteins in samples, and for the creation of methods of utilizing SIBLINGS proteins to inhibit complement formation.

The present invention also takes advantage of the recognition that the population of BSP (one member of the SIBLINGS family of proteins) in serum contains two distinct sub-populations. The first sub-population of BSP is relatively non-acidic, and it is the predominant sub-population found in normal subjects. The second sub-population of BSP is relatively acidic and it is found predominantly in subjects with various types of tumors. Accordingly, the invention also provides methods of detecting the relatively acidic BSP and the relatively non-acidic BSP. Furthermore, these methods can be practiced for example, by using monoclonal antibodies specific for either the relatively acidic BSP or by simply separating the different forms of BSP based upon their ionic charges.

One aspect of the invention involves a method, according to claim 1, of detecting at least one member of the SIBLINGS family of proteins in a manner that Factor H does not inhibit the detection of the SIBLINGS protein. The detection of the SIBLINGS protein can *in vitro*, for example in the context of a sample.

In another embodiment the invention provides methods of detecting the SIBLINGS protein by separating the SIBLINGS protein from the SIBLINGS/Factor H complex and then detecting the SIBLINGS protein. Separation of the SIBLINGS protein from Factor H can be accomplished in a variety of ways, for example by heating the sample in the presence of reducing agents such as DTT (1,4-dithiothreitol) and β-mercaptoethanol. These methods can also incorporate the use of denaturants such as urea, SDS, and formamide.

Factor H can also be separated from the SIBLINGS protein based upon the differences between the ionic charge of the SIBLINGS protein and Factor H. Factor H can also be separated from the SIBLINGS protein by reacting a SIBLINGS/Factor H complex with a specific binding agent.

The specific binding agent can be specific for the portion of the SIBLINGS protein that is exposed in the SIBLINGS/Factor H complex or it can be specific for the SIBLINGS/Factor H complex itself. When the specific binding agent is specific for the SIBLINGS/Factor H complex itself it is not necessary to separate the SIBLINGS protein from Factor H prior to detection. Furthermore, in some instances the specific binding agent can serve to separate and identify the SIBLINGS protein simultaneously.

The methods provided by the invention are particularly useful for screening for the presence or the recurrence of a tumor, such as a liquid tumor, prostate tumor, thyroid tumor, lung tumor, or breast tumor that is associated with abnormal SIBLINGS protein production. The methods can be practiced for example, by testing samples of body fluids, such as blood, serum, or saliva. In some instances the tumor may produce a relatively acidic SIBLINGS protein, such as BSP, and therefore, the invention provides methods of detecting the relatively acidic BSP population.

In another embodiment of the invention the SIBLINGS protein can be detected in a sample from a subject that is suspected of having abnormal bone turnover, for example abnormal bone turnover associated with osteoporosis.

These and other aspects of the invention will become readily apparent in light of the description provided below.

### BRIEF DESCRIPTION OF THE FIGURES

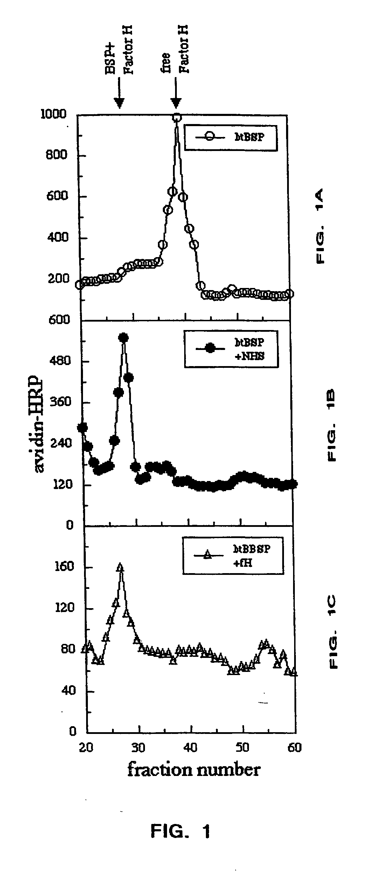

**Figs. 1A-C** are graphs which show the binding of BSP to Factor H as detected with HP-conjugated avidin after separation on a molecular sieve column. Fig. 1A shows a chromatograph of biotinylated BSP. Fig. 1B shows biotinylated BSP preincubated with normal human serum, and Fig. 1C shows biotinylated BSP preincubated with Factor H.

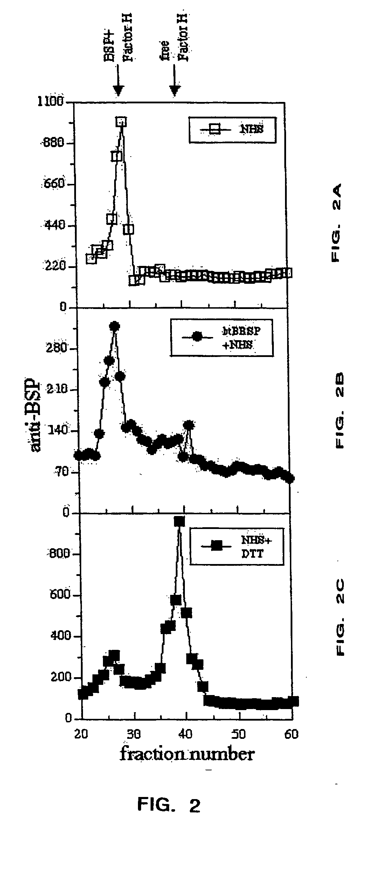

**Figs. 2A-C** are graphs which show the separation of BSP from Factor H after treatment with formamide, DTT, and heat, as detected with LF-100 antisera after separation on a molecular sieve column. Figure 2A shows a chromatograph of normal human serum (NHS). Figure 2B shows biotinylated BSP-(btBSP) incubated with normal human serum and Fig. 2C shows normal human serum after incubation with DTT in the presence of formamide and heat.

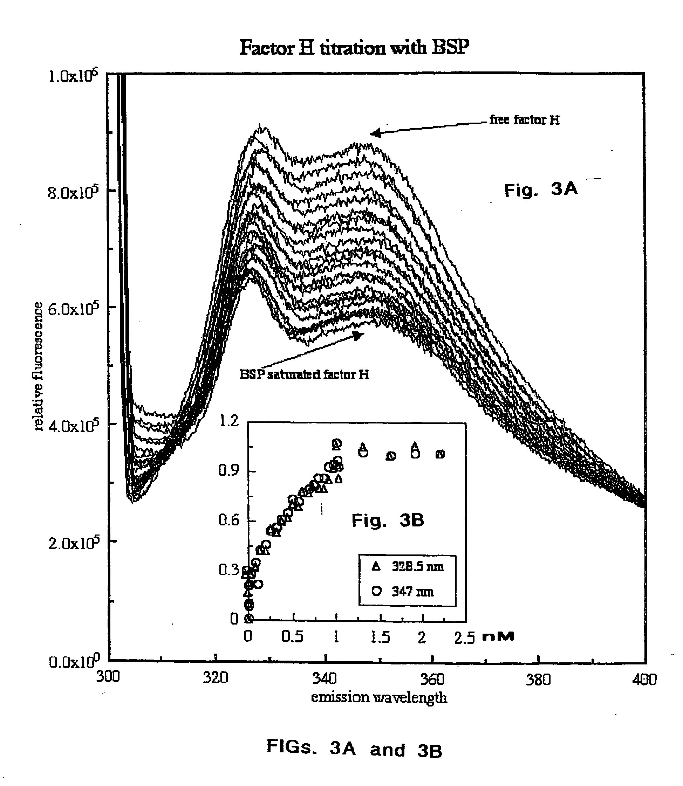

**Figs. 3A and 3B** are graphs which show the result of a titration of Factor H with BSP. Fig. 3A shows relative fluorescence of Factor H, and Factor H complexed with BSP. Fig. 3B shows the fractional acceptor saturation (fₐ) vs. the molar ratio of BSP to Factor H (Cₛ)_{'} the latter of which was constant throughout the assay. This figure shows that BSP binds 1:1 with Factor H and has a binding constant of ≤ 1 nM. Similar results were seen with OPN and DMP1.

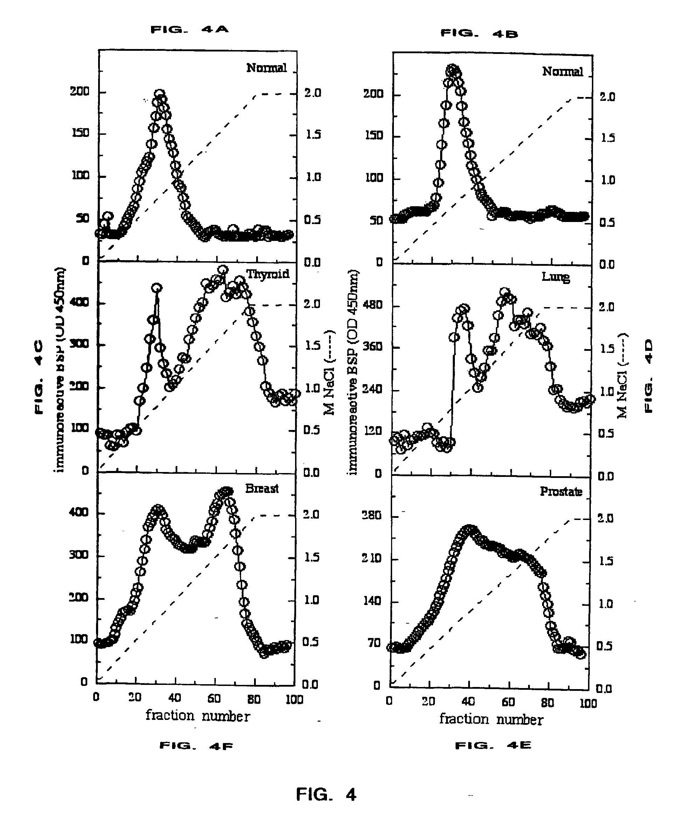

**Figs. 4A-F** are graphs which show profiles of serum samples generated by separating the two sub-populations of BSP on a Toyopearl QAE column. Fig. 4A and B show two normal sera, Fig. 4C shows the profile from a subject known to have a thyroid tumor. Fig. 4D shows the profile from a subject known to have a lung tumor. Fig. 4E shows the profile from a subject known have a breast tumor, and Fig. 4F shows the profile from a subject known to have a prostate tumor.

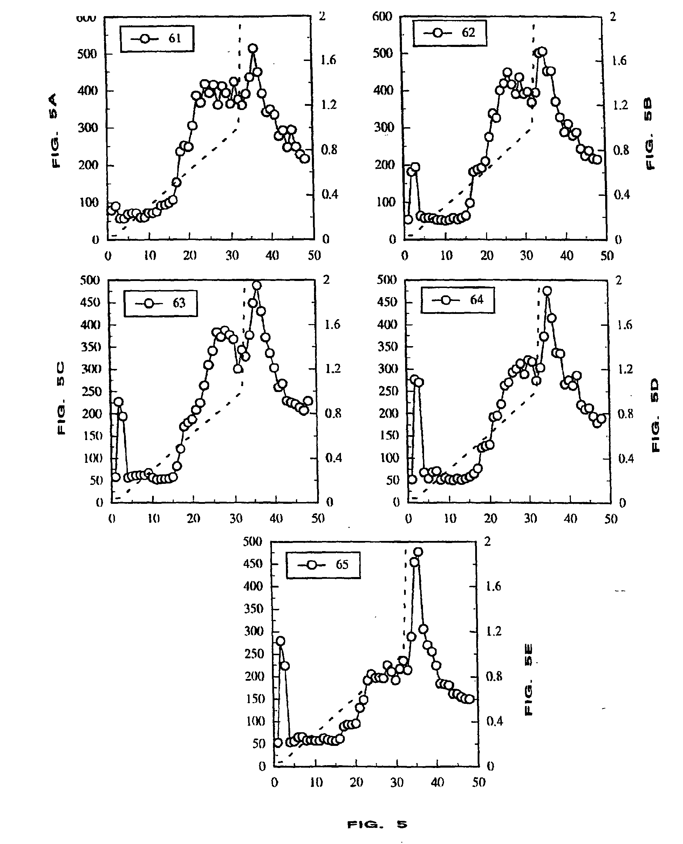

**Figs. 5A-E:** show profiles from 5 different subjects that were known to have breast cancer.

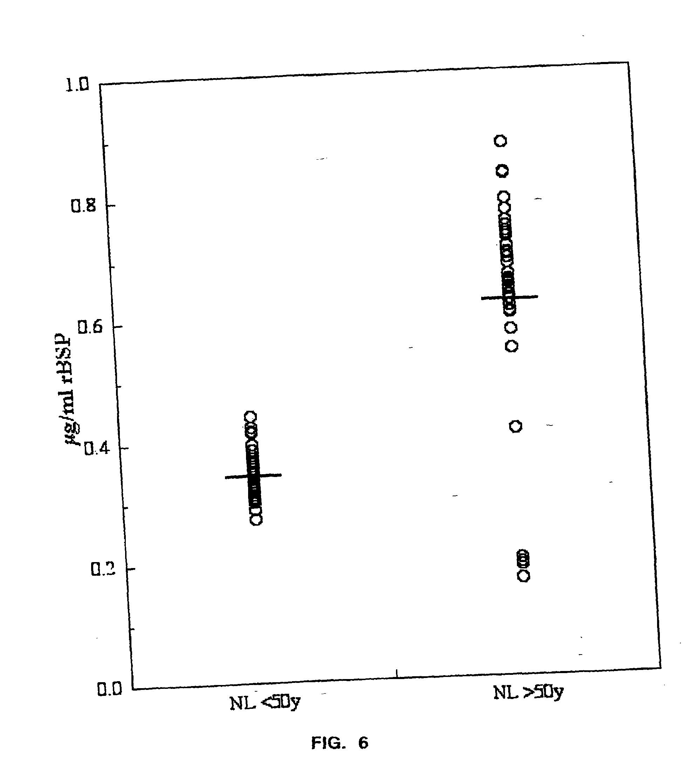

**Fig. 6** shows the level of serum BSP detected in samples taken from 32 individuals under the age of 50, and the serum BSP levels from 36 individuals over the age of 50.

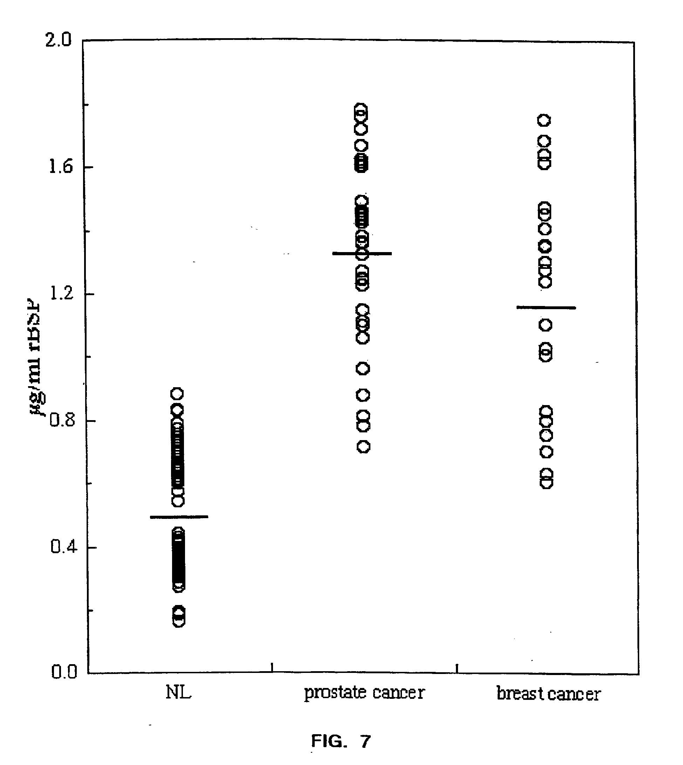

**Fig. 7** shows the level of serum BSP detected in samples taken from normal subjects, subjects known to have prostate cancer, and subjects known to have breast cancer.

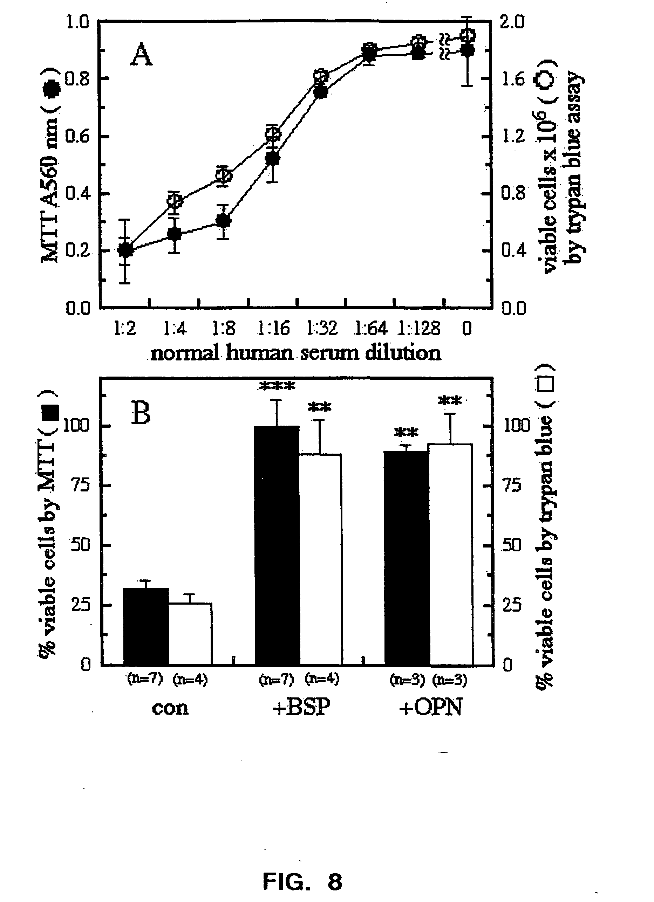

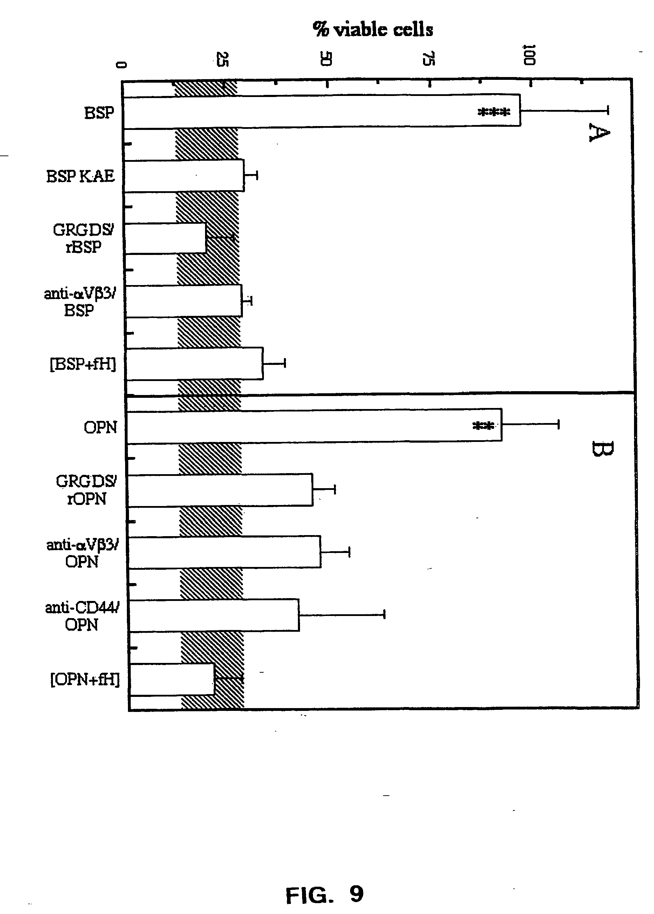

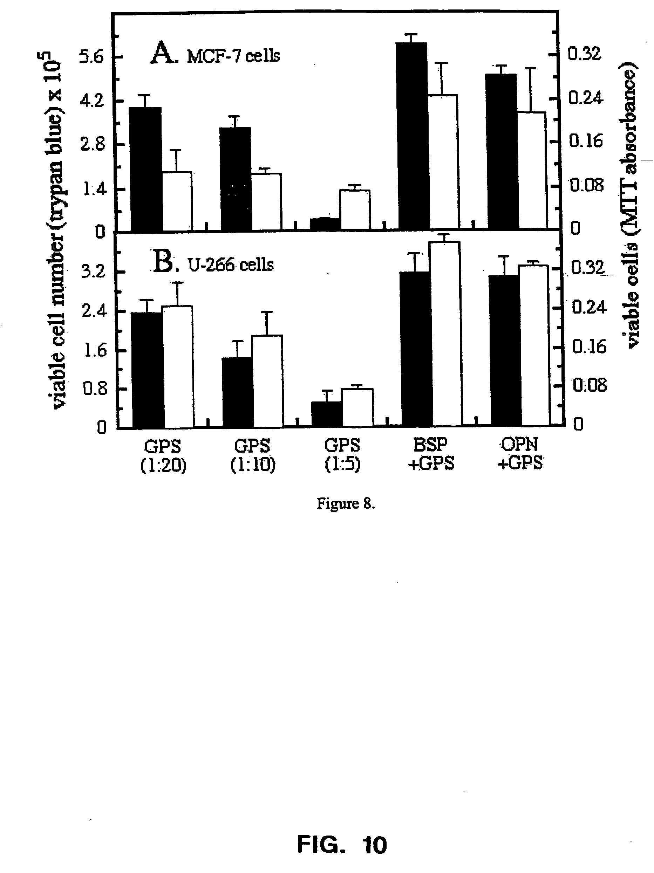

**Figs. 8A and 8B** are graphs which show that recombinantly produced BSP and OPN protect MEL cells from complement mediated lysis. MEL cells were rinsed three times with GVB-MgEGTA buffer, resuspended in GVB-MgEGTA at a density of 5 x 10⁶ cells/mL, and incubated at 37 ° C with different concentrations of normal human serum. After 2 hrs, cells were harvested for trypan blue exclusion assay. The thiazolium blue assay was carried out at identical serum dilutions and cell viability was determined by absorbance at 560 nm. Each data point represents the average of three measurements. The error bars represent the standard deviation of the mean (A). MEL cells in GVB-MgEGTA buffer were incubated with 10 µg/mL of either rBSP or rOPN for 10 minutes at 37°C. Normal human serum was then added at a dilution of 1:10 and the cells were returned to 37°C, incubated for 2 hours and cell viability was determined by trypan blue and thiazolyl blue (MTT) reduction assays (B). Data from 3-7 separate experiments, with treatment replicates in triplicate, was combined to yield mean values. Error bars represent the standard error of the mean. N = number of experiments combined.
**Fig. 9** is a bar graph which shows the involvement of integrin and CD44 in protection from complement mediated lysis. MEL cells prepared as in Fig. 8 were treated with normal rBSP (10 µg/mL), rBSP whose RGD sequence had been mutated to KAE (10 µg/mL), and either GRGDS (SEQ ID NO: 10) peptide (400 µM) followed by rBSP or an αVβ3 antibody (1:4000) followed by rBSP for 10 minutes prior to the addition of normal human serum. The cells were then incubated for 10 minutes after which cell viability determined using the MTT assay (A). A cohort of MEL cells were pretreated with rOPN (10 µg/mL) alone, GRGDS (SEQ ID NO: 10) peptide followed by rOPN, the αVβ3 antibody followed by rOPN, or an anti-CD44 antibody (Chemicon, Co.) followed by rOPN, or hyaluronan (HA) followed by rOPN (B). Cells were then treated with normal human serum and viability assayed as in Fig. 8. Treatment of MEL cells with a pre-formed complex of either BSP-Factor H ([BSP+fH]) or OPN-Factor H ([OPN+fH]) abolished the protection from 23 complement-mediated lysis. The data represents the mean and standard error of the mean for three separate experiments. Statistical significance was determined by analysis of variance. Percent cell viability was determined using A560 absorbance values of various conditions and a control where no serum had been added (100 % viable). The cross-hatched region represents that range of values observed when normal human serum (1:10) alone was added (maximal cell death).
**Figs 10A and 10B** are bar graphs which show that human cancer cell lines can be protected from complement mediated lysis through the addition of SIBLINGS proteins. Human cancer cell lines, MCF-7 (A) and U-266 (B) cells were rinsed three time with GVB-MgEGTA buffer and subsequently treated exactly as in the MEL cell complement-mediated cell lysis assay described above except complement active guinea pig serum (GPS) was substituted for human serum. Three different dilutions of GPS were assayed. For cultures pretreated with 10 µg/mL BSP or OPN, the dilution of GPS used was 1:5. Cell viability was determined by both trypan blue exclusion (solid bar) and MTT (open bar) assay. The bars represent the mean values of triplicate samples and the error bars are the standard deviation values.

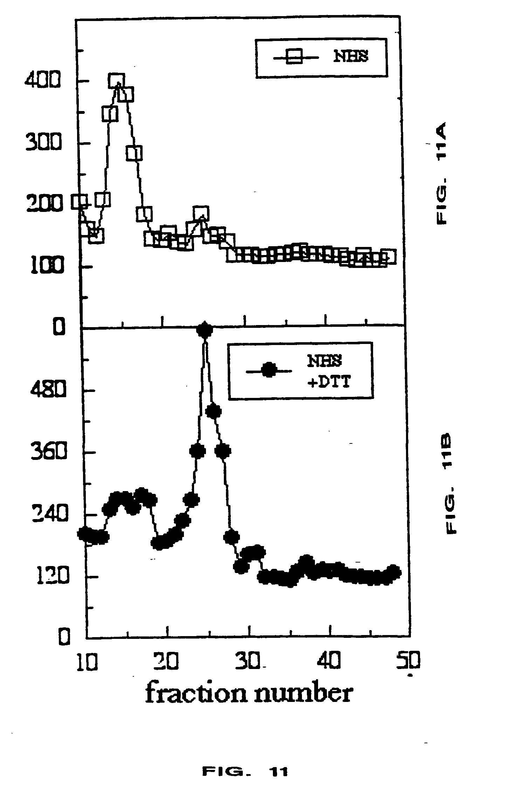

**Figs 11A and 11B** are bar graphs which show the separation of DMP1 from Factor H on a molecular sieve column after treatment with formanide, DTT, and heat, as detected by DMP1 specific polyclonal antisera. Fig. 11A shows normal human serum (NHS) and Fig. 11B shows NHS that has been treated.

### SEQUENCE LISTINGS

The nucleic and amino acid sequences listed in the accompanying sequence listing are shown using standard letter abbreviations for nucleotide bases, and three letter code for amino acids. Only one strand of each nucleic acid sequence is shown, but the complementary strand is understood as included by any reference to the displayed strand.

**SEQ ID NO: 1:** shows the amino acid sequence of human BSP.

**SEQ ID NOS: 2-7:** show various amino acid sequences which have been used as immunogens for the creation of BSP specific antibodies.

**SEQ ID NO: 8:** shows a cDNA sequence encoding human BSP.

**SEQ ID NO: 9:** shows an amino acid sequence that was used as an immunogen for the creation of BSP specific antibodies.

**SEQ ID NO: 10:** shows the amino acid sequence of a peptide that was used to compete with proteins from the SIBLINGS family of proteins for integrin type binding.

**SEQ ID NO: 11:** shows the nucleic acid sequence of an OPN encoding sequence (Accesion No. J04765).

**SEQ ID NO: 12:** shows the amino acid sequence derived from the sequence shown SEQ ID NO:11.

**SEQ ID NO: 13:** shows the nucleic acid sequence of an OPN encoding sequence (Accesion No. X13694).

**SEQ ID NO: 14:** shows the amino acid sequence derived from the sequence shown SEQ ID NO:13.

**SEQ ID NO: 15:** shows the nucleic acid sequence of a DMP1 encoding sequence (Accession No. NM_004407).

**SEQ ID NO: 16:** shows the amino acid sequence derived from the sequence shown in SEQ ID NO: 15.

**SEQ ID NO: 17:** shows the nucleic acid sequence of a DSPP encoding sequence (Accession No. AF163151).

**SEQ ID NO: 18:** shows the amino acid sequence derived from the sequence shown in SEQ ID NO: 17.

### DETAILED DESCRIPTION

### I. Abbreviations and Definitions

**Abnormal**: Deviation from normal characteristics. Normal characteristics can be found in a control, a standard for a population, etc. For instance, where the abnormal condition is a disease condition, such as a tumor, a few appropriate sources of normal characteristics might include an individual who is not suffering from the disease (*e*.*g*., breast cancer), a population standard of individuals believed not to be suffering from the disease, etc.

Likewise, abnormal may refer to a condition that is associated with a disease. The term "associated with" includes an increased risk of developing the disease as well as the disease itself. For instance, a certain abnormality (such as an increase in the expression of one or more SIBLINGS proteins) can be described as being associated with the biological condition of breast cancer; thus, the abnormality is predictive both of an increased risk of developing breast cancer and of the presence of breast cancer.

Abnormal protein expression, such as abnormal SIBLINGS protein expression, refers to expression of a protein that is in some manner different from expression of the protein in a normal (wildtype) situation. This includes but is not necessarily limited to: (1) a mutation in the protein such that one or more of the amino acid residues is different; (2) a short deletion or addition of one or a few amino acid residues to the sequence of the protein; (3) a longer deletion or addition of amino acid residues, such that an entire protein domain or sub-domain is removed or added; (4) expression of an increased amount of the protein, compared to a control or standard amount; (5) expression of an decreased amount of the protein, compared to a control or standard amount; (6) alteration of the subcellular localization or targeting of the protein; (7) alteration of the temporally regulated expression of the protein (such that the protein is expressed when it normally would not be, or alternatively is not expressed when it normally would be); (8) alteration in post translational processing; and (9) alteration of the localized *(e.g.,* organ or tissue specific) expression of the protein (such that the protein is not expressed where it would normally be expressed or is expressed where it normally would not be expressed), each compared to a control or standard.

Controls or standards appropriate for comparison to a sample, for the determination of abnormality, include samples believed to be normal as well as laboratory values, even though possibly arbitrarily set, keeping in mind that such values may vary from laboratory to laboratory. Laboratory standards and values may be set based on a known or determined population value and may be supplied in the format of a graph or table that permits easy comparison of measured, experimentally determined values.

**Implant:** A non-self object that is placed in contact with a subject. For example, the object can be an artificial limb, an intraosseous, or intravascular implant or a dental prosthesis. Such objects can comprise molecules that are generated through biosynthetic pathways, such as hydroxyapatite, or collagen, or the object can contain molecules that are generated through non-biosynthetic means, such as metal alloys or plastics. Furthermore, such objects can contain a mixture of molecules such that some of the molecules are biosynthetically generated and some of the molecules are generated through non-biosynthetic means.

**Small Integrin-Binding Ligand, N-Linked Glycoproteins (SIBLINGS):** The family of secreted phophoproteins that includes, for example, bone sailoprotein (BSP), osteopontin (OSP), dentin matrix protein (DMP1), and dentin sialophosphoprotein (DSPP). It is also likely that other member of the SIBLINGS family of proteins will be identified in the future, and that these additional members of the SIBLINGS family will also bind to Factor H. Hence, when the term SIBLINGS protein is used it refers to not only to the four family members described above, but also to additional family members that will be identified in the future.

The genes encoding the four members of the SIBLINGS family are located on human chromosome 4. Members of the SIBLINGS family of protein are shown, *infra,* to be capable of binding Factor H. The SIBLINGS/Factor H complex can cause the SIBLINGS protein to be nearly undetectable unless the complex is first disrupted. Hence, the discovery that members of the SIBLINGS family of proteins binds to Factor H allows for more accurate and sensitive quantification of the concentration of SIBLINGS proteins. The discovery that SIBLINGS proteins bind Factor H has also led to the development of methods of using SIBLINGS proteins to selectively protect cells from complement mediated lysis.

**Biologically Active SIBLINGS Proteins:** Biologically active SIBLINGS proteins are characterized by their ability to protect cells from complement mediated lysis. This activity can be readily assessed using the MEL cell assay described below. These proteins can be "derived" from SIBLINGS proteins such that the endogenous SIBLINGS protein's amino acid sequence is altered to contain amino acid substitutions, deletions and/or additions. If, however, the endogenous SIBLINGS protein sequence is altered it will continue to display its ability to inhibit complement-mediated lysis and the derived SIBLINGS protein sequence will be additionally characterized by having at least 50%, 60%, 70%, 80%, or 90% sequence identity to the endogenous sequence.

As mentioned above, the inhibition of complement-mediated lysis can be detected using the MEL cell based assay described below. A SIBLINGS protein will be found to be "biologically active" if when tested in the MEL assay it confers protection to cells compared to a control sample that does not contain the SIBLINGS protein.

**BSP**: The normal endogenous population of bone sialoprotein. This population contains a wide range of BSP proteins, some that are not extensively post-translationally modified, as well as some of which are extensively post-translationally modified. Furthermore, this normal population contains within it at least two distinct sub-populations. The first sub-population is relatively non-acidic, and it is the sub-population that is most prominent in the serum of normal subjects (subjects without tumors). The second sub-population of BSP is relatively acidic and found in subjects with tumors, for example in subjects with breast, lung, prostate, and thyroid tumors.

**Relatively Acidic BSP:** The term relatively acidic BSP refers to a sub-population of BSP found primarily in the serum of subjects who have various types of tumors. There are multiple methods that can be used to detect the presence of this sub-population in a subject. These methods include column chromatography methods and electrophoresis. Therefore, for clarity the relatively acidic BSP is defined through its behavior on the specific ion exchange column assay described in section IV, below. Briefly, this section details the use of a Toyopearl QAE column for the separation of the relatively non-acidic BSP from the relatively acidic BSP. Characterized in this way, and using the assay provided, the relatively acidic BSP will elute from the column at a salt concentration of greater than 0.5 M NaCI.

While not wishing to be bound to a particular theory, it is believed that the relatively acidic BSP is post-translationally modified to contain sialic acids, phosphate, and/or sulfate. In that case, the relative concentration of each sub-population of phosphorylated or sulfated BSP can be determined by assays that quantify the concentration of sulfur and/or phosphate.

**Specific binding agent:** An agent that binds substantially only to a defined target. Thus, a SIBLINGS protein specific binding agent binds substantially only a particular SIBLINGS protein. For example, the term "BSP specific binding agent" includes anti-BSP and other agents that bind substantially only to BSP. Similarly, the term "relatively acidic BSP specific binding agent" includes antibodies that are specific for the relatively acidic BSP and other agents that bind substantially only to the relatively acidic BSP. The term "relatively non-acidic BSP specific binding agent" includes antibodies that are specific for the relatively acidic BSP and other agents that bind substantially only to the relatively non-acidic BSP. The term "exposed portion of SIBLINGS protein specific binding agent" includes antibodies and other agents that are specific for the exposed portion of a SIBLINGS protein when it is complexed with Factor H.

The discussion below specifically mentions anti-BSP antibodies, however it also applies to anti-relatively acidic BSP antibodies, anti-relatively non-acidic BSP antibodies, as well as anti-exposed portion of SIBLINGS protein antibodies.

The term anti-SIBLINGS protein antibodies encompasses monoclonal and polyclonal antibodies that are specific for SIBLINGS proteins, i.e. which bind substantially only to BSP when assessed using the methods described below, as well as immunologically effective portions (fragments) thereof. Preferably, the anti-SIBLINGS protein antibodies used in the present invention are monoclonal antibodies (or immunologically effective portions thereof) and may also be humanized monoclonal antibodies (or immunologically effective portions thereof). Immunologically effective portions of monoclonal antibodies include Fab, Fab', F(ab')₂ Fabc and Fv portions (for a review, see Better and Horowitz, *Methods Enzymol*. **178**:476-496, 1989). Anti-BSP antibodies may also be produced using standard procedures described in a number of texts, including Antibodies, A Laboratory Manual by Harlow and Lane, Cold Spring Harbor Laboratory (1988).

The determination that a particular agent binds substantially only to the desired immunogen may readily be made by using or adapting routine procedures. One suitable *in vitro* assay makes use of the Western blotting procedure (described in many standard texts, including Antibodies, A Laboratory Manual by Harlow and Lane, Cold Spring Harbor Laboratory, 1988). Western blotting may be used to determine that a given specific binding agent, such as an anti-SIBLINGS protein monoclonal antibody, binds substantially only to a SIBLINGS protein, as described in Fisher et al., *JBC,* **262**:9702-08, 1987.

**Vector**: A nucleic acid molecule as introduced into a host cell, thereby producing a transformed host cell. A vector may include nucleic acid sequences that permit it to replicate in the host cell, such as an origin of replication. A vector may also include one or more selectable marker genes and other genetic elements known in the art.

**Transformed**: A transformed cell is a cell into which has been introduced a nucleic acid molecule by molecular biology techniques. As used herein, the term transformation encompasses all techniques by which a nucleic acid molecule might be introduced into such a cell, including transfection with viral vectors, transformation with plasmid vectors, and introduction of naked DNA by electroporation, lipofection, and particle gun acceleration.

**Sample:** The term sample includes any artificially generated or biological specimen that is of interest and is being tested for the presence of SIBLINGS proteins. More specifically, the term biological specimen includes any specimen that was generated by a living cell. Therefore, samples derived from cells in a tissue culture setting, including the cells themselves fall within the definition or biological specimen. Furthermore, samples taken from a subject, such as tissue samples and bodily fluids (such a blood plasma or serum) also fall within the definition of biological specimen.

**Tumor:** Tumors are abnormal growths which can be either malignant or benign, solid or liquid (for example, hematogenous). This term particularly includes malignant tumors which can be either solid (such as a breast or prostate carcinoma) or non-solid (such as a leukemia). Tumors can also be further divided into subtypes, such as adenocarcinomas (e.g. of the breast, prostate or lung).

**Metastasis:** A tumor implant discontinuous with a primary tumor. Any cancer may metastasize to bone, but metastases from carcinomas are the most common. There is a subset of carcinomas that is clinically recognized as more likely to metastasize to bone, and this subset includes carcinomas of the breast, lung, prostate, kidney and thyroid. Even if a tumor is not presently recognized as being particularly likely to metastasize to bone, it can be so categorized by a diagnosis in a particular subject that a skeletal metastasis has occurred (for example by scintigram and biopsy), or by studies of populations of subjects presenting with a particular histological sub-type of cancer.

**Isolated:** An isolated biological component (such as a nucleic acid or protein) has been substantially separated or purified away from other biological components in the biological specimen in which the component naturally occurs, i.e., other chromosomal and extrachromosomal DNA and RNA, and proteins. Nucleic acids and proteins which have been isolated thus include nucleic acids and proteins purified by standard purification methods. The term also embraces nucleic acids and proteins prepared by recombinant expression in a host cell as well as chemically synthesized nucleic acids and proteins.

**Purified:** The term purified does not require absolute purity; rather, it is intended as a relative term. Thus, for example, a purified BSP protein preparation is one in which the BSP is more enriched than the protein is in its natural environment. That environment being either in serum or within a cell. Preferably, a preparation of BSP is purified such that BSP represents at least 50 % of the total protein content of the preparation.

**Operably linked:** A first nucleic acid sequence is operably linked with a second nucleic acid sequence when the first nucleic acid sequence is placed in a functional relationship with the second nucleic acid sequence. For instance, a promoter is operably linked to a coding sequence, if the promoter affects the transcription or expression of the coding sequence. Generally, operably linked DNA sequences are contiguous and, where necessary to join two protein coding regions, in the same reading frame.

**Recombinant:** A recombinant nucleic acid is one that has a sequence that is not naturally occurring or has a sequence that is made by an artificial combination of two otherwise separated segments of sequence. This artificial combination is often accomplished by chemical synthesis or, more commonly, by the artificial manipulation of isolated segments of nucleic acids, e.g., by genetic engineering techniques.

**Subject:** This term includes both human and non-human mammals. Similarly, the term subject includes both human and veterinary subjects.

**Sequence identity:** The term "sequence identity" is used to describe the similarity between two nucleic acid sequences or between two amino acid sequences. Sequence identity is typically expressed in terms of percentage identity; the higher the percentage, the more similar the two sequences.

Methods for aligning sequences for comparison purposes are well known in the art. Various programs and alignment algorithms are described in: Smith and Waterman, *Adv. Appl. Math.* **2**:482, 1981; Needleman and Wunsch, *J. Mol. Biol.* **48**:443, 1970; Pearson and Lipman, *Proc. Natl. Acad. Sci. USA* **85**:2444-2448, 1988; Higgins and Sharp, *Gene* **73**:237-244, 1988; Higgins and Sharp, *CABIOS* **5**:151-153, 1989; Corpet et al., *Nucleic Acids Research* **16**:10881-10890, 1988; Huang et al., *Computer Applications in the Biosciences* **8**:155-165, 1992; and Pearson et al., *Methods in Molecular Biology* **24**:307-331, 1994; Altschul et al., *J. Mol. Biol.* **215**:403-410, 1990, presents a detailed consideration of sequence alignment methods and homology calculations.

The NCBI Basic Local Alignment Search Tool (BLAST™, Altschul et al., *J. Mol. Biol.* **215**:403-410, 1990) is available from several sources, including the National Center for Biotechnology Information (NCBI, Bethesda, MD) and on the Internet, for use in connection with the sequence-analysis programs blastp, blastn, blastx, tblastn and tblastx.

For comparisons of amino acid sequences of greater than about 30 amino acids, the "Blast 2 sequences" function in the BLAST™ program is employed using the default BLOSUM62 matrix set to default parameters, (gap existence cost of 11, and a per-residue gap cost of 1). When aligning short peptides (fewer than about 30 amino acids), the alignment should be performed using the Blast 2 sequences function, employing the PAM30 matrix set to default parameters (open gap 9, extension gap 1 penalties). Proteins having even greater similarity to the reference sequences will show increasing percentage identities when assessed by this method, such as at least 45% , at least 50%, at least 60%, at least 70%, at least 75% , at least 80 %, at least 85% , at least 90%, or at least 95 % sequence identity.

A first nucleic acid is "substantially similar" to a second nucleic acid if, when optimally aligned (with appropriate nucleotide insertions or deletions) with the other nucleic acid (or its complementary strand), nucleotide sequence identity occurs in at least about 60%, 75%, 80%, 85%, 90% or 95 % of the nucleotide bases. (As used herein, "optimally aligned" sequences exhibit a maximal possible sequence identity). Sequence similarity can be determined by comparing the nucleotide sequences of two nucleic acids using the BLAST™ sequence analysis software (blastn) available from The National Center for Biotechnology Information. Such comparisons may be made using the software set to default settings (expect = 10, filter = default, descriptions = 500 pairwise, alignments = 500, alignment view = standard, gap existence cost = 11, per residue existence = 1, per residue gap cost = 0.85). Similarly, a first polypeptide is substantially similar to a second polypeptide if it shows sequence identity of at least about 75%-90% or greater when optimally aligned and compared using BLAST™ software (blastp) using default settings.

**Conservative amino acid substitutions**: Conservative amino acid substitutions usually have minimal impact on the activity of the resultant protein. Such substitutions are described below.

Conservative substitutions replace one amino acid with another amino acid that is similar in size, hydrophobicity, etc. Such substitutions generally are conservative when it is desired to finely modulate the characteristics of the protein. Examples of amino acids which may be substituted for an original amino acid in a protein and which are regarded as conservative substitutions include: Ser for Ala; Lys for Arg; Gln or His for Asn; Glu for Asp; Ser for Cys; Asn for Gln; Asp for Glu; Pro for Gly; Asn or Gln for His; Leu or Val for Ile; Ile or Val for Leu; Arg or Gln for Lys; Leu or Ile for Met; Met, Leu or Tyr for Phe; Thr for Ser; Ser for Thr; Tyr for Trp; Trp or Phe for Tyr; and Ile or Leu for Val.

More substantial changes in enzymatic function or other features may be obtained by selecting substitutions that are less conservative than those above, i.e., selecting residues that differ more significantly in their effect on maintaining: (a) the structure of the polypeptide backbone in the area of the substitution, for example, as a sheet or helical conformation, (b) the charge or hydrophobicity of the molecule at the target site, or (c) the bulk of the side chain. Substitutions that generally produce the greatest changes in protein properties are those in which: (a) a hydrophilic residue, e.g., seryl or threonyl, is substituted for (or by) a hydrophobic residue, e.g., leucyl, isoleucyl, phenylalanyl, valyl, or alanyl; (b) a cysteine or proline is substituted for (or by) any other residue; (c) a residue having an electropositive side chain, e.g., lysyl, arginyl, or histadyl, is substituted for (or by) an electronegative residue, e.g., glutamyl or aspartyl; or (d) a residue having a bulky side chain, e.g., phenylalanine, is substituted for (or by) one not having a side chain, e.g., glycine. The effects of these amino acid substitutions or deletions or additions may be assessed for SIBLINGS protein derivatives by analyzing the ability of the respective modified polypeptide to inhibit complement-mediated cell lysis.

Variant SIBLINGS protein encoding cDNAs or genes may be produced by standard DNA mutagenesis techniques, for example, M13 primer mutagenesis. Details of these techniques are provided in Sambrook et al. (ed.), *Molecular Cloning: A Laboratory Manual*, 2nd ed., vol. 1-3, Ch. 15, Cold Spring Harbor Laboratory Press, Cold Spring Harbor, NY, 1989, and Ausubel et al. (ed.) *Current Protocols in Molecular Biology,* Greene Publishing and Wiley-Interscience, New York (with periodic updates), 1987. By the use of such techniques, variants may be created that differ slightly from the endogenous SIBLINGS protein encoding cDNA or gene sequences, yet that still encode a biologically active SIBLINGS protein. DNA molecules and nucleotide sequences that are derivatives of those specifically disclosed herein and that differ from those disclosed by the deletion, addition, or substitution of nucleotides while still encoding a biologically active SIBLINGS protein are comprehended by this invention. In their simplest form, such variants may differ from the disclosed sequences by alteration of the coding region to fit the codon usage bias of the particular organism into which the molecule is to be introduced.

Additionally, as mentioned above new members of the SIBLINGS family of proteins can be identified by searching various DNA databases, such as the database maintained at the National Center for Biotechnoloy Information in Bethesda, MD. Searches can be done such that sequences that are conserved between existing members of the SIBLINGS family of proteins are used to identify other members of the family. Once identified these members can be used to the assays described below to determine if they maintain SIBLINGS protein biological activity.

Throughout the specification and claims, reference to the singular (such as "a" or "the") includes the plural, unless clearly indicated otherwise by context.

### II. Identification and Characterization of SIBLINGS/Factor H Complex

Initial experimentation, which is described below, led to the identification of a BSP/Factor H complex. The population of BSP in serum from subjects displaying tumors was additionally found to contain a sub-population of relatively acidic BSP. Subsequent experimentation showed that both OPN and DMP1 bound to Factor H and it is likely that the detection of these SIBLINGS proteins, as well as other members of the protein family, will be enhanced by disrupting the SIBLINGS/Factor H complex prior to quantifying protein levels.

### A. Identification and Characterization of the BSP/Factor H Complex

BSP is an acidic protein, containing not only many acidic amino acids (particularly glutamic acid) but also phosphate, sulfated tyrosines and oligosaccharides, as well as many sialic acid groups. As such it should bind to an anion exchange column under conditions of low salt (<0.1 M NaCl) and neutral pH (6-8). This is indeed the case, and BSP from bone extracts and from culture medium has been found to elute off of a DEAE column at about 0.4 M NaCl in 7 M urea at pH 6.

Surprisingly, serum BSP failed to behave in the same manner as BSP from bone extracts and cell culture medium as described above. In fact, injecting normal human serum onto a DEAE or QAE HPLC column resulted in all of the detectable BSP eluting in the unbound fraction, therefore it behaved as a neutral or positively charged protein. This indicated that the BSP was bound to something that masked its many negative charges, hence attempts were made to disrupt the complex. These attempts involved using denaturants, such as 50% formamide or 8 M urea, reducing agents such as 1 mM DTT or 10 mM β-mercaptoethanol, and heating to 100°C for 5 minutes. None of these approaches was independently successful in disrupting the complex, as indicated by the failure of the BSP to bind to an anion exchange column.

However, a combination of a denaturant, a reducing agent and heating finally did separate the BSP from the complex. This separation was noted by the increased binding of the BSP to an anion exchange column with subsequent elution in a salt gradient and detection with polyclonal antisera. The anti-sera used was raised in rabbit against human BSP or fragments thereof, and termed LF-6, LF-83, LF-84, LF-100, LF-101, LF-119, LF-120, and LF-125 (see Table 2), which were tested for specificity via either ELISA assay or Western blotting. (Acta Orthop. Scand, (Suppl. 266) **66**:61-65, 1995. The LF-142 (Table 2) polyclonal antisera was raised in rabbit against a synthetic BSP peptide which contained several sulfated tyrosines.

The previously uncharacterized protein described above, that was found to mask the otherwise acidic BSP, was subsequently identified as being Factor H. The identity of the protein was determined by several different assay techniques, and these techniques, as well as the results are described below.
**1)** Factor H specific monoclonal antibodies were obtained from Quidel, San Diego, California, USA. These antibodies were used to precipitate Factor H from human sera. One sample was left untreated while another was heated to 100°C for 5 minutes in the presence of 1 mM DTT and 50% formamide. These samples were then separated on a SDS gel and subjected to western blotting analysis using a BSP specific antibody LF-100. Results from the Western blot analysis showed a high molecular weight band (Mr ~200,000 Da) in the lane without DTT, and the more typical BSP size of 75,000 Da in the lane treated with DTT and formamide.
   Furthermore, a similar high molecular weight band was also seen when a Western blot was performed using as a sample purified human Factor H (Quidel) complexed with BSP. This artificially created complex was immunoprecipitated by the same Factor H antisera (see above), and analyzed with the same results as with the whole human serum described above. This showed that BSP was bound to Factor H.
**2)** Biotinylated human BSP was added to normal human serum and the material was chromatographed on a calibrated molecular sieve column (Superose 6, Pharmacia, Fig. 1B). A separate chromatogram with the biotinylated BSP alone was also generated. The biotinylated products were subsequently detected using HRP-conjugated avidin on a 96-well direct ELISA (Fig. 1A). The BSP alone chromatographed to the expected approximate 75,000 Da position, while the BSP added to the human serum chromatographed to a position consistent with being complexed to Factor H (about 200,000 Da, Fig. 1B, detected with HRP-conjugated avidin). Furthermore, no 75,000 Da, (i.e. free) BSP could be detected, suggesting that all was bound to the Factor H (Factor H is found at concentration of about 500 µg/mL human serum). An additional chromatograph was generated with biotinylated BSP added to Factor H (Fig. 1C). This chromatograph showed that the product eluted at the same size at the sample containing biotinylated BSP and normal human serum (Fig. 1B).
   The effect of treatment with DTT and 50% formamide was observed using a molecular sieve column and detecting the products with the polyclonal antibody LF-100. This antibody was previously found to bind to both the bound and unbound form of BSP, however, this binding is probably due to the fact that the SIBLINGS/Factor H complex is at least partially disrupted. Results showed that the BSP from untreated normal human serum eluted at about 200,000 Da (Fig. 2A). Similarly, biotinylated BSP with normal human serum showed a major peak at about 200,000 Da, the expected size of the BSP/Factor H complex, and a second small peak about 75,000 Da (Fig. 2B). Finally, a sample of normal human serum incubated in the presence of 50% formamide and 1 mM DTT at 100°C for five minutes resulted in a peak corresponding to BSP (Fig. 2C).
**3)** Biotinylated human BSP bound normally to the anion exchange column. Addition of human serum prior to loading on the anion exchange column caused the biotin activity to elute in the unbound fraction. Pretreating with purified human Factor H gave the same results. These results showed that Factor H could render the BSP unable to bind to the column, a property of the BSP found naturally in human serum.
**4)** Fluorescence spectroscopic analysis of the tryptophanes of Factor H (BSP does not contain tryptophane) during the binding of BSP shows that the two rapidly form a strong interaction that is stoichiometric (1:1) and saturable (Fig. 3A and 3B).

### B. Characterization of OPN and DMP1/Factor H Binding

The salient structural features of Factor H include 20 short consensus repeats (SCRs) that contain four cysteine residues forming two disulfide bonds per repeat. In addition, each SCR contains one conserved tryptophan residue per repeat and Factor H is known to interact with several sialic acid-containing proteins. Therefore, binding interactions between members of the SIBLINGS family of proteins and Factor H can be studied by steady state fluorescence.

Steady state fluorescence studies were accomplished by titrating purified human complement Factor H with rBSP, rOPN, and rDMPl followed by excitation at 295 nm and monitoring emission between 300 and 450 nm. The emission profile of Factor H alone yields a peak at 347 nm. The addition of rBSP, rOPN, or rDMP1 in nm increments causes a relative fluorescent intensity quenching. Conversion of the fluorescent intensity titration into a binding curve by determining the fraction of binding sites occupied as the fractional change in fluorescence quenching at 347 nm yields a saturable binding curve. By steady state fluorescence, the binding of BSP, OPN, and DMP1, by Factor H are saturable and possess a 1:1 stoichiometry and the binding constants are in the nM range. Given that normal serum concentration of Factor H is ∼ 0.5 mg/mL it is probable that virtually all BSP, OPN, and DMP1 in serum will be complexed with Factor H.

### III. Detection of Elevated Levels of Members of the SIBLINGS Family of Proteins in Serum

Recent observations have shown that BSP and OPN are expressed by malignant tissue. BSP is expressed in primary breast cancers (Gillespie et al., *Int. J. Cancer* **73**(6):813-815, 1997; Bellahcene et al., *Cancer Res.* **54**(11):2823-2826, 1994; Bellahcene et al., *Int. J. Cancer* **69**(4):350-353, 1996; Seibel et al., J. Clin. Endocrinol Metab. **81**(**9**):3289-3294, 1996), prostate cancer (Waltregny et al., *J. Natl. Cancer Inst.* **90**(13):1000-1008, 1998) , lung cancer (Bellahcene et al., *Calcif. Tissue Int.* **61**(3):183-188, 1997), thyroid cancer (Bellahcene et al., *Thyroid* **8**(8):637-641, 1998), malignant bone disease (Chen et al., *Histochem*. **30**(1):1-6, 1998), and by neoplastic odonotoblasts (van der Pluijm et al., *Cancer Res.* **56**(8):1948-1955, 1996). In addition, BSP peptides are potent inhibitors of breast cancer cell adhesion to bone (van der Pluijm et al., *Lab Invest.* **77**(6):665-675, 1997; Tunio et al., *Arch. Pathol. Lab. Med..* **122**(12):1087-1090, 1998). OPN is expressed in breast cancer (Gillespie et al., *Int. J. Cancer.* **73**(6):812-815, 1997; Sung et al., *Exp. Cell Res.* **241**(2):273-284, 1998; Bellahcene and Castronovo, *Bull. Cancer.* **84**(1):17-24, 1997; Tuck et al., *Int. J. Cancer* **79**(5):502-508, 1998), as well as in prostate cancer (Koeneman et al., *Prostate* **39**(4):246-261, 1999), thyroid cancer (Wright et al., *Int. J. Cancer.* **46**(1):39-49, 1990), skin cancer (Senger et al., *CBiochim. Biophys. Acta* **996**(1-2):43-48, 1989) and several other types of cancer (Senger et al., *AntiCancer Res.* **56**(8):1948-1955, 1996; Chambers et al., *Anticancer Res..* **12**(1):43-47, 1992; Mevoracj et al., *J. Exp. Med..* **188**(12):2313-2320, 2331, 1998). Given that these two SIBLINGS proteins are expressed in cancer tissue it is likely that other members of the family will also be found to be over expressed in tumors. Hence, it is desirable to develop sensitive assays for detecting the presence of SIBLINGS proteins.

As described above, it has been shown the BSP, OPN, and DMP1 bind to Factor H. Hence, it is likely that these members of the SIBLINGS family of proteins as, well as the yet untested DPP1 protein, are bound to Factor H *in vivo.* Therefore, in order to accurately detect the concentration of these proteins in serum the SIBLINGS/Factor H complex should be disrupted prior to quantifying the SIBLINGS protein level.

Serum levels of BSP have been quantified previously via radioimmunoassays (RIAs). These assays, which involve incubation for a 24 hour period in the presence of antibody, have led to the determination that the circulating levels of BSP in normal subjects are between 5 ng/mL and 24 ng/mL (Karmatschek, *Clinical Chemistry* **43**:2076-82, 1997). However, using the experimental procedures detailed below, which exploit the finding that BSP is at least partially undetectable when bound to Factor H, it has been determined that the circulating concentration of BSP in normal subjects may extend across a much broader range, for example between 200 ng/mL serum and 1000 ng/mL serum. Thus, the discovery that BSP is bound to Factor H allows BSP levels to be more accurately determined.

The direct detection of BSP as well as other SIBLINGS proteins in serum is hampered by the fact that it is effectively bound by Factor H and is not expected to be easily detected by most antisera. For example, serum samples where eluted from an ion exchange column, and ELISA assays (described below) were preformed using polyclonal antisera LF-6 and LF-83 (Table 2). These antisera were the same polyclonal antisera that were used by Bellahcene et al., *Cancer Research* **54**:823-826, 1994; Bellahcene et al., *Calcif. Tissue Int.* **61**:183-188, 1997; Bellahcene et al., *Calcif. Tissue Int.* **61**:183-188, 1998a; and Bellahcene et al., *Thyroid* **8**:637-641. 1998b, to detect the extopic expression of BSP in various tumor types. However, these antisera, as well as several others failed to detect the BSP/Factor H complex found in serum. Therefore, disrupting the BSP/Factor H complex prior to quantitating serum BSP levels allows for a more accurate assessment of serum BSP concentration.

There are several different methods which could be used to disrupt the SIBLINGS/Factor H complex and detect serum SIBLINGS protein levels. However, the methods used ideally take into account: 1) the properties of the SIBLINGS/Factor H complex compared to the SIBLINGS protein once it is freed from Factor H; 2) the possible problems resulting from denaturing other serum proteins, such as albumin (for example, denatured serum in 50% formamide is acceptable, but if the denaturant present is subsequently removed, the serum forms a gel even if diluted 1:10 in normal saline buffer); and 3) the SIBLINGS protein should also end up in a medium that is suitable for conducting immunoassays after the SIBLINGS/Factor H complex is disrupted.

One particularly useful method of detecting SIBLINGS proteins involves separating the SIBLINGS protein from Factor H, inactivating the contaminants, such as DTT and BME, and then diluting the sample such that the sample is in a media that allows for immunoassay techniques to be used. This assay method is more feasible, in part, because of the considerably higher concentration of SIBLINGS protein that is available after separation form Factor H.

Another example of a method for quantifying serum levels of BSP as well as other SIBLINGS proteins involves: 1) separating the SIBLINGS/Factor H complex from most other serum components, particularly albumin; 2) disrupting the complex (by substantial separation of Factor H from the SIBLINGS protein); and 3) introducing the SIBLINGS protein into buffer conditions compatible with binding to antibodies. The initial separation step involved diluting 50 µl serum with 150 µl PBS. The resulting diluted serum was then placed on a disposable anion exchange column (Toyopearl QAE column available from Taso-Haas, a part of Rhom&Hass, Montgomeryville, Pennsylvania). The albumin present in the serum was then bound to the column and the Factor H- SIBLINGS protein complex passed through. The complex was then separated via incubating at 100°C in the presence of DTT and formamide. The denaturants and reducing agents were then removed by placing the sample on a sephadex G25 column available from Amersham Pharmacia, Piscataway, New Jersey, which was previously equilibrated with normal saline or some other ELISA- or RIA-compatible buffer. The eluted SIBLINGS protein was then detected by ELISA assay.

Used in this way the invention allows for the development of screening assays for the detection of SIBLINGS protein in serum. These assays involve separating the SIBLINGS protein from the SIBLINGS protein/Factor H complex and identifying the free SIBLINGS protein. Therefore, these assays can be used in conjunction with several already existing monoclonal and polyclonal antisera, as well as other antibodies developed in the future. This will allow for the more accurate assessment of SIBLINGS protein levels as well as the ability to assess the presence of several disease states in subjects. For example, the presence of tumors, Paget's disease, rheumatoid arthritis and hyperparathyroidism can be detected via the above-described assay.

### A. Identification Of A Highly Acidic Form Of BSP In Subjects Known To Have Tumors

As described above, subjects with certain types of tumors have a population of BSP which ranges from a peak which elutes at a salt concentration that is similar to that of normal subjects, to a peak which elutes later than BSP from a normal subjects. Therefore, in subjects with tumors (particularly certain types of tumors, such as those that often metastasize to bone) the serum BSP tends to be not only increased in total amount but also differs from that of a normal subject. This difference, between the BSP populations of normal subjects and subjects with tumors, can be exploited for the development of an assay for detecting tumors. One such assay is described below.

Differences in elution profiles between subjects with tumors and subjects without tumors was illustrated by taking 50 µl of serum from normal volunteers and from patients with tumors prior to surgery (no special treatment is required for drawing or holding the serum). Subsequently, 50 µl of fresh formamide and 1 µl of 100 mM DTT were added to the serum sample, and it was heated in a sand-filled heating block (100°C) for 5 minutes, thereby separating the BSP from Factor H. The sample was then microfuged for 1 minute at 15,000 x g and injected onto a 1 mL packed volume Toyopearl QAE (Toso-Haas) column, that had been pre-equilibrated with 0.05 M sodium phosphate, 50% fresh formamide, pH 7.4. The column was then washed using a flow rate of 2 mL/minutes for 5 minutes in the same low salt buffer that was used for equilibration, and then the salt concentration was linearly increased to 2 M NaCl (in the same buffer, formamide is optional) over 80 minutes (Figure 4A-F). Finally, the salt concentration was at 2 M NaCl for the final 15 minutes.

Similar results were seen in five different subjects with breast tumors. The method used is described above with the exception that a steeper salt gradient was used (25 minutes) followed by 2 M NaCl for the final 15 minutes (Fig. 5A-E).

Then 100 µl of each fraction was placed in a corresponding well of a microtiter plate. It was found that Greiner High-binding plates or Nunc Maxisorp worked well for binding both the relatively non-acidic form of BSP and the relatively acidic form of BSP. The microtiter plates were incubated for between 1 and 18 hours at 4°C. The plates were then washed three times (5 minutes each) with >200 µL/well Tris-buffered saline plus 0.05% Tween 20 (TBS-T) and exposed to 100 µL of rabbit polyclonal antiserum (LF-100) raised against human BSP in TBS-T for 1 hour at room temperature. The plates were then washed again as described above, and exposed to 100 µL 1:2000 HRP-conjugated goat anti-rabbit IgG (human serum adsorbed, Kirkegaard & Perry, Gaithersburg, Maryland) in TBS-T for one hour at room temperature. Finally, the plates were washed a third time and the color was developed using 3,3', 5,5' tetramethylbenzidine and H₂O₂ for 10-30 minutes at room temperature. Color development was stopped by that addition of 25 µL 1 N H₂SO₄ and analyzed at 450 nm.

Results from the above described HPLC separation technique indicated that the relatively acidic BSP was detected only in sera from subjects with various tumors and was eluted from the column generally at salt concentrations greater than 1 M NaCl. It was readily apparent that subjects with these various tumors not only had increased levels of BSP, but also that they had a different population of BSP compared to that of normal subjects.

The use of the semi-quantitative method described above allowed for the detection of a relatively non-acidic form of BSP that eluted early in the gradient (≤1 M NaCl). Of course, altering the chromatography procedure used, such as by changing the type of resin, could lead to elution of the peak at a different salt concentration, however, regardless of the separation method used there will be a sub-population of detected BSP which is less negatively charged, and will elute separately from the more negatively charged fraction. In normal subjects this relatively less negatively charged sub-population would be the only or at least the dominant population which is detected. However, in the case of normal subjects it is still possible that there will be at least a trace amount of the relatively acidic BSP. It is believed that the relatively non-acidic BSP can be used to measure bone turnover generally.

Furthermore, the relatively non-acidic BSP population has been detected in both normal subjects, as well as in subjects known to have tumors. Subjects who have prostate tumors may have elevated levels of the relatively non-acidic BSP compared to normal, which possibly indicates that there is an elevated level of bone turnover.

The above-described assay is designed to detect any tumor that makes BSP and has a sufficiently high tumor load. Using the techniques described herein, it can be determined whether any particular tumor type is associated with increased BSP levels, and particularly acidic forms of BSP. Furthermore, the identification of the relatively acidic form also provides a more sensitive assay than those based on merely assessing total serum BSP levels.

Furthermore, the above-described assay could be simplified so that only three steps are needed. These steps are, removal of the albumin by binding decomplexed BSP in low salt (≤ .2 M NaCl), eluting the relatively non-acidic BSP with 1 M NaCl and subsequently eluting the relatively acidic BSP with 2 M NaCl. Samples can then be placed in a solution that allows for antibody binding and the BSP recovered can be detected by ELISA.

### B. Creation of Specific Binding Agents Which Recognize Free BSP, As Well As Bound BSP

Nine rabbit (polyclonal) antisera against human BSP or fragments (LF-6, LF-83, LF-84, LF-100, LF-101, LF-119, LF-120 and LF-125) (including peptides) have been made. These include eight polyclonal antibodies whose activity is summarized in the Acta Orthop. Scand, (Suppl. 266) **66**:61-65, 1995, as well as one made since (LF-142) that has not been published. The immunogens used to produce these antisera is provided below in Table 2.

**TABLE 2**

| **Gene Product** | **Antiserum** | **Antigen** | **Known Species** |
|---|---|---|---|
| Human BSP | LF-6 | human bone BSP | H,M,D,R,Mou |
| Human BSP | LF-83 | YESENGEPRGDNYRAY ED- (LPH) (SEQ ID NO: 2) | H,M,D,R,Mou,C |
| Human BSP | LF-84 | YESENGEPRGDNYRAY ED- (LPH) (SEQ ID NO: 2) | H,M,D,R,Mou,C |
| Human BSP , | LF-100 | AIQLPKKAGDIC-(LPH) (SEQ ID NO: 3) | 'H,M,D,R |
| Human BSP | LF-101 | AIQLPKKAGDIC-(CSA) (SEQ ID NO: 3) | H,M,D,R,P,B |
| Human BSP | LF-II9 | recomb.RGDdomain (AA257-317) (SEQ ID NO: 4) | H,M,D,C |
| Human BSP | LF-120 | recomb. Frag I (AA 129--281) (SEQ ID NO: 5) | H,M,D,P,R |
| Human BSP | LF-125 | recomb. (36-61) (SEQ ID NO: 6) | H,M,P,R,S |
| Human BSP | LF-142 | CTVEY*EGEY*EY*TGA NDY*DNGY*EIY*ESEN Y*(SEQ ID NO: 7) | H |

| | | | |
|---|---|---|---|
| H=human, M=monkey, D=dog R=rat, Mou=mouse, C=chicken, P=pig, S=sheep, B=bovine, LPH=horseshoe crab hemocyanin, CSA=chicken serum albumin | | | |
| *indicates sulfated tyrocine | | | |

Of these 9 polyclonal antisera only LF-100 can immunoprecipitate natural BSP, and even LF-100 only binds to a portion of the BSP (< 5 %) when it is complexed with Factor H in human serum as detected by Western blot analysis. The other antisera precipitate little or no BSP/Factor H complex.

A similar finding was made when an artificial complex was made by incubating an excess of purified Factor H with biotinylated BSP. Once again, only the LF-100 antisera was capable of precipitating even a small portion of the BSP/Factor H complex.

The ability of polyclonal antisera against LF-100 to precipitate both bound and unbound BSP makes it highly likely that a monoclonal antibody to the same fragment would behave in the same manner. This monoclonal equivalent to LF-100 can be made by injecting into mice the identical or similar peptide-carrier conjugate, as discussed in Example 2. Clones derived from these mice can also be screened for the ability to recognize both bound and unbound BSP.

It is also believed that a monoclonal antibody against the amino-terminus or carboxy-terminus of BSP will work because these domains may remain free of the BSP-Factor H complex.

Alternatively, a monoclonal antibody may be made by first obtaining the BSP/Factor H complex, and then using the complex itself as an immunogen. The BSP/Factor H complex is obtained either through isolating it from serum or through artificially creating it using isolated Factor H and recombinant BSP. For example, 5 mg of purified human Factor H (from Quidel, LaJolla CA) can be complexed *in vitro* with 2 mg of purified human BSP from the adenoviral system described below. The complex can then be passed through a QAE column without denaturants to remove any unbound BSP. The complex can then be chromatographed on a molecular sieve column (Superose 6, Pharmacia) to separate the complex from any unbound Factor H. The resulting complex is injected into mice for monoclonal production. All clones that can bind to recombinant BSP in the initial screening can then be assayed to see if they can immunoprecipitate the BSP/Factor H complex. Even those that can not immunoprecipitate the intact complex will be useful as BSP monoclonals for the detection of BSP after it has been separated from Factor H, or for use in screening for ectopic expression as described below.

The method of using the BSP/Factor H complex as an immunogen described above can also be used with other SIBLINGS protein. Thus, monoclonal antibodies which bind to other SIBLINGS/Factor H complexes can be generated.

### C. Creation Of Specific Binding Agents Which Recognize The Relatively Acidic Form Of BSP

Monoclonal antibodies that recognize the relatively acidic form of BSP can be made by using the relatively acidic BSP isolated from human serum; using relatively acidic BSP isolated from tumor tissue; using synthetic peptides which are highly phosphorylated or sulfated; or by using recombinant BSP. A particularly promising method of producing monoclonal antibodies to the relatively acidic form of BSP is by complexing relatively acidic BSP to Factor H and using the complex as an immunogen. This offers the possibility of recovering an antibody which recognizes both the bound and unbound forms of the relatively acidic BSP.

Methods of obtaining the recombinant BSP for use as an immunogen are described below, and methods of obtaining the BSP/Factor H complex for use as an immunogen are described above.

The relatively acidic form of BSP can be isolated directly from human tumors using the denaturing procedures provided in Fisher et al., *J Biol. Chem.* **258**:12723-727, 1983. Furthermore, methods of generating such monoclonal antibodies are well known to those of skill in the art and these methods are additionally detailed below. Briefly, subsequent to injection of the immunogen and fusion, the clones are screened for binding to the relatively acidic form of BSP, and then subsequently screened for binding to the relatively non-acidic form of BSP. Monoclonal antibodies that specifically detect the relatively acidic form of BSP are selected for subsequent identification of this substance in biological specimens.

### D. Detection of Elevated Levels of Serum BSP in Subjects known to have Breast Cancer or Prostate cancer

Sera were obtained from clinically defined normal, prostate and breast cancer patients (East Coast Biologics, Inc., North Burwick, Maine and Johns Hopkins Bayview Medical Center, General Clinical Research Center) under informed consent with IRB approval and quenched samples were taken for analysis.

The day before serum was collected a 20 µg/mL solution of recombinant BSP (rBSP) was diluted out from a 1 mg/mL stock solution in 4 M guanidine HCl. 100 µl of the dilute BSP was added each well of a Greiner high binding (product number 655061) 96 well plate. The plate was then covered and incubated overnight at 4° C, however, shorter incubation periods are possible.

A solution of TBS-Tween containing 0.05 M Tris-HCl, pH 7.5, 0.15 M NaCl, 0.05 % Tween 20, was prepared. A blocking buffer containing Tris buffered saline (TBS) + 1 %BSA + 5% powdered milk was also prepared.

Samples were diluted 1:10 in a 30% formamide 40 mM phosphate buffer, pH 7.4. The samples were then reduced with 2 mM dithiothreitol (1/100 of stock, =2.5 µL) at 100°C for 5 minutes. Residual reducing agent was inactivated by the addition of equimolar H₂O₂. BSP standards containing 2000, 400, 80, 16, 3.2, 0.64, and 0.13 ng/mL rBSP were then diluted out.

LF-100 antibody was then diluted 1:2,000 in TBS-Tween and 100 µl /well of Ab solution was added to polypropylene 96 well 0.5 mL volume plate (USA Scientific which is same as Nunc version). 100 µl of standard, or sample was then added to each well. The lid was then placed on the plate and reacted at room temp, 1 hour.

During incubation of the samples the rBSP coated plate, described above, was removed from the cold and 250 µl blocking buffer was added to each well. The blocking buffer was allowed to react for 5 minutes and then it was removed. The plate was then washed three times with TBS-Tween.

100 µl reaction mixture was added to each well of the rBSP-coated plate. The plate was then incubated for 1 hour at room temp. After incubation the plate was washed three times using a buffer containing ∼ 250 µL/well TBS-Tween, and then blotted and drained.

The secondary antibody (Goat anti-rabbit IgG (H&L), peroxidase-labeled antibody conjugate, that was human serum adsorbed) which is available from Kirkegaard & Perry, Gaithersburg, MD, was diluted 1:20,000 in TBS-Tween. 100 µL of the secondary antibody was then added to each well and the plates were incubated for 1 hour at room temp. After incubation, the plates were washed three times with ∼ 250 µL/well TBS-Tween, blotted and then drained.

The results were visualized by providing 100 µL/well TMB (3,3', 5,5"-tetramethylbenzidine) microwell peroxidase substrate (BioFX Laboratories, Randallstown, MD) and incubating the plates for 30 minutes. The reaction was then stopped through the addition of 100 µL stop solution/well and the plates were read at 450 nM .

The results are shown in Fig. 7, and Table 3 below. The mean concentration of BSP for the 32 subjects with breast cancer was 1170 ng/mL, the mean concentration of BSP for the 22 subjects tested with prostate cancer was 1320 ng/mL, and finally the mean concentration of BSP for the 72 normal subjects was 490 ng/mL. Clearly, most subjects tested in both the prostate group and the breast cancer group have elevated levels of BSP. Some have BSP levels that are within the normal range, however, this is expected because not all tumors are thought to express BSP.

### E. Detection of Elevated Levels of Serum BSP in Subjects over Age 50

A group of 36 individuals over the age of 50, and a group of 32 individuals under the age of 50 were tested to determine their serum BSP levels. The results are shown in Fig. 6, and Table 3, below. The mean of the group under the age of 50 was clearly less than that of the over 50 group. This assay, therefore, shows that serum levels of BSP and possibly other members of the SIBLINGS family of proteins can be used to measure bone turnover or other age-related changes.

**Table 3**

| | < 50 years Normal | > 50 years Normals | prostate | breast | All Normals |
|---|---|---|---|---|---|
| BSP mean (ng/mL) | 344.266 | 619.787 | 1320 | 1170 | 490.13 |
| Standard Deviation | 41.318 | 179.953 | 289.582 | 364.396 | 192.096 |
| Number of Individuals Sampled | 32 | 36 | 32 | 22 | 68 |

### F. Creation Of Cell Cultures Expressing Human BSP In The Highly Acidic Form

Full-length human BSP cDNA (GenBank accession number J05213; Seq. ID NO: 8) was excised from the original plasmid with *Eco*RI and subcloned into the *Eco*RI site of the pAC-EF1 adenovirus construct plasmid. This construct contained the EF-1 promoter and the bovine growth hormone (BGH) 3' polyadenylation message stabilization sequences, which is a modification of the original pAC vector (Gluzman et al., Eucaryotic Viral Vectors, Cold Spring Harbor Laboratory Press, Cold Spring Harbor, New York, 187-192, 1982). Selection for double cross-over within the replication-deficient adenovirus genome results in replication-deficient adenovirus that can infect most cell types but can be amplified only in specific cell culture lines. Infected cells produce the human BSP, modify it according to their specific ability, and secrete the product into the culture media.

A similar cloning scheme allowed for the production of adenovirus constructs that encode OPN and DMP1 allowing choice of all lines.

One method of producing the relatively acidic BSP involves human marrow stromal fibroblasts. These cells in primary culture modify the BSP and can produce the BSP under serum-free conditions for several days. A small percentage of the BSP made in these marrow fibroblasts elutes in the high salt portion of the anion exchange column. Additionally, this relatively acidic BSP produced by the fibroblasts infected with the adenoviral vector described above stains a darker blue (characteristic of being more acidic) than other fractions of BSP that are eluted off of the anion exchange columns earlier, when using StainsAll dye for detection ((1-ethyl-2-[3-(1-ethylnaptho[1,2d]-thiazolin-2-ylidene)-2-methyl propenyl]naptho[1,2d]thiazolium bromide, made by Eastman Kodak, Rochester, New York).

Additional adenoviral constructs were also made that contain coding regions for OPN and DMP1. These constructs also can be used to express OPN and DMP1 in various cell types. It is also believed that SIBLINGS proteins, such as OPN and DMP1, also will be found to be postranslationaly modified such as has been seen in the case of BSP. Hence, the adenoviral vectors that are used to produce OPN and DMP1 can be used to produce different varieties of postranslationaly modified OPN and DMP1 by simply infecting different cell types. The expressed OPN and DMP1 can then be used in various assays for controls and to generate antibodies.

Transformation of normal fibroblasts with the BSP encoding vector (~ 100,000,000 cells on a total of eight 15 cm plates) allowed for the purification 20-40 mg of human BSP. Amino terminal sequencing verified that the amino-terminus corresponds to that of human BSP. Additionally, LF-83 bound to the product, hence the carboxy-terminus of the recombinant BSP is complete at least through amino acid 295 (of 317).

Furthermore, it is believed that other primary cell lines, as well as other cancer cell lines, transformed with the vector described above produce one or both sub-populations of BSP in cell culture. Several cancer cell lines are readily available from ATCC and other sources (for example: MCF-7, CRL-1435, CRL-1470, SAOS-2, MG-63 etc.).

### G. Uses Of Specific Binding Agents Directed Against The Relatively Acidic BSP

BSP specific binding agents, as well as other SIBLINGS protein specific binding agents, such as those made by the methods described herein, can be used for immunostaining of biopsies and sections of various tumor types, which can assist in diagnosing and determining the severity of disease. BSP levels may also be used to determine possible treatments that would be most suitable for a particular patient. For example, a very strong BSP positive breast cancer biopsy can be used as an indicator that more aggressive treatment is required following surgery (such as adjuvant chemotherapy or radiation therapy). Furthermore, monoclonal antibodies to human BSP may also be useful as immunostaining agents for determining that an otherwise inconclusive biopsy is, in fact, cancer.

Examples of BSP specific binding agents are the 13 monoclonal antibodies that were produced through the use of a synthetic 27-amino acid peptide (256T-282N). Of the 13 monoclonal antibodies produced 4 are IgG type and 9 are IgM type. This peptide was derived from the human BSP sequence, and it was designed such that it contained five tyrosine sulfates and was conjugated to a carrier protein through an amino terminal Cys (keyhole limpet hemocyanin, KLH). Monoclonal antibodies from mice injected with this construct were selected if they bound to 1) the same peptide conjugated to a different carrier protein (bovine serum albumin); 2) the same peptide, but without the tyrosines sulfation conjugated to the second carrier protein; and 3) recombinant BSP made from human marrow fibroblasts infected with the virus construct described above.

### H. Use Of Specific Binding Agents That Bind To The Portion Of BSP That Does Not Contribute To Increased Activity

This method allows for the detection of the relatively acid BSP. The BSP/Factor H complex is disrupted and the decomplexed BSP is allowed to bind to an anti-BSP antibody coated plate or bead that specifically binds to regions of BSP that are not involved in increased acidity. (Examples of such regions are epitopes with the domains used for LF-100 or LF-125, but many other likely areas can be easily chosen.) Then a second binding agent can be added, such as an antiserum, lectin, or other chemical reagent (including those commercially available) which can detect various chemical groups that may contribute to the increased relative acidity of the BSP in cancer patient serum (phosphoserine, phosphothreonine, sulfated tyrosine, various sialic acids, various sulfated sugar groups, etc.). This second binding agent binds to the BSP that was previously bound by the first antiserum. The second antiserum or chemical reagent is then quantified using standard direct or indirect methods. This approach allows development of a quantitative assay more specific for the relatively acidic BSP without necessarily relying on a single antibody system that is specific to the BSP protein. For example, if the increased acidity is determined to be caused by a sialic acid group the BSP can be trapped by an antiserum, such as LF-100 or a monoclonal antibody devised to that domain, and then the sialic acid can be detected and quantified. The post-translational modification itself may be common in many proteins in the serum (or urine etc.), however the detection of the modification on the captured BSP makes the assay useful for the detection of a disease.

### IV. Inhibition of Complement Cascade via Expression of SIBLINGS Proteins

### A. Results

The key role of Factor H is the regulation of complement and alternate complement activity. Factor H can also be 'hijacked' by invading organisms to permit pathogen evasion of complement mediated lysis. Pathogens such as *Streptococcus pyogenes* (Kotarsky et al., *J. Immunol.* **160(**7):3349-3354, 1998; Jarvis and Vedros, *Infect. Immun*. **55**(1):174-180, 1987), *Neisseria gonorrhoeae* (Ram et al., *J. Exp. Med.* **188**(4):671-680, 1999; Ram et al., *J. Exp. Med.,* **187**(5):743-752, 1998; Diaz and Sim, *J. Immun*. **80**(3):473-482, 1995), and *Echinococcus granulosus* (Diaz and Sim, *J. Immun.* **158**(8):3779-3786, 1997; Wiles et al., *J. Mol. Biol*. **272**(2):253-265, 1997) bind Factor H to their cell surface and are resistant to complement-mediated cell lysis. In addition, molecular mimicry of Factor H occurs when a pathogen makes a protein that is similar in sequence to Factor H to defend against attack by the host complement system as described in Vaccinia virus (Isaacs et al., *Proc. Natl. Acad. Sci.* **89**(2):628-632, 1992; Huemer et al., *Immunology* **79**(4):639-647, 1993), Herpes simplex virus (Donelson et al., *Molecular Biol.* **91**(1):51-66, 1998), and Trypanosoma cruzi (Hall and Joiner, *J. Eukaryot Microbiol.* **40**(2):207-213, 1993; Kipnes and daSilva, *Braz. J. Med. Biol. Res.* **22**(1):1-16, 1989; Ryden et al., *Eur. J. Biochem.* **184**(2):331-336, 1989). The following data demonstrates that BSP, DMP1, and OPN play a similar role in tumor cell complement evasion.

The first model system employed was a murine erythroleukemia (MEL) cell line which when incubated with normal human serum can be readily assayed for alternate complement pathway (ACP)-mediated cell lysis (Varnet et al., *Curr. Opin. Cell Biol.,* **8**(5):724-730, 1996). Cell survival was measured by both trypan blue dye exclusion and by thiazolyl blue (MTT) reduction by living mitochondria. Titration with dilutions of normal human serum and time courses were carried out to define optimal incubation conditions. At 1:10 dilution, human serum totally lysed the MEL cells as measured by both assay systems (Fig. 8A). The addition of purified recombinant BSP to MEL cells followed by treatment with normal human serum completely protected the cells from lysis (Fig. 8B). Treatment of MEL cells with recombinant OPN (Fig. 8B) or DMP1 also conferred protection from ACP-mediated cell lysis. The protection of MEL cells from alternate complement pathway-mediated cell lysis by BSP, OPN, and DMP1 exhibited dose related responses.

The mechanism of protection from complement-mediated lysis was investigated. Preincubation of MEL cells with rBSP in which the RGD sequence had been mutated to KAE completely removed the protective affects of this protein (Fig. 9). Furthermore, preincubation of MEL cells with either GRGDS (SEQ ID NO: 10) peptide or an αVβ3 antibody (which blocks that integrin's binding activity) negated the protective effect of rBSP. Up-regulation of the αVβ3 integrin has also been shown to correlate with invasive potential (Culty et al., *J. Cell Biol.* **111**(6 Pt 1):2765-2774, 1990). For OPN, pretreatment with GRGDS (SEQ ID NO: 10) peptide or the αVβ3 antibody reduced cell survival, though the magnitude of reduction was not as great as that seen for BSP (Fig. 9B). OPN has multiple potential receptors including αVβ1,β3, β5-containíng integrin receptors as well as CD44, a receptor implicated in attachment, homing and aggregation of lymphocytes as well as neoplastic cells. Pretreatment of MEL cells with hyaluronan, a natural ligand for CD44 (Faulk and Temple, *Nature* **262**(5571):799-802, 1976), as well as with an anti-CD44 antibody also reduced the protective effect of added rOPN (Fig. 9). Treatment of MEL cells with a pre-formed complex of either rBSP-Factor H or rOPN-Factor H abolished the protection from complement-mediated lysis. For BSP, this is consistent with immunoprecipitation data that indicates that the RGD moiety is inaccessible in the solution phase Factor H complex. Hence, both of these proteins are believed to be entirely masked by Factor H shortly after being secreted by a cell.

Additional assays were conducted to determine if DMP1 could also confer protection against complement. DMP1 was found to protect the cells from complement-mediated lysis. Preincubation of the cells with antisera to either αVβ3 integrin or CD44 diminished the DMP1 protection and together they abolished it (p> 0.001). Addition of the synthetic GRGDS (SEQ ID NO: 10) peptide diminished but did not abolish the protective properties of DMP1. DMP1 appears to protect the cells from attack by complement by bridging the major complement control protein, Factor H, to cell surface integrins through its RGD motif and through CD44.

To verify that the protective effect of BSP and OPN is exhibited in human cancer cells. MCF-7 breast cancer cells selected for non-adherent growth and U-266 myeloma cells were used in the alternate complement pathway cell lysis assay with guinea pig serum as the source of complement activity. Both cell types exhibited enhanced survival and protection from complement-mediated cell lysis when rBSP or rOPN were present (Fig 10). Increasing concentration of guinea pig serum led to decreasing cell viability, while the pretreatment with either 10 µg/mL rBSP or rOPN resulted in increased cell viability.

Collectively the above described results show that DMP1, OPN, and BSP bridge Factor H to integrins and inactivate the destructive complement pathway. Theses results also confirm that OPN and DMP1 bind to CD44. Furthermore, it is likely that other members of the SIBLINGS family will have the same biological activity.

### B. Experimental Procedures

### 1. Reagents

Rabbit Anti-human BSP antibodies LF-83, LF-100, LF-119, LF-120, LF-125 have been previously described (Fisher et al., *Bacta Orthop. Scand. Suppl.* **266**(1):61-65, 1995). Rabbit Anti-BSP peptide-derived antibody LF-142 and mouse monoclonal antibodies LFmAb-11, LFmAb-2, LFmAb-12, and LFmAb-13 were raised against the sequence EY*EY*TGVNEY*DNGY*EIY*ESENGEP (amino acids 258 - 285; SEQ ID NO: 9) conjugated to horseshoe crab hemocyanin, where the Y* denotes tyrosine-sulfates. Normal human serum, purified human complement Factor H protein and mouse monoclonal antibody against Factor H were obtained from Quidel Corporation (San Diego, CA). Polyclonal antibodies against CD-44 and a "functional" antibody against αVβ3 were obtained from Chemicon Co. (Temecula, CA). Synthetic purified glycine-arginine-aspartate-serine peptide (GRGDS; SEQ ID NO: 10) was obtained from Calbiochem-NovaBiochem Corp. (La Jolla, CA). Pre-immune serum, human serum adsorbed goat anti-rabbit IgG conjugated to horse radish peroxidase (HRP) as well as goat anti-mouse IgG conjugated to HRP were obtained from Kirkegaard & Perry (Gaithersburg, MD). HRP-conjugated strepavidin and sulfosuccinimidobiotin were obtained from Pierce Chemical Co. (Chicago, IL). Alpha-Minimal Essential Medium (MEM), Dulbecco's Modified Essential Medium (DMEM), RPMI 1640, Eagle's Minimal Essential Medium (EMEM), Earle's Balanced salt solution, Hank's balanced salt solution and heat inactivated fetal bovine serum (FBS) were obtained from BioFluids, Inc. (Rockville, MD).

### 2. Western Blotting

Samples diluted in gel sample buffer were resolved by SDS-PAGE 4 - 20 % gradient gels (Novex Corg, San Diego, CA), transferred to nitrocellulose following standard conditions (Towbin et al., *Proc. Natl. Acad. Sci.* **76**(9):4350-4354, 1979). Nitrocellulose membranes were rinsed with Tris-buffered saline (0.05 M Tris-HCl, pH 7.5, 0.15 M NaCl) containing 0.05 % Tween 20 (TBS-Tween). After a 1 hour incubation in blocking solution (TBS-Tween + 5 % non-fat powdered milk) at room temperature on rotary shaker, 1:5000 primary antibody was added and incubated overnight at 4°C. The nitrocellulose sheet was washed in TBS-Tween four times for 5 minutes each time with TBS-Tween and then 1:50,000 HRP conjugated second antibody in TBS-Tween + 5 % milk was added and incubated for 2 hours at room temperature. Following removal of the second antibody solution the membrane was washed three times with TBS-Tween and rinsed a final time in enzyme substrate buffer for 5 min. Enhanced chemiluminescence reagents were employed for signal detection (Pierce Chemical Co., Chicago, IL) with x-ray film.

### 3. High Performance Liquid Chromatography

A Shimadzu LC10AS binary gradient system was employed for chromatographic separations. A 1.0 mL packed volume ToyoPearl QAE (TosoHaas, Montgomeryville, PA) column was pre-equilibrated with 0.05 M sodium phosphate, pH 7.4, containing 50 % fresh formamide. A linear salt gradient increasing to 2.0 M NaCl at 2.0 mL/minute flow rate over 50 minutes was employed collecting 1 minute fractions. Size exclusion chromatography utilized a 1.0 x 30 cm Superose 6 column (Amersham Pharmacia, Piscataway, New Jersey) equilibrated in 0.05 M sodium phosphate, pH 7.4, containing 50 % fresh formamide at a flow rate of 0.5 mL/min. The column was calibrated using commercially available protein standards of known molecular weight (Amersham Pharmacia, Piscataway, New Jersey).

### 4. Direct ELISA

Greiner high-binding 96 well plates were coated with 100 µl HPLC fractions overnight at 4°C. Plates were washed three times (5 minutes each) with TBS-Tween and exposed to 100 µl of 1:2000 primary antibody for 1 hour at room temperature. Plates were washed three times and exposed to 100 µl of 1:2000 HRP-conjugated goat anti-rabbit IgG. Following a one hour incubation at room temperature, plates were washed again three times with TBS-Tween and color was developed using 3,3', 5,5', tetramethylbenzidine and H₂O₂ for 10 minutes at room temperature. Color development was stopped by the addition of 25 µL 1N H₂SO₄ and analyzed at 450 nm.

### 5. Immunoprecipitation

Aliquots of normal human serum diluted 1:100 in immunoprecipitation buffer (0.1 M Tris, pH 7.2, 0.15 M NaCl, .05% Tween 20, and 1% aprotinin) were incubated sequentially with 0.1 mL each of (a) Protein G agarose (Kirkegaard & Perry; Gaithersburg, MD), (b) normal rabbit serum IgGs bound to Protein G agarose; (c) rabbit anti-BSP antibodies bound to Protein G agarose. After incubation for 1 hour at 4°C the reaction was terminated by centrifugation at 10,000 x *g* for 5 minutes and the supernatant taken to the next immunoprecipitation. The first two incubations removed proteins binding non-specifically to agarose and to normal rabbit serum. The anti-BSP incubation immunoprecipitate was dissolved in gel sample buffer and analyzed by 4 - 20 % gradient SDS-PAGE.

### 6. Production of Recombinant SIBLINGS Proteins

Adenoviral constructs encoding BSP (rBSP), DMP1 (rDMP1), and OPN (rOPN) were generated by subcloning BSP (Fisher et al., *J. Biol. Chem.* **265**(4):2347-2351, 1990), BSP-KAE or OPN (Young et al. *Genomics* **7**:491-502, 1990; and Kiefer et al., *Nucleic Acid Research* **17**:3306, 1989) cDNA into high expression, replication-deficient adenovirus (Ad5) using EF-1 (BSP) and CMV (BSP-KAE, OPN, DMP1) promoters, respectively. The RGD- > KAE constructs were made using *in situ* mutagenesis and the entire insert checked for fidelity. Adenoviruses were plaque-selected and propagated on HEK 293 cells (ATCC #CRL1573). Cells were harvested when cytopathic effects were present and lysed by 5 freeze-thaw cycles. Cellular debris was removed and viral particles were purified by banding twice on CsCl. After dialysis in Tris/ MgCl₂ Glycerol Buffer at 4°C, viruses were aliquoted and frozen at -70°C. Evaluation of viral titers was carried out by plaque formation of virus dilutions on HEK293 cells (Becker et al., *Methods Cell Biol.* **43**(Pt A):161-189, 1994). Typically ∼2-4 x 10¹¹ pfu/mL were obtained from one viral preparation. Recombinant proteins were generated by infecting subconfluent normal human marrow stromal fibroblasts with 10,000 pfu/cell. Cells were maintained in alpha-MEM, 20 % FBS and 100 IU/mL penicillin/ 100 µg/mL streptomycin in a humidified atmosphere of 95 % air and 5% CO2 at 37°C. Medium was changed to serum-free conditions after 48 hours. Subsequently, medium was collected every 24-48 hours and frozen at -70°C. Aliquots were assayed by SDS-Gel electrophoresis and Western blot for BSP, DMP1, and OPN expression. Expression was found to be at highest levels at ~168 hours post infection. The proteins were purified by routine column chromatography. Native BSP, BSP-KAE, DMP1, and OPN proteins were purified by diluting medium from normal human marrow stromal fibroblast cells 1:1 with 40 mM phosphate buffer pH 7.4 and loading directly on a 5.0 x 2.0 cm column packed with ToyoPearl TSK QAE resin. A linear salt gradient to 2.0 M NaCl was employed to purify the BSP, DMP1, and OPN to ~95% purity as measured by SDS PAGE.

### 7. Biotinylated BSP

12 µg of recombinant human BSP was dissolved in 50 µL of PBS. 2 µL of a fresh 1 mg/mL solution of NHS-LC-Biotin (Pierce Chemical Co., Chicago, IL) was added and the reaction incubated at room temperature for 45 min. The unreacted biotin was removed by repeated washing with TBS-Tween and centrifugation in a Microcon 30 (Amicon, Beverly, MA). A final volume of 50 µL was retained.

### 8. Alternate Complement Mediated Cell Lysis Assay

Murine erythroleukemia (MEL) cells (a gift of Dr. Marilyn Farquhar, Univ. CA San Diego.) grown in DMEM containing 10 % fetal bovine serum and 4 mM glutamine were rinsed three times with gelatin veronal buffer with Mg₂⁺ and EGTA (GVB-MgEGTA, Sigma, St. Louis, MO). Cells were resuspended in GVB-MgEGTA at a density of 5 x 10⁶cells/mL and incubated at 37°C with different concentrations of normal human serum diluted in GVB-MgEGTA. After 2 h, cells were harvested for trypan blue exclusion assay by removing a 50 µL aliquot, incubating for 15 minutes in 0.4 % trypan blue, and counting viable cells under an inverted microscope. The thiazolium blue assay was carried out at identical serum dilutions by incubating a 50 µL aliquot of the cell suspension in an equal volume of 1 mg/mL thiazolyl blue (MTT) for 45 min. Cell viability was determined spectrophotometrically by absorbance at 560 nm. Cells in GVB-MgEGTA buffer were pre-incubated with 10 µg of either rBSP, rDMP1, or rOPN in 1 mL for 10 minutes at 37°C. Normal human serum collected for good complement activity was then added at a dilution of 1:10 and the cells returned to 37°C for 2 hours and cell viability was determined by trypan blue-exclusion and MTT assays. For the assay of human cancer cell lines, loosely adherent MCF-7 cells were selected for by sequential growth in EMEM with 2 mM L-glutamine and Earle's balanced salt solution adjusted to contain 1.5 g/L sodium bicarbonate, 0.1 mM non-essential amino acids and 1.0 mM sodium pyruvate and 10%; fetal bovine serum, while U-266 cells were cultured in RPMI 1640 medium containing 15 % fetal bovine serum. Cells were collected by centrifugation and rinsed three times with GVB-MgEGTA buffer and subsequently treated exactly as for MEL cells, substituting guinea pig serum (Sigma, St. Louis, MO) for human serum.

### EXAMPLES

### EXAMPLE 1

### Isolation of SIBLINGS Proteins and the Relatively Acidic form of BSP

### A. Disruption of SIBLINGS/Factor H Complex

A variety of methods of disrupting protein-protein interactions are well known in the art. Such methods can involve treatment with harsh chemicals and/or severe temperatures. Typically protein complexes such as the SIBLINGS/Factor H complex can be disrupted through the use of reducing agents, denaturants and/or increased temperatures. The SIBLINGS/Factor H complex also can be disrupted using a combination of reducing agents, denaturants, and increased temperatures. The particular method of disrupting the complex is not critical, as long as the complex is disrupted.

Reducing agents are agents that are capable of donating hydrogen atoms, and as such serve to disrupt disulfide bonds between amino acids. These agents particularly disrupt disulfide bonds that commonly link two proteins or portions of the same protein together. Commonly used reducing regents are β-mercaptoethanol, 1,4-dithiothreitol (DTT) and trialkyl phosphines. Additionally, the ability of a reducing agent to facilitate the separation of SIBLINGS proteins from Factor H will be increased by increasing the temperature at which the solution containing the reducing agent and SIBLINGS/Factor H complex is incubated.

Denaturants serve to relax the conformational structure of proteins, and there are a variety of denaturants that can be employed to effectuate the separation of SIBLINGS proteins from Factor H. Examples of denaturants that are particularly useful in conjunction with ion exchange columns are urea and formamide. However, denaturants such as guanidine hydrochloride or guanidine thiocyanate also may be useful for separating Factor H from SIBLINGS proteins in non-ion exchange based methods. The ability of the denaturant to relax the protein will also be enhanced by increasing the temperature at which the sample containing the SIBLINGS/Factor H complex is incubated.

SIBLINGS proteins, such as BSP can also be separated from Factor H by adding competitive binding agents to the sample containing the BSP/Factor H complex. Upon addition to the sample, a competitive binding agent binds to Factor H and displaces BSP. Ideally, the agent used binds to Factor H selectively and not to BSP. Examples of such competitive binding agents are, recombinant fragments of BSP, sialic acids, and synthetic BSP fragments.

Since a variety of methods can be employed to separate Factor H from SIBLINGS proteins, the invention also provides a method of determining when the proteins have become separated. As mentioned above, when Factor H is complexed to SIBLINGS proteins, the relatively acidic BSP, or to the relatively non-acidic BSP, it masks the ionic charge normally associated with protein. Therefore, when the complex is placed on an anion exchange column, it readily flows through the column and the complex fails to bind. Hence, after treatment with one or more denaturants and reducing agents, the sample can be tested for the its ability to bind to an anion exchange column. This assay can confirm whether separation of Factor H from the complex has occurred.

It is also possible to simultaneously confirm that a SIBLINGS protein has been separated from Factor H and measure the SIBLINGS protein level using column chromatography. This can be done by first mixing a sample with an anion exchange resin under conditions that disrupt the Factor H/SIBLINGS complex and then allowing the SIBLINGS protein to bind to the anion exchange resin. The uncomplexed SIBLINGS protein will then bind to the resin, denaturants and other contaminants can be washed away, and the SIBLINGS-resin complex can then be directly assayed using SIBLINGS protein specific binding agents.

Additionally, the column conditions can be modified such that either the relatively acidic BSP or the relatively non-acidic BSP remains bound. Thus, BSP specific binding agents used to detect the BSP resin complex will actually be detecting either the relatively non-acidic BSP or the relatively acidic BSP.

### B. Separation of the Total Bound SIBLINGS protein from Factor H and the Separation of the Relatively Non-Acidic BSP from the

### Relatively Acidic BSP

The separation of the total bound SIBLINGS protein from Factor H, and the subsequent separation of the relatively acidic and the relatively non-acidic BSP, can be accomplished using multi-step procedures that include various chromatographic techniques (Robyt and White, Biochemical Techniques Theory and Practice, Waveland Press, Inc, 1990). Such multi-step procedures will commonly operate to separate the proteins by exploiting their different physical characteristics. There are many different methods that can be used to effectively separate SIBLINGS protein from Factor H, as well as to separate the relatively acidic BSP from the relatively non-acidic SIBLINGS protein. Therefore, the following discussion provides a broad description of various chromatography techniques that can be used in either a single step process or in multi-step process. Furthermore, it is possible that a single variety of chromatography be repeatedly used to effectuate either the isolation of the relatively acidic SIBLINGS protein, or the entire population of SIBLINGS protein present in the sample.

### 1. Column Chromatography

One method of isolating the SIBLINGS protein and/or relatively acidic BSP is through adsorption chromatography. This method involves the exploitation of the differential affinity of the protein for the medium in the column, and the affinity of the protein to the eluting solvent. An example of a suitable medium for use in the column is hydroxyapatite, which is a crystalline form of calcium phosphate. This medium tends to adsorb acidic proteins (such as BSP), which can subsequently be eluted with phosphate ions, which have a high affinity for the calcium ions present in the hydroxyapatite.

Ion exchange chromatography can also be used to isolate SIBLINGS protein, and/or the relatively acidic BSP. This approach is a variation of adsorption chromatography, in which the solid adsorbent has charged groups chemically linked to an inert solid. The ionic charge of the SIBLINGS protein then causes the SIBLINGS protein to become linked to the chemically charged groups on the solid support, and the SIBLINGS protein is subsequently released by passing a solution containing an ion gradient over the solid adsorbent. Examples of solid supports that can be used in this technique include DEAE-cellulose, DEAE-Sephadex, DEAE-Bio-Gel, DEAE cellulose, DEAE sepharose, DEAE sephacryl, DEAE trisacryl, Q sepharose, ecteola cellulose, QAE cellulose, express ion exchanger Q, PEI cellulose, and other polystyrene-based anion exchangers.

Another type of adsorption chromatography which can be used to isolate SIBLINGS protein and/or the relatively acidic form of BSP is affinity chromatography. This method involves covalently linking to an inert solid support a ligand which has binding affinity for the protein of interest, which in this case is either SIBLINGS protein or the relatively acidic BSP. Commonly, the ligand is a specific binding agent, which selectively binds the protein of interest as it contacts the solid support. Alternatively, the SIBLINGS protein and/or the relatively acidic BSP can be separated based upon hydrophobicity. For example, alkyl chains may be linked to the inert support, to supply sites for hydrophobic bonding interactions. The eluting solvent then contains a hydrophobic gradient.

High performance liquid chromatography provides yet another method that can be utilized to isolate SIBLINGS protein and/or the relatively acidic BSP. All of the major classes of chromatographic separations are possible using this method, for example, adsorption, liquid-liquid partition, ion exchange, exclusion, and affinity chromatography can be used in conjunction with HPLC. Additionally, the HPLC method allows for reversed phase and ion pair partition. Reversed phase partition allows for quick elution using a non-polar stationary phase and a polar mobile phase, while ion pair partition involves pairing the charged polar substance with its counter ion to create a less polar species that then flows through the column.

### 2. Electrophoresis

Electrophoresis is a well-established technique for the separation and analysis of mixtures by differential migration and separation of molecules in an electric field, based on differences in mobility through a support. Many different forms of electrophoresis have been developed to permit the separation of different classes of compounds. These forms include paper and cellulose acetate electrophoresis, thin-layer electrophoresis, gel electrophoresis, immunoelectrophoresis and isoelectric focusing. Paper electrophoresis operates best for the separation of relatively low molecular weight protein molecules, and gel electrophoresis is a much better candidate for use in isolating the relatively acidic BSP. There are a variety of different matrices available for forming gels, but matrices with a relatively smaller pore size are most suitable for the separation of two similar molecules. Furthermore, proteins may be separated based upon size by conducting the electrophoresis under disassociating conditions, for example using SDS. The SDS relaxes the protein and masks the ionic charge of the protein, which leaves protein size as the only distinguishing characteristic. Hence in the case of the relatively acidic BSP, SDS would not be used for separating the two populations of BSP (the relatively acidic versus the relatively non-acidic), because the separation will depend on the ionic charge that would be masked by SDS.

Separation can also be achieved using immunoelectrophoresis, in which proteins are separated on a gel based upon their charge-to-mass ratio and their antigenicity. This is done by first separating the proteins on a gel, and then adding an antibody specific for the protein of interest to a well created in the gel, and allowing the antibody to diffuse through the gel. Precipitate then forms in regions in which the antibody reacts with the protein.

The relatively acidic form of BSP can also be isolated through the use of isoelectric focusing. In this type of electrophoresis the protein is placed on a substrate having a pH gradient, and it will move under the influence of an electrical field until it reaches an equilibrium point with the pH gradient.

Finally, the characterization of the relatively acidic BSP population indicates that other SIBLINGS proteins may exist in multiple forms. Moreover, SIBLINGS proteins may also have relatively acidic population and these populations can be detected using the above described techniques.

### EXAMPLE 2

### Identifying SIBLINGS Proteins with Specific Binding Agents

A SIBLINGS protein can be identified using specific binding agents. These specific binding agents may bind for example, BSP generally, or they may selectively bind to the relatively acidic BSP, the relatively non-acidic BSP, or the exposed portion of SIBLINGS protein in the SIBLINGS/Factor H complex. The latter specific binding agent will be particularly useful for more accurately determining the total amount of SIBLINGS protein in the biological specimen without the need to separate the SIBLINGS protein from the Factor H.

### A. Antibodies

Antibody preparations prepared according to these protocols are useful in quantitative immunoassays such as ELISAs, Western blotting and RIAs, to determine concentrations of antigen-bearing substances in biological samples; they are also used semi-quantitatively or qualitatively to identify the presence of antigen in a biological specimen.

### 1. Production of an Antibody to SIBLINGS Proteins

Monoclonal or polyclonal antibodies may be produced to SIBLINGS proteins or portions thereof. Optimally, antibodies raised against a particular SIBLINGS protein, such as BSP will specifically detect BSP. That is, antibodies raised against BSP would recognize and bind BSP and would not substantially recognize or bind to other proteins found in human cells. The determination that an antibody specifically detects a SIBLINGS protein, such as BSP is made by any one of a number of standard immunoassay methods; for instance, the Western blotting technique (Sambrook et al., In Mol. Clon.: A Lab. Man., Cold Spring Harbor, N.Y., 1989). To determine that a given antibody preparation (such as one produced in a mouse against the human BSP) specifically detects BSP by Western blotting, serum is extracted from a subject (for example, to obtain lymphocytes) and electrophoresed on a sodium dodecyl sulfate-polyacrylamide gel. The proteins are then transferred to a membrane (for example, nitrocellulose or nylon) by Western blotting, and the antibody preparation is incubated with the membrane.

After washing the membrane to remove non-specifically bound antibodies, the presence of specifically bound antibodies is detected by the use of an anti-mouse antibody conjugated to an enzyme such as alkaline phosphatase; application of the substrate 5-bromo-4-chloro-3-indolyl phosphate/nitro blue tetrazolium results in the production of a dense blue compound by immuno-localized alkaline phosphatase. Antibodies that specifically detect SIBLINGS protein will, by this technique, be shown to bind substantially only the SIBLINGS protein band (which will be localized at a given position on the gel determined by its molecular weight). Non-specific binding of the antibody to other proteins may occur and may be detectable as a weak signal on the Western blot. The non-specific nature of this binding will be recognized by one skilled in the art by the weak signal obtained on the Western blot relative to the strong primary signal arising from the specific antibody-SIBLINGS protein binding.

Antibodies that specifically bind to SIBLINGS protein belong to a class of molecules that are referred to herein as specific binding agents. Specific binding agents that are capable of specifically binding to SIBLINGS protein may include polyclonal antibodies, monoclonal antibodies (including humanized monoclonal antibodies) and fragments of monoclonal antibodies such as Fab, F(abγ)2 and Fv fragments, as well as any other agent capable of specifically binding to SIBLINGS proteins.

Depending on the desired specificity of the antibody, different forms of a SIBLINGS protein can be used as immunogens for injection into subjects for the creation of antibodies. To create antibodies which recognize the exposed portion of a SIBLINGS protein when it is bound to Factor H, the entire SIBLINGS/Factor H complex can be used as an immunogen. Similarly, if the creation of an antibody specific for the relatively acidic BSP is desired, the relatively acidic BSP can be isolated and used to inoculate a mouse. Additionally, as mentioned below, fragments of the relatively acidic BSP or fragments of the exposed portion of BSP can be used as immunogens for the creation of specific binding agents.

Regardless of the particular form of SIBLINGS protein used to create the immunogen the concentration of protein in the final preparation is adjusted, for example, by concentration on an Amicon filter device, to the level of a few micrograms per milliliter. Alternatively, as previously mentioned, peptide fragments of the SIBLINGS proteins may be utilized as immunogens. Such fragments may be chemically synthesized using standard methods, or may be obtained by cleavage of the whole SIBLINGS protein followed by purification of the desired peptide fragments. Peptides as short as 3 or 4 amino acids in length are immunogenic when presented to the immune system in the context of a Major Histocompatibility Complex (MHC) molecule, such as MHC class I or MHC class II. Accordingly, peptides comprising at least 3 and preferably at least 4, 5, 6 or more consecutive amino acids of the disclosed SIBLINGS protein amino acid sequences may be employed as immuogens to raise antibodies.

Because naturally occurring epitopes on proteins frequently include amino acid residues that are not adjacently arranged in the peptide when the peptide sequence is viewed as a linear molecule, it may be advantageous to utilize longer peptide fragments from the SIBLINGS protein amino acid sequence in order to raise antibodies. Thus, for example, peptides that comprise at least 10, 15, 20, 25 or 30 consecutive amino acid residues of the BSP amino acid sequence may be employed. Monoclonal or polyclonal antibodies to the intact BSP or peptide fragments of this protein may be prepared as described below.

### 2. Monoclonal Antibody Production by Hybridoma Fusion

Monoclonal antibody to epitopes of BSP identified and isolated as described can be prepared from murine hybridomas according to the classical method of Kohler and Milstein, Nature **256**:495, 1975, or derivative methods thereof. Briefly, a mouse is repetitively inoculated with a few micrograms of the selected protein over a period of a few weeks. The mouse is then sacrificed, and the antibody-producing cells of the spleen isolated. The spleen cells are fused by means of polyethylene glycol with mouse myeloma cells, and the excess unfused cells destroyed by growth of the system on selective media such as aminopterin (HAT media). The successfully fused cells are diluted and aliquots of the dilution placed in wells of a microtiter plate, where growth of the culture is continued. Antibody-producing clones are identified by detection of antibody in the supernatant fluid of the wells by immunoassay procedures, such as ELISA, as _ originally described by Engvall, *Enzymol*. **70**:419, 1980, and derivative methods thereof. Selected positive clones can be expanded and their monoclonal antibody product harvested for use. Detailed procedures for monoclonal antibody production are described in Harlow and Lane, Antibodies, A Lab. Man., Cold Spring Harbor, N.Y., 1988. In addition, protocols for producing humanized forms of monoclonal antibodies (for therapeutic applications) and fragments of monoclonal antibodies are known in the art.

### 3. Polyclonal Antibody Production by Immunization

Polyclonal antiserum containing antibodies to heterogeneous epitopes of a single protein can be prepared by immunizing suitable animals with the expressed protein, which can be unmodified or modified to enhance immunogenicity. Effective polyclonal antibody production is affected by many factors related both to the antigen and the host species. For example, small molecules tend to be less immunogenic than others and may require the use of carriers and adjuvant. Also, host animals vary in response to site of inoculations and dose, with both inadequate or excessive doses of antigen resulting in low titer antisera. Small doses (ng level) of antigen administered at multiple intradermal sites appear to be most reliable. An effective immunization protocol for rabbits can be found in Vaitukaitis et al., *J. Clin. Endocrinol. Metab.* **33**:988-991, 1971.

Booster injections can be given at regular intervals, and antiserum harvested when antibody titer thereof, as determined semi-quantitatively, for example, by double immunodiffusion in agar against known concentrations of the antigen, begins to fall. See, for example, Ouchterlony et al. , In Handbook of Experim. Immunol., Wier, D. ed., ch. 19. Blackwell, 1973. Plateau concentration of antibody is usually in the range of 0.1 to 0.2 mg/mL of serum (about 12 M). Affinity of the antisera for the antigen is determined by preparing competitive binding curves, as described, for example, by Fisher, 1980.

### 4. Antibodies Raised by Injection of SIBLINGS protein cDNA

Antibodies may also be raised against SIBLINGS proteins by subcutaneous injection of a DNA vector that expresses the SIBLINGS protein in laboratory animals, such as mice. Delivery of the recombinant vector into the animals may be achieved using a hand-held form of the Biolistic system (Sanford et al., *Paniculate Sci. Technol.* **5**:27-37, 1987) as described by Tang et al., *Nature* (London) **356**:152-154, 1992. Expression vectors suitable for this purpose may include those that express a cDNA encoding a SIBLINGS protein under the transcriptional control of either the human β-actin promoter or the cytomegalovirus (CMV) promoter. Methods of administering naked DNA to animals in a manner to cause expression of that DNA in the body of the animal are well known and are described, for example, in U.S. Patent Nos. 5,620,896 (DNA vaccines against rotavirus infection), 5,643,578 (Immunization by inoculation of DNA transcription unit) and 5,593,972 (Genetic immunization), and references cited therein.

### 5. Antibody Fragments

Antibody fragments may be used in place of whole antibodies and may be readily expressed in prokaryotic host cells. Methods of making and using immunologically effective portions of monoclonal antibodies, also referred to as antibody fragments, are well known and include those described in Better and Horowitz, *Advances in Gene Technol. : The Mol. Biol. Of Immune Disease & the Immune Response* (ICSU Short Reports), 1989; Glockshuber et al., *Biochem.* **29**:1362-1367 (A Comparison of Strategies to Stabilize Immunoglobulin Fᵥ Fragments), 1990; and U.S. Patent Nos. 5,648,237 (Expression of Functional Antibody Fragments), 4,946,778 (Single Polypeptide Chain Binding Molecules), and 5,455,030 (Immunotherapy Using Single Chain Polypeptide Binding Molecules), and references cited therein.

### 6. Humanized Antibodies

Humanized monoclonal antibodies are preferred in clinical applications. Methods of making humanized monoclonal antibodies are well known, and include those described in U.S. Patent Nos. 5,585,089 (Humanized Immunoglobulins), 5,565,332 (Production of Chimeric Antibodies--A Combinatorial Approach), 5,225,539 (Recombinant Altered Antibodies And Methods Of Making Altered Antibodies), 5,693,761 (Polynucleotides Encoding Improved Humanized Immunoglobulins), 5,693,762 (Humanized Immunoglobulins), 5,585,089 (Humanized Immunoglobulins), and 5,530,101 (Humanized Immunoglobulins), and references cited therein.

Antibody preparations prepared according to these protocols are useful in quantitative immunoassays to determine concentrations of antigen-bearing substances in biological samples; they are also used semi-quantitatively or qualitatively to identify the presence of antigen in a biological sample.

### EXAMPLE 3

### Characterizing SIBLINGS Protein Populations in Biological Specimens

In addition to the assays provided above, the present invention allows for further characterization of SIBLINGS protein populations in biological specimens, and the development of screening assays that can detect tumors, as well as abnormal bone turnover. It is additionally contemplated that tumors such as sarcomas and carcinomas, fibrosarcoma, myxosarcoma, liposarcoma, chondrosarcoma, osteogenic sarcoma, and other sarcomas, synovioma, mesothelioma, Ewing's tumor, leiomyosarcoma, rhabdomyosarcoma, colon carcinoma, pancreatic cancer, breast cancer, ovarian cancer, prostate cancer, squamous cell carcinoma, basal cell carcinoma, adenocarcinoma, sweat gland carcinoma, sebaceous gland carcinoma, papillary carcinoma, papillary adenocarcinomas, medullary carcinoma, bronchogenic carcinoma, renal cell carcinoma, hepatoma, bile duct carcinoma, choriocarcinoma, Wilms' tumor, cervical cancer, testicular tumor, bladder carcinoma, CNS tumors (such as a glioma, astrocytoma, medulloblastoma, craniopharyogioma, ependymoma, pinealoma, hemangioblastoma, acoustic neuroma, oligodendroglioma, menangioma, melanoma, neuroblastoma and retinoblastoma) may prove to express BSP and be detectable through the use of the disclosed assays.

### A. Development of Screening Assays

The invention allows for the development of screening assays that can detect other disease states which are associated with elevated SIBLINGS protein levels, and/or shifts in the population of BSP from the relatively non-acidic BSP to the relatively acidic form of BSP. This can be done by separating the SIBLINGS protein from Factor H and correlating the relative concentration of SIBLINGS protein in the test sample to the relative concentration in a control. The control in many cases is a like sample from a subject that does not have the particular malady in question. For example, a screening assay can be developed to detect abnormal bone turnover in subjects having osteoporosis or osteoarthritis, by taking a sample from the subject that is know to have the particular malady, and characterizing the SIBLINGS protein concentration using the methods provided above. This same procedure can be then be used to characterize a sample from a normal subject and subsequently a correlation can be drawn between the results from the two subjects. If present the correlation can then be used to detect the malady in other subjects.

### B. Controls For Assays In Which The Relatively Acidic BSP Is Used As A Marker

The above data shows subjects suffering with breast, prostate, thyroid, multiple myeloma and lung tumors have readily distinguishable populations of BSP. These differences have permitted the development of an assay that can be used to detect the presence or recurrence of such tumors (such as recurrence following surgical excision of a tumor). Generally, such assays include a control that allows a comparison to be made between a normal individual and the test subject. However, in some cases this control can be recombinantly expressed BSP, or the test sample can be classified as positive for a tumor based upon the ratio of the relatively non-acidic BSP to the relatively acidic BSP. The above separation methods describe different mechanisms for separating proteins based upon ionic charge and these methods can be used to provide information on the relative concentration of the relatively non-acidic form of BSP, as well as the relatively acidic form of BSP. Therefore, used in this way the assay does not require the use of an external control; rather the ratio itself can be used to indicate the presence of a tumor.

### EXAMPLE 4

### Use Of Members of the SIBLINGS Family of Proteins to Confer Protection against Complement

The above-described methods and results show that when members of the SIBLINGS family of proteins are associated with cells they can serve to protect the cells from complement mediated lysis. In the case of tumor cells it is likely that the expression of SIBLINGS proteins serves to protect the tumor from complement lysis. This protection may be especially important during the process of metastasis when tumor cells are invading other tissue such as bone.

The complement protective activity of SIBLINGS proteins can also be exploited to protect cells and/or implants from complement mediated immune responses. In the case of implants SIBLINGS proteins can be used to coat implants such as described in U.S. Patent No. 6,024,918 to Hendriks, et al. or they can be delivered from a reservoir that is placed in the body. This protection can be imparted to a cell by either coating the cell with the SIBLINGS protein, or genetically engineering a cell to express the SIBLINGS protein. Used in this way a cell that has been engineered to express a transgene is designed to also express one or more of the members of the SIBLINGS family of proteins. The co-expression of the SIBLINGS protein then facilitates the binding of Factor H to the transgenic cell. This binding results in the cell becoming protected from complement mediated lysis. Expressing the SIBLINGS protein with a membrane localization peptide could further enhance the protection conferred to the transgenic cell.

The complement protective activity of SIBLINGS proteins also could be used to provide protection against non-self cells that are introduced into a subject's body, for example by implantation of a graft. Used in this way a transgene encoding the SIBLING protein could be delivered to the graft via various vectors such as described below.

### A. Expression Cassette Transfer

A preferred method of supplying exogenous SIBLINGS proteins is by transferring a vector comprising an expression cassette to cells associated with the region of interest such that the cells express the SIBLINGS protein.

### 1. Expression Cassettes

Expression cassettes employed in the present inventive methods are of any type appropriate for cells containing the cassette to express the protein of interest. Thus, for example, an expression cassette comprises a polynucleotide encoding BSP operably linked to a promoter.

Any promoter and/or enhancer sequence appropriate for controlling expression of polynucleotides from the vector can be used in constructing an expression cassette. While such promoters/enhancer elements are well known in the art, examples of suitable promoters include prokaryotic promoters or viral promoters, (e.g., ITRs, or LTRs; immediate early viral promoters, such as herpesvirus IE promoters, or cytomegalovirus (CMV) IE promoters; or other viral promoters, such as Rous Sarcoma Virus (RSV) promoters, or Murine Leukemia Virus (MLV) promoters). Other suitable promoters are eukaryotic promoters, such as constitutively active promoters (e.g., the β-actin promoter), signal specific promoters (e.g., inducible promoters, such as a promoter responsive to TNF), or tissue-specific promoters, (e.g., those active in epidermal tissue, dermal tissue, tissue of the digestive organs such as cells of the esophagus, stomach, intestines, or colon), smooth muscles, such as vascular smooth muscles, cardiac muscles, skeletal muscles, lung tissue, hepatocytes, lymphocytes, endothelial cells, sclerocytes, kidney cells, glandular cells (e.g., those in the thymus, ovaries, testicles, pancreas, adrenals, pituitary, etc.), tumor cells, cells in connective tissue, cells in the central nervous system (e.g., neurons, neuralgia, etc.), cells in the peripheral nervous system, or other cells of interest.

Which promoter is used in a given expression cassette will depend, in part, on the choice of vector: Thus, for example, an expression cassette can comprise a native retroviral LTR promoter operably linked to a coding polynucleotide when the vector is a retroviral vector.

In addition to a promoter, an expression cassette will also contain a polynucleotide encoding the protein of interest. Preferably, the polynucleotide is a synthetic DNA, cDNA or genomic DNA fragment encoding the SIBLINGS protein or fragment thereof that imparts protection against complement mediated lysis to the cell that expresses it. Expression cassettes can also include other polynucleotides, such as a polyadenylation sequence. Also, expression cassettes can encode more than one SIBLINGS protein.

### 2. Vectors

An expression cassette for use in the present inventive methods such as those described supra is contained within a vector. Of course, in addition to the SIBLINGS protein encoding sequence, the vector can include other expression cassettes, such as, for example, cassettes for expressing a selectable marker (e.g., β-gal or a marker conferring resistance to a toxin), a pharmacologically active protein, a transcription factor, or other biologically active substances.

Any vector appropriate for transferring an exogenous expression cassette to a cell is included within the scope of the present inventive methods. Preferably, the vector is a viral vector. Examples of viral vectors employed in accordance with the present inventive method include, but are not limited to, retroviral vectors, adenoviral vectors, adeno-associated viral vectors, herpesviral vectors, SV40 viral vectors, polyoma virus vectors, pappiloma virus vectors, picnoravirus vectors, vaccinia virus vectors, or other suitable vectors.

In addition to viral vectors, any non-viral vector capable of expression upon infection of target cells can be used in the present inventive methods. Preferably, a non-viral vector is a plasmid.

The skilled artisan will be able to incorporate an expression cassette into the nucleic acid sequence of the vector. Methods for incorporating expression cassettes into viral vectors are well known in the art (see e.g., Sambrook, et al. *Molecular Cloning: a Laboratory Manual,* 2d edition, Cold Spring Harbor Press, 1989) and include direct cloning, site specific recombination using recombinases, such as the flp recombinase or the cre-lox recombinase system (reviewed in Kilby et al., Trends in Genetics, **9**:413-21, 1993), homologous recombination, and other suitable methods of constructing a recombinant vector.

### 3. Target Cells

The present inventive methods also comprise transferring an expression cassette to cells associated with the region of interest (i.e., a graft). Any suitable cells associated with the region of interest which are capable of supporting expression of the coding polynucleotide of the cassette are encompassed within the present inventive methods. Such cells can be cells *in situ*, such as cells of the graft tissue.

Following transfer of the vector comprising the expression cassette to the cells *in vitro,* the cells associated with the region are transferred to the region of interest. Transfer of cells *in vitro* to a region of interest is accomplished by known means. For example, cells can be transferred to internal tissue of a patient by methods described herein. Further methods of transferring cells containing an expression cassette are described in International Patent Application No. WO 96/00006, (Billiar et al.).

### 4. Vector Transfer

The present inventive methods comprise transferring a vector comprising an expression cassette, such as those described herein, to cells associated with the region of interest (e.g., graft). Any method of transferring the expression cassette to the cells is appropriate so long as a product of the expression cassette is produced in the cells.

### a. Non-Viral Vectors

Any means of introducing non-viral vectors into target cells, such as by direct DNA injection, electroporation, calcium-phosphate mediated cell transfection, lipofectamine, DAEA-dextran-mediated cell transfection, polybrene-mediated delivery, host cell fusion, microinjection, and polylysine-mediated cell transfection is appropriate within the present inventive context. Such methods are well known in the art, and some are described in International Patent Application No. WO 96/00006, (Billiar et al.). A preferred method of transferring the expression cassette within a nonviral vector, such as a plasmid, is via liposome-mediated transfection of the target cells, such as endothelial cells, *in vitro* or *in situ*. If the vector is transfected *in vitro,* the cells associated with the region of interest are subsequently transferred to the patient.

### b. Viral Vectors

Transfer of an expression cassette to cells associated with the region of interest where the cassette is within a viral vector is accomplished by infecting the cells with the virus. Where the virus is a retrovirus, the virus can be first transfected into an appropriate packaging cell line for generation of infectious virus. Cells associated with the region of interest can be infected *in vitro* or *in situ*. Moreover, the mode of transfer of the expression cassette does not depend on the identity of the coding polynucleotide.

### c. In vitro Delivery

Infectious viruses are used to infect cultured cells *in vitro.* The precise method for infecting target cells *in vitro* will depend upon the viral vector used; however, such methods are well known in the art. Some suitable methods are described in International Patent Application No. WO 96/00006, (Billiar et al.).

### 5. In situ Delivery

Some applications of the present inventive methods involve transfer of expression cassettes *in situ. In situ* transfer can be either *in vivo* (i.e., to a wound or to a graft following implantation), or *ex vivo* (i.e., to a graft prior to implantation). Any method of delivering the vector comprising the expression cassette to cells associated with the region of interest *in situ* is within the present methods. Such methods also apply to transfer of exogenous cells to the region of interest. Methods for *in situ* delivery of vectors preferably involve physically segregating the region of interest from the remainder of the patient's tissue in order to properly target the vector within the region. Upon segregation, the vector is-applied to the region of interest in a manner appropriate to transfer the expression cassette into the cells associated with the region of interest.

Tissue *ex vivo,* such as, for example, a graft, is completely isolated from the patient. For *ex vivo* vector transfer, the vector can be applied to the tissue in any suitable manner, e.g., in a carrier appropriate for transferring the vector. For example, the tissue can be incubated in a carrier containing the vector particles by any suitable incubation method. Other tissue can be perfused with the solution containing the vector.

The vectors or exogenous cells preferably remain in contact with the wound or tissue for a period of time sufficient to promote the transfer. For example, a carrier comprising a vector will remain in contact with the wound or *ex vivo* tissue from about one minute to about 2 hours, more preferably between 10 minutes and an hour, and most preferably from about 20 minutes to 40 minutes. In many applications, the carrier will optimally remain in contact with the wound or tissue for about 30 minutes.

### 6. Internal Delivery

For *in situ* delivery of a vector internally, the region of interest desirably is further segregated from the remainder of the subject's tissue. Any of a variety of known surgical procedures for physically segregating the region of interest is appropriate. Various endovascular surgical techniques appropriate for segregating a region of interest are available, depending upon the location of the target.

Endovascular surgical procedures include, but are not limited to, balloon angioplasty, intravascular stents, laser-assisted balloon angioplasty, double balloon catheterization, mechanical endarterectomy and vascular endoscopy. For a review of endovascular alternatives, see generally Ahn, "Endovascular Surgery," in Vascular Surgery, A Comprehensive Review, Ed. W. S. Moore, W. B. Saunders & Co., Philadelphia, 1993.

Several catheter designs can be utilized for local delivery of a vector. One catheter design consists of two independently inflated balloons, one proximal and one distal to the vascular delivery site. Inflation of these balloons provides an evacuated isolated arterial segment into which vectors for expression cassette delivery can be infused. This system is, however, limited by absence of distal arterial perfusion. A second catheter design developed by Wolinsky allows the infusion of the SIBLINGS protein-encoding vector through 25-100 µM pores under pressures up to 5 atm. This perfusion pressure increases the depth of penetration by the vectors and additionally increases expression cassette transfer efficiency. Yet another catheter design utilizes an expandable stent which traps the balloon against the arterial wall and allows intramural delivery of the expression cassette through spaces in the stent material. Additionally, these stents can be modified with burrs that create holes deeper in the vessel wall and allow flow of the expression cassette delivery agents to these sites to allow more uniform delivery of the expression cassette throughout the vessel wall.

Another delivery mechanism is to coat the catheter with a hydrophilic polyacrylic acid polymer that acts as a drug-absorbing sponge. By disrupting the vessel during the angioplasty procedure, this hydrogel is deposited within the vessel wall and will allow sustained delivery of the vector at the arterial wound site. Additionally, the iontophoretic balloon catheter is a catheter design that uses low electrical current to change the cell membrane polarity and allow the diffusion of charged DNA particles into the cell. This is a potential delivery mechanism for plasmid DNA expression cassette constructs. Also, biodegradable stents formed from agents such an ethylenevinyl acetic copolymer are appropriate for localized delivery to vascular tissue. Alternatively, an intravascular stent can be utilized wherein the endovascular scaffold of the stent is bathed in a ointment, cream, lotion, colloidal dispersion such as a gel or magma or any other acceptable carrier which comprises the SIBLINGS protein encoding vector for delivery to the targeted portion of a vessel segment. This solution is applicable to either an *in situ* or *ex vivo* based vessel delivery.

### EXAMPLE 5

### Additional Examples of Methods for Detecting and/or Quantifying SIBLINGS proteins

The following assays describe the detection of BSP, however, these assays, with appropriate modifications (i.e., antibody substitutions), can also be used to detect other SIBLINGS proteins.

### A. Chemiluminescent Sandwich Assay for BSP in Human Serum

Pierce Reacti-Bind White Opaque 96-Well EIA plates (product # 15042) are coated with 100 µL of 1 µg/mL Goat anti Mouse IgG (H & L), human serum adsorbed (Kirkegaard & Perry, Gaithersburg, MD) in PBS. The plates are then covered and incubated overnight at 4°C. The next morning the wells are emptied and 250 µL of 1 % bovine serum albumin is added for 15 min. The albumin is then removed and the plates are washed 3 times with ∼300 µL PBS-0.05% Tween 20 (PBS-T) for 5 minutes each at room temperature. 100 µL 1:1000 LFMb-2 (monoclonal to human BSP) in PBS-T is added to each well and the plate is incubated again for 1 hour at room temp and then washed three times (PBS-Tween).

The samples are prepared from 25 µL human. 50 µL fresh formamide and 1 µL of 0.1 M Dithiothreitol is added to the sample and the sample is then heated at 100°C for 5 minutes and diluted to 1 mL with PBS-T containing 0.03% H₂O₂. This addition dilutes the fomamide to levels sufficiently low enough to promote normal antibody binding and the H₂O₂ inactivates the remaining DTT.

The BSP standard (5 ng to 0.15 ng) and a negative control (without BSP) are prepared in PBS-T and added to individual wells. 100 µL diluted sample is also added to the individual wells. The samples and the standards are then incubated 1 hour at room temperature and then washed 3 times with PBS-Tween. 100 µL of 1:1000 LF-100 polyclonal (rabbit anti-human BSP) antibody in PBS-T plus 1% normal mouse serum is then added to each well and the plate is incubated for 1 hour at room temperature and then washed 3 times. 100 µL of 1:50,000 diluted peroxidase-conjugated goat anti rabbit IgG (H&L), multi-serum adsorbed (Kirkegaard & Perry) is then added to each well and the plate is incubated for 1 hour at room temperature, washed 3 times (PBS-Tween).

The results are then visualized by adding 100 µL of Pierce Supersignal ELISA Pico Chemiluminescent Substrate (product # 37070) and incubating 10 minutes at room temperature. Luminescence can then be detected in a luminometer.

### B. Western Blot and Quantitative Immunoanalysis

5 µL of serum is added to 95 µL of sample buffer (0.0625 M Tris, pH 6.8, 2.3% SDS, 5 % 2-mercaptoethanol or 1 mM dithiothreitol, 8 M urea or 50% formamide, bromophenol blue tracking dye), the sample is then heated for 5 minutes at 100°C. 10 µL of the sample is then added to each well of a 4-20 % or 4-12 % gradient acrylamide gel (Novex Corp) and the gel is electrophoresed with 100-200 V constant voltage until the dye front reaches the bottom of the gel. Molecular weight standards (prestained) and BSP standards are also electrophoresed.

The protein is then transferred to nitrocellulose or other western blot membranes under standard conditions. If nitrocellulose is used transfer can be achieved in 60 minutes at 100 volts with cooling in the Novex XCell II Blot Module in Tris/Glycine buffer containing 20% methanol.

Protein is then detected by blocking the membrane with 3% BSA-NGS in PBS for 15 minutes and then adding 1 % normal goat serum for 45 minutes. An antibody, for instance the polyclonal antibodye LF-100, or monoclonal LF-Mb-11 is then diluted at 1:2000 and added to the membrane for 1 hour at room temperature. The membrane is then washed 3 times with PBS-Tween 20 (0.05%). 3 % BSA-1 % NGS is added and then HRP-conjugated second antibody (goat anti-rabbit or goat anti-mouse IgG respectively) at is added to a final concentration of 1:50,000. The membrane is then incubated for 1 hour at room temperature and washed 3 times. Finally, Pierce SuperSIgnal West Pico Chemiluminescent reagent is added for 5 minutes and the membrane is placed in clear plastic bag and exposed to a chilled digital camera (Fuji LAS-100).

## Claims

1. A method of detecting a SIBLINGS protein in a sample in vitro comprising:
disrupting a SIBLINGS protein/Factor H protein complex; and
contacting the sample with a specific binding agent, wherein the specific binding agent binds to the SIBLINGS protein;
thereby detecting the SIBLINGS protein.

2. The method of claim 1 wherein the method further comprises:
separating a Factor H protein from the SIBLINGS protein using a denaturing agent.

3. The method of claim 2, wherein separating the Factor H protein from the SIBLINGS protein comprises the use of electrophoresis.

4. The method of claim 1 wherein disrupting the SIBLINGS protein/Factor H protein complex comprises an addition of heat, a reducing agent, a denaturing agent, a combination thereof or a competitive binding agent.

5. The method of claim 4 wherein the reducing agent comprises DTT (1,4-dithiothreitol), β-mercaptoethanol, trialkyl phosphines.

6. The method of claim 1 wherein the SIBLINGS protein is bone sialoprotein (BSP).

7. The method of claim 6, further comprising separating a relatively acidic BSP from a relatively non-acidic BSP, wherein the relatively acidic BSP elutes from a quaternary amine ethyl (QAE) column at a salt concentration greater than 1M NaCl and the relatively non-acidic BSP elutes from a QAE column at a salt concentration less than or equal to 1M NaCl.

8. The method of claim 2, wherein separating the Factor H protein from the SIBLINGS protein comprises the use of chromatography.

9. The method of claim 1, wherein the sample comprises cells from a tumour.

10. The method of claim 9, wherein the tumour is a multiple myeloma, a breast tumor, a lung tumor, a thyroid tumor, or a prostate tumor.

11. The method of claim 9, wherein the tumor produces a relatively acidic BSP elutes from a quaternary amine ethyl (QAE) column at a salt concentration greater than 1M NaCl.

12. The method of claim 1, wherein the sample comprises bone extract.

13. The method of claim 1, wherein the sample comprises bone marrow fibroblasts.

14. The method of claim 1, wherein the specific binding agent is a polyclonal antibody, a monoclonal antibody, an immunologically effective portion of a monoclonal antibody, or a combination thereof.

15. The method of claim 1, wherein the SIBLINGS protein is osteopontin, dentin matrix protein, or dentin sialophosphoprotein.

16. The method of claim 1, wherein the sample comprises body fluid.

17. The method of claim 1, wherein the sample comprises blood, serum, or saliva.

18. The method of claim 1, wherein the sample is used to screen for abnormal bone turnover in a subject.

19. The method of claim 18, wherein the subject is a human.

20. A method of screening for a tumor in a subject comprising:
separating a Factor H protein from a relatively acidic BSP in a Factor H/BSP complex in a sample from the subject, wherein the relatively acidic BSP elutes from a quaternary amine ethyl (QAE) column at a salt concentration greater than 1M NaCl; and
detecting a concentration of the relatively acidic BSP, thereby screening for the tumor.

21. The method of claim 20, wherein the tumor is a breast tumor, a lung tumor, a thyroid tumor, a multiple myeloma, or a prostate tumor.

22. The method of claim 20, further comprising comparing the concentration of the relatively acidic BSP to a control.

23. The method of claim 20, wherein detecting the concentration of the relatively acidic BSP comprises contacting the sample with a specific binding agent.

24. The method of claim 23, wherein the specific binding agent comprises a polyclonal antibody or a monoclonal antibody that binds a relatively acidic BSP.

25. The method of claim 20, wherein screening for the tumor further comprises separating the relatively acidic BSP from a relatively non-acidic BSP.

26. The method of claim 25, further comprising detecting a relatively non-acidic form of BSP in the sample and comparing the amount of relatively non-acidic BSP in the sample to an amount of relatively acidic BSP in the sample.

## Revendications

1. Méthode de détection d'une protéine SIBLINGS dans un échantillon *in vitro,* comprenant :
la dissociation d'un complexe de protéine SIBLINGS/protéine du Facteur H ; et
la mise en contact l'échantillon avec un agent liant spécifique, l'agent liant spécifique se liant à la protéine SIBLINGS ;
pour détecter ainsi la protéine SIBLINGS.

2. Méthode selon la revendication 1, dans laquelle la méthode comprend de plus :
la séparation de la protéine du Facteur H d'avec la protéine SIBLINGS en utilisant un agent dénaturant.

3. Méthode selon la revendication 2, dans laquelle la séparation de la protéine du Facteur H d'avec la protéine SIBLINGS comprend l'utilisation d'électrophorèse.

4. Méthode selon la revendication 1, dans laquelle la dissociation du complexe de protéine SIBLINGS/protéine du Facteur H comprend l'apport de chaleur, d'un agent réducteur, d'un agent dénaturant, d'une association d'entre eux ou d'un agent liant compétitif.

5. Méthode selon la revendication 4, dans laquelle l'agent réducteur comprend du DTT (1,4-dithiothréitol), du β-mercaptoéthanol, des trialkylphosphines.

6. Méthode selon la revendication 1, dans laquelle la protéine SIBLINGS est une sialoprotéine osseuse (BSP).

7. Méthode selon la revendication 6, comprenant de plus la séparation d'une BSP relativement acide d'une BSP relativement non acide, dans laquelle la BSP relativement acide s'élue d'une colonne d'amine quaternaire-éthyle (QAE) à une concentration de sel supérieure à 1M de NaCl et la BSP relativement non acide s'élue d'une colonne QAE à une concentration de sel inférieure ou égale à 1M de NaCl.

8. Méthode selon la revendication 2, dans laquelle la séparation de la protéine du Facteur H d'avec la protéine SIBLINGS comprend l'utilisation de chromatographie.

9. Méthode selon la revendication 1, dans laquelle l'échantillon comprend des cellules provenant d'une tumeur.

10. Méthode selon la revendication 9, dans laquelle la tumeur est un myélome multiple, une tumeur mammaire, une tumeur du poumon, une tumeur de la thyroïde ou une tumeur de la prostate.

11. Méthode selon la revendication 9, dans laquelle la tumeur produit une BSP relativement acide qui s'élue d'une colonne d'amine quaternaire-éthyle (QAE) à une concentration de sel supérieure à 1M de NaCl.

12. Méthode selon la revendication 1, dans laquelle l'échantillon comprend un extrait d'os.

13. Méthode selon la revendication 1, dans laquelle l'échantillon comprend des fibroblastes de moelle osseuse.

14. Méthode selon la revendication 1, dans laquelle l'agent liant spécifique est un anticorps polyclonal, un anticorps monoclonal, une partie immunologiquement active d'un anticorps monoclonal, ou une association d'entre eux.

15. Méthode selon la revendication 1, dans laquelle la protéine SIBLINGS est l'ostéopontine, la protéine de la matrice dentinaire ou la sialophosphoprotéine dentinaire.

16. Méthode selon la revendication 1, dans laquelle l'échantillon comprend un fluide corporel.

17. Méthode selon la revendication 1, dans laquelle l'échantillon comprend du sang, du sérum ou de la salive.

18. Méthode selon la revendication 1, dans laquelle l'échantillon est utilisé pour dépister un renouvellement osseux anormal chez un sujet.

19. Méthode selon la revendication 18, dans laquelle le sujet est un être humain.

20. Méthode de dépistage d'une tumeur chez un sujet, comprenant :
la séparation d'une protéine du Facteur H d'avec une BSP relativement acide dans un complexe de Facteur H/BSP dans un échantillon provenant du sujet, dans laquelle la BSP relativement acide s'élue d'une colonne d'amine quaternaire-éthyle (QAE) à une concentration de sel supérieure à 1M de NaCl ; et
la détection d'une concentration de la BSP relativement acide, pour dépister ainsi la tumeur.

21. Méthode selon la revendication 20, dans laquelle la tumeur est une tumeur mammaire, une tumeur du poumon, une tumeur de la thyroïde, un myélome multiple ou une tumeur de la prostate.

22. Méthode selon la revendication 20, comprenant de plus la comparaison de la concentration de la BSP relativement acide à un témoin.

23. Méthode selon la revendication 20, dans laquelle la détection de la concentration de la BSP relativement acide comprend la mise en contact de l'échantillon avec un agent liant spécifique.

24. Méthode selon la revendication 23, dans laquelle l'agent liant spécifique comprend un anticorps polyclonal ou un anticorps monoclonal qui lie une BSP relativement acide.

25. Méthode selon la revendication 20, dans laquelle le dépistage de la tumeur comprend de plus la séparation de la BSP relativement acide d'une BSP relativement non acide.

26. Méthode selon la revendication 25, comprenant de plus la détection d'une forme relativement non acide de BSP dans l'échantillon et la comparaison de la quantité de BSP relativement non acide dans l'échantillon à une quantité de BSP relativement acide dans l'échantillon.

## Patentansprüche

1. Verfahren zum Nachweis eines SIBLINGS-Proteins in einer Probe in vitro, umfassend:
Zerstören eines SIBLINGS-Protein/Faktor-H-Protein-Komplexes; und
In-Kontakt-Bringen der Probe mit einem spezifischen Bindungsmittel, wobei das spezifische Bindungsmittel an das SIBLINGS-Protein bindet;
wodurch das SIBLINGS-Protein nachgewiesen wird.

2. Verfahren nach Anspruch 1, wobei das Verfahren weiterhin umfaßt:
Trennen eines Faktor-H-Proteins von dem SIBLINGS-Protein unter Verwendung eines Denaturierungsmittels.

3. Verfahren nach Anspruch 2, wobei das Trennen des Faktor-H-Proteins von dem SIBLINGS-Protein die Verwendung von Elektrophorese umfaßt.

4. Verfahren nach Anspruch 1, wobei das Zerstören des SIBLINGS-Protein/Faktor-H-Protein-Komplexes eine Zugabe von Wärme, ein Reduktionsmittel, ein Denaturierungsmittel, eine Kombination davon oder ein kompetitives Bindungsmittel umfaßt.

5. Verfahren nach Anspruch 4, wobei das Reduktionsmittel DTT (1,4-Dithiothreitol), â-Mercaptoethanol, Trialkylphosphine umfaßt.

6. Verfahren nach Anspruch 1, wobei das SIBLINGS-Protein Knochen-Sialoprotein (BSP) ist.

7. Verfahren nach Anspruch 6, weiterhin umfassend das Trennen eines relativ sauren BSP von einem relativ nicht-sauren BSP, wobei das relativ saure BSP von einer quartären Aminethyl-Säule (QAE) bei einer Salzkonzentration größer als 1 M NaCl eluiert, und das relativ nicht-saure BSP von einer QAE-Säule bei einer Salzkonzentration von weniger als oder gleich 1M NaCl eluiert.

8. Verfahren nach Anspruch 2, wobei das Trennen des Faktor-H-Proteins von dem SIBLINGS-Protein die Verwendung von Chromatograhie umfaßt.

9. Verfahren nach Anspruch 1, wobei die Probe Zellen von einem Tumor umfaßt.

10. Verfahren nach Anspruch 9, wobei der Tumor ein multiples Myelom, ein Brusttumor, ein Lungentumor, ein Schilddrüsentumor oder ein Prostatatumor ist.

11. Verfahren nach Anspruch 9, wobei der Tumor ein relativ saures BSP erzeugt, das von einer quartären Aminethyl-Säule (QAE) bei einer Salzkonzentration größer als 1M NaCl eluiert.

12. Verfahren nach Anspruch 1, wobei die Probe einen Knochenextrakt umfaßt.

13. Verfahren nach Anspruch 1, wobei die Probe Knochenmarkfibroblasten umfaßt.

14. Verfahren nach Anspruch 1, wobei das spezifische Bindungsmittel ein polyklonaler Antikörper, ein monoklonaler Antikörper, ein immunologisch wirksamer Teil eines monoklonalen Antikörpers oder eine Kombination davon ist.

15. Verfahren nach Anspruch 1, wobei das SIBLINGS-Protein Osteopontin, Dentin-Matrix-Protein oder Dentin-Sialophosphoprotein ist.

16. Verfahren nach Anspruch 1, wobei die Probe Körperflüssigkeit umfaßt.

17. Verfahren nach Anspruch 1, wobei die Probe Blut, Serum oder Speichel umfaßt.

18. Verfahren nach Anspruch 1, wobei die Probe verwendet wird, um auf einen abnormen Knochenumsatz in einem Patienten zu screenen.

19. Verfahren nach Anspruch 18, wobei der Patient ein Mensch ist.

20. Verfahren zum Screenen auf einen Tumor in einem Patienten, umfasssend:
Trennen eines Faktor-H-Proteins von einem relativ sauren BSP in einem Faktor H/BSP-Komplex in einer Probe von dem Patienten, wobei das relativ saure BSP von einer quartären Aminethyl-Säule (QAE) bei einer Salzkonzentration größer als 1M NaCl eluiert; und
Nachweis einer Konzentration des relativ sauren BSP, wodurch auf den Tumor gescreent wird.

21. Verfahren nach Anspruch 20, wobei der Tumor ein Brusttumor, ein Lungentumor, ein Schilddrüsentumor, ein multiples Myelom oder ein Prostatatumor ist.

22. Verfahren nach Anspruch 20, weiterhin umfassend das Vergleichen der Konzentration des relativ sauren BSP mit einer Kontrolle.

23. Verfahren nach Anspruch 20, wobei das Nachweisen der Konzentration des relativ sauren BSP das In-Kontakt-Bringen der Probe mit einem spezifischen Bindungsmittel umfaßt.

24. Verfahren nach Anspruch 23, wobei das spezifische Bindungsmittel einen polyklonalen Antikörper oder einen monoklonalen Antikörper umfaßt, der an ein relativ saures BSP bindet.

25. Verfahren nach Anspruch 20, wobei das Screenen auf den Tumor weiterhin das Trennen des relativ sauren BSP von einem relativ nicht-sauren BSP umfaßt.

26. Verfahren nach Anspruch 25, weiterhin umfassend das Nachweisen einer relativ nicht-sauren Form von BSP in der Probe und Vergleichen der Menge an relativ nicht-saurem BSP in der Probe mit einer Menge an relativ saurem BSP in der Probe.


### SEQUENCE LISTING

<110> Fisher et al., Larry
<120> Complex Formed by Small Integrin-Binding Ligand, N-Linked Glycoproteins (SIBLINGS) and Factor H
<130> 54101
<140>
   <141>
<150> 60/128,468
   <151> 1999-04-09
<160> 18
<170> PatentIn Ver. 2.1
<210> 1
   <211> 317
   <212> PRT
   <213> Homo sapiens
<400> 1
   Met Lys Thr Ala Leu Ile Leu Leu Ser Ile Leu Gly Met Ala Cys Ala
    1               5                  10                  15
   Phe Ser Met Lys Asn Leu His Arg Arg Val Lys Ile Glu Asp Ser Glu
               20                  25                  30
   Glu Asn Gly Val Phe Lys Tyr Arg Pro Arg Tyr Tyr Leu Tyr Lys His
           35                  40                  45
   Ala Tyr Phe Tyr Pro His Leu Lys Arg Phe Pro Val Gln Gly Ser Ser
       50                  55                  60
   Asp Ser Ser Glu Glu Asn Gly Asp Asp Ser Ser Glu Glu Glu Glu Glu
    65                 70                  75                  80
   Glu Glu Glu Thr Ser Asn Glu Gly Glu Asn Asn Glu Glu Ser Asn Glu
                   85                  90                  95
   Asp Glu Asp Ser Glu Ala Glu Asn Thr Thr Leu Ser Ala Thr Thr Leu
               100                 105                 110
   Gly Tyr Gly Glu Asp Ala Thr Pro Gly Thr Gly Tyr Thr Gly Leu Ala
           115                 120                 125
   Ala Ile Gln Leu Pro Lys Lys Ala Gly Asp Ile Thr Asn Lys Ala Thr
       130                 135                 140
   Lys Glu Lys Glu Ser Asp Glu Glu Glu Glu Glu Glu Glu Glu Gly Asn
   145                 150                 155                 160
   Glu Asn Glu Glu Ser Glu Ala Glu Val Asp Glu Asn Glu Gln Gly Ile
                   165                 170                 175
   Asn Gly Thr Ser Thr Asn Ser Thr Glu Ala Glu Asn Gly Asn Gly Ser
               180                 185                 190
   Ser Gly Gly Asp Asn Gly Glu Glu Gly Glu Glu Glu Ser Val Thr Gly
           195                 200                 205
   Ala Asn Ala Glu Gly Thr Thr Glu Thr Gly Gly Gln Gly Lys Gly Thr
       210                 215                 220
   Ser Lys Thr Thr Thr Ser Pro Asn Gly Gly Phe Glu Pro Thr Thr Pro
   225                 230                 235                 240
   Pro Gln Val Tyr Arg Thr Thr Ser Pro Pro Phe Gly Lys Thr Thr Thr
                   245                 250                 255
   Val Glu Tyr Glu Gly Glu Tyr Glu Tyr Thr Gly Val Asn Glu Tyr Asp
               260                 265                 270
   Asn Gly Tyr Glu Ile Tyr Glu Ser Glu Asn Gly Glu Pro Arg Gly Asp
           275                 280                 285
   Asn Tyr Arg Ala Tyr Glu Asp Glu Tyr Ser Tyr Phe Lys Gly Gln Gly
       290                 295                 300
   Tyr Asp Gly Tyr Asp Gly Gln Asn Tyr Tyr His His Gln
   305                 310                 315
<210> 2
   <211> 18
   <212> PRT
   <213> Artificial Sequence
<220>
   <223> Description of Artificial Sequence:immunogen
<400> 2
   Tyr Glu Ser Glu Asn Gly Glu Pro Arg Gly Asp Asn Tyr Arg Ala Tyr
    1               5                  10                  15
   Glu Asp
<210> 3
   <211> 12
   <212> PRT
   <213> Artificial Sequence
<220>
   <223> Description of Artificial Sequence:immunogen
<400> 3
   Ala Ile Gln Leu Pro Lys Lys Ala Gly Asp Ile Cys
    1               5                  10
<210> 4
   <211> 60
   <212> PRT
   <213> Artificial Sequence
<220>
   <223> Description of Artificial Sequence:immunogen
<400> 4
   Glu Tyr Glu Gly Glu Tyr Glu Tyr Thr Gly Val Asn Glu Tyr Asp Asn
   1 5 10 15
   Gly Tyr Glu Ile Tyr Glu Ser Glu Asn Gly Glu Pro Arg Gly Asp Asn
   20 25 30
   Tyr Arg Ala Tyr Glu Asp Glu Tyr Ser Tyr Phe Lys Gly Gln Gly Tyr
   35 40 45
   Asp Gly Tyr Asp Gly Gln Asn Tyr Tyr His His Gln
   50 55 60
<210> 5
   <211> 152
   <212> PRT
   <213> Artificial Sequence
<220>
   <223> Description of Artificial Sequence:immunogen
<400> 5
   Ile Gln Leu Pro Lys Lys Ala Gly Asp Ile Thr Asn Lys Ala Thr Lys
   1 5 10 15
   Glu Lys Glu Ser Asp Glu Glu Glu Glu Glu Glu Glu Glu Gly Asn Glu
   20 25 30
   Asn Glu Glu Ser Glu Ala Glu Val Asp Glu Asn Glu Gln Gly Ile Asn
   35 40 45
   Gly Thr Ser Thr Asn Ser Thr Glu Ala Glu Asn Gly Asn Gly Ser Ser
   50 55 60
   Gly Gly Asp Asn Gly Glu Glu Gly Glu Glu Glu Ser Val Thr Gly Ala
   65 70 75 80
   Asn Ala Glu Gly Thr Thr Glu Thr Gly Gly Gln Gly Lys Gly Thr Ser
   85 90 95
   Lys Thr Thr Thr Ser Pro Asn Gly Gly Phe Glu Pro Thr Thr Pro Pro
   100 105 110
   Gln Val Tyr Arg Thr Thr Ser Pro Pro Phe Gly Lys Thr Thr Thr Val
   115 120 125
   Glu Tyr Glu Gly Glu Tyr Glu Tyr Thr Gly Val Asn Glu Tyr Asp Asn
   130 135 140
   Gly Tyr Glu Ile Tyr Glu Ser Glu
   145 150
<210> 6
   <211> 25
   <212> PRT
   <213> Artificial Sequence
<220>
   <223> Description of Artificial Sequence:immunogen
<400> 6
   Val Phe Lys Tyr Arg Pro Arg Tyr Tyr Leu Tyr Lys His Ala Tyr Phe
   1 5 10 15
   Tyr Pro His Leu Lys Arg Phe Pro Val
   20 25
<210> 7
   <211> 28
   <212> PRT
   <213> Artificial Sequence
<220>
   <223> Description of Artificial Sequence:immunogen
<220>
   <221> VARIANT
   <222> (5)
   <223> Sulfated
<220>
   <221> VARIANT
   <222> (9)
   <223> sulfated
<220>
   <221> VARIANT
   <222> (11)
   <223> sulfated
<220>
   <221> VARIANT
   <222> (17)
   <223> Sulfated
<220>
   <221> VARIANT
   <222> (21)
   <223> sulfated
<220>
   <221> VARIANT
   <222> (24)
   <223> sulfated
<400> 7
   Cys Thr Val Glu Tyr Glu Gly Glu Tyr Glu Tyr Thr Gly Ala Asn Asp
   1 5 10 15
   Tyr Asp Asn Gly Tyr Glu Ile Tyr Glu Ser Glu Asn
   20 25
<210> 8
   <211> 1037
   <212> DNA
   <213> Homo sapiens
<400> 8
   ccttctctgc cctctcactc ccttgagcct gcttcctcac tccaggactg ccagaggaag 60
   caatcaccaa aatgaagact gctttaattt tgctcagcat tttgggaatg gcctgtgctt 120
   tctcaatgaa aaatttgcat cgaagagtca aaatagagga ttctgaagaa aatggggtct 180
   ttaagtacag gccacgatat tatctttaca agcatgccta cttttatcct catttaaaac 240
   gatttccagt tcagggcagt agtgactcat ccgaagaaaa tggagatgac agttcagaag 300
   aggaggagga agaagaggag acttcaaatg aaggagaaaa caatgaagaa tcgaatgaag 360
   atgaagactc tgaggctgag aataccacac tttctgctac aacactgggc tatggagagg 420
   acgccacgcc tggcacaggg tatacagggt tagctgcaat ccagcttccc aagaaggctg 480
   gggatataac aaacaaagct acaaaagaga aggaaagtga tgaagaagaa gaggaggaag 540
   aggaaggaaa tgaaaacgaa gaaagcgaag cagaagtgga tgaaaacgaa caaggcataa 600
   acggcaccag taccaacagc acagaggcag aaaacggcaa cggcagcagc ggaggagaca 660
   atggagaaga aggggaagaa gaaagtgtca ctggagccaa tgcagaaggc accacagaga 720
   ccggagggca gggcaagggc acctcgaaga caacaacctc tccaaatggt gggtttgaac 780
   ctacaacccc accacaagtc tatagaacca cttccccacc ttttgggaaa accaccaccg 840
   ttgaatacga gggggagtac gaatacacgg gcgtcaatga atacgacaat ggatatgaaa 900
   tctatgaaag tgagaacggg gaacctcgtg gggacaatta ccgagcctat gaagatgagt 960
   acagctactt taaaggacaa ggctacgatg gctatgatgg tcagaattac taccaccacc 1020
   agtgaagctc cagcctg                                                1037
<210> 9
   <211> 24
   <212> PRT
   <213> Artificial Sequence
<220>
   <221> THIOETH
   <222> (2)..(17)
<220>
   <223> Description of Artificial Sequence: Immunogen
<400> 9
   Glu Tyr Glu Tyr Thr Gly Val Asn Glu Tyr Asp Asn Gly Tyr Glu Ile
    1               5                  10                  15
   Tyr Glu Ser Glu Asn Gly Glu Pro
               20
<210> 10
   <211> 5
   <212> PRT
   <213> Artificial Sequence
<220>
   <223> Description of Artificial Sequence: Competitor peptide
<400> 10
   Gly Arg Gly Asp Ser
    1               5
<210> 11
   <211> 1447
   <212> DNA
   <213> Homo sapiens
<220>
   <221> CDS
   <222> (109)..(1011)
<400> 11
   gaggcagcag cagcaggagg aggcagacac agcatcgtcg ggaccagact cgtctcaggc 60
   cagttgcagc cttctcagcc aaacgccgac caaggaaaac tcactacc atg aga att  117
                                                        Met Arg Ile
                                                         1
   gca gtg att tgc ttt tgc ctc cta ggc atc acc tgt gcc ata cca gtt   165
   Ala Val Ile Cys Phe Cys Leu Leu Gly Ile Thr Cys Ala Ile Pro Val
       5                   10                  15
   aaa cag gct gat tct gga agt tct gag gaa aag cag ctt tac aac aaa   213
   Lys Gln Ala Asp Ser Gly Ser Ser Glu Glu Lys Gln Leu Tyr Asn Lys
   20                  25                  30                  35
   tac cca gat gct gtg gcc aca tgg cta aac cct gac cca tct cag aag   261
   Tyr Pro Asp Ala Val Ala Thr Trp Leu Asn Pro Asp Pro Ser Gln Lys
                   40                  45                  50
   cag aat ctc cta gcc cca cag acc ctt cca agt aag tcc aac gaa agc   309
   Gln Asn Leu Leu Ala Pro Gln Thr Leu Pro Ser Lys Ser Asn Glu Ser
               55                  60                  65
   cat gac cac atg gat gat atg gat gat gaa gat gat gat gac cat gtg   357
   His Asp His Met Asp Asp Met Asp Asp Glu Asp Asp Asp Asp His Val
           70                  75                  80
   gac agc cag gac tcc att gac tcg aac gac tct gat gat gta gat gac   405
   Asp Ser Gln Asp Ser Ile Asp Ser Asn Asp Ser Asp Asp Val Asp Asp
       85                  90                  95
   act gat gat tct cac cag tct gat gag tct cac cat tct gat gaa tct   453
   Thr Asp Asp Ser His Gln Ser Asp Glu Ser His His Ser Asp Glu Ser
   100                 105                 110                 115
   gat gaa ctg gtc act gat ttt ccc acg gac ctg cca gca acc gaa gtt   501
   Asp Glu Leu Val Thr Asp Phe Pro Thr Asp Leu Pro Ala Thr Glu Val
                   120                 125                 130
   ttc act cca gtt gtc ccc aca gta gac aca tat gat ggc cga ggt gat   549
   Phe Thr Pro Val Val Pro Thr Val Asp Thr Tyr Asp Gly Arg Gly Asp
               135                 140                 145
   agt gtg gtt tat gga ctg agg tca aaa tct aag aag ttt cgc aga cct   597
   Ser Val Val Tyr Gly Leu Arg Ser Lys Ser Lys Lys Phe Arg Arg Pro
           150                 155                 160
   gac atc cag tac cct gat gct aca gac gag gac atc acc tca cac atg   645
   Asp Ile Gln Tyr Pro Asp Ala Thr Asp Glu Asp Ile Thr Ser His Met
       165                 170                 175
   gaa agc gag gag ttg aat ggt gca tac aag gcc atc ccc gtt gcc cag   693
   Glu Ser Glu Glu Leu Asn Gly Ala Tyr Lys Ala Ile Pro Val Ala Gln
   180                 185                 190                 195
   gac ctg aac gcg cct tct gat tgg gac agc cgt ggg aag gac agt tat   741
   Asp Leu Asn Ala Pro Ser Asp Trp Asp Ser Arg Gly Lys Asp Ser Tyr
                   200                 205                 210
   gaa acg agt cag ctg gat gac cag agt gct gaa acc cac agc cac aag    789
   Glu Thr Ser Gln Leu Asp Asp Gln Ser Ala Glu Thr His Ser His Lys
               215                 220                     225
   cag tcc aga tta tat aag cgg aaa gcc aat gat gag agc aat gag cat    837
   Gln Ser Arg Leu Tyr Lys Arg Lys Ala Asn Asp Glu Ser Asn Glu His
           230                 235                     240
   tcc gat gtg att gat agt cag gaa ctt tcc aaa gtc agc cgt gaa ttc    885
   Ser Asp Val Ile Asp Ser Gln Glu Leu Ser Lys Val Ser Arg Glu Phe
       245                 250                 255
   cac agc cat gaa ttt cac agc cat gaa gat atg ctg gtt gta gac ccc    933
   His Ser His Glu Phe His Ser His Glu Asp Met Leu Val Val Asp Pro
   260                 265                 270                 275
   aaa agt aag gaa gaa gat aaa cac ctg aaa ttt cgt att tct cat gaa    981
   Lys Ser Lys Glu Glu Asp Lys His Leu Lys Phe Arg Ile Ser His Glu
                   280                 285                 290
   tta gat agt gca tct tct gag gtc aat taa aaggagaaaa aatacaattt     1031
   Leu Asp Ser Ala Ser Ser Glu Val Asn
               295                 300
   ctcactttgc atttagtcaa aagaaaaaat gctttatagc aaaatgaaag agaacatgaa 1091
   atgcttcttt ctcagtttat tggttgaatg tgtatctatt tgagtctgga aataactaat 1151
   gtgtttgata attagtttag tttgtggctt catggaaact ccctgtaaac taaaagcttc 1211
   agggttatgt ctatgttcat tctatagaag aaatgcaaac tatcactgta ttttaatatt 1271
   tgttattctc tcatgaatag aaatttatgt agaagcaaac aaaatacttt tacccactta 1331
   aaaagagaat ataacatttt atgtcactat aatcttttgt tttttaagtt agtgtatatt 1391
   ttgttgtgat tatctttttg tggtgtgaat aaatctttta tcttgaatgt aataag     1447
<210> 12
   <211> 300
   <212> PRT
   <213> Homo sapiens
<400> 12
   Met Arg Ile Ala Val Ile Cys Phe Cys Leu Leu Gly Ile Thr Cys Ala
    1               5                  10                  15
   Ile Pro Val Lys Gln Ala Asp Ser Gly Ser Ser Glu Glu Lys Gln Leu
               20                  25                  30
   Tyr Asn Lys Tyr Pro Asp Ala Val Ala Thr Trp Leu Asn Pro Asp Pro
           35                  40                  45
   Ser Gln Lys Gln Asn Leu Leu Ala Pro Gln Thr Leu Pro Ser Lys Ser
       50                  55                  60
   Asn Glu Ser His Asp His Met Asp Asp Met Asp Asp Glu Asp Asp Asp
   65                  70                  75                  80
   Asp His Val Asp Ser Gln Asp Ser Ile Asp Ser Asn Asp Ser Asp Asp
                   85                  90                  95
   Val Asp Asp Thr Asp Asp Ser His Gln Ser Asp Glu Ser His His Ser
               100                 105                 110
   Asp Glu Ser Asp Glu Leu Val Thr Asp Phe Pro Thr Asp Leu Pro Ala
           115                 120                 125
   Thr Glu Val Phe Thr Pro Val Val Pro Thr Val Asp Thr Tyr Asp Gly
       130                 135                 140
   Arg Gly Asp Ser Val Val Tyr Gly Leu Arg Ser Lys Ser Lys Lys Phe
   145                 150                 155                 160
   Arg Arg Pro Asp Ile Gln Tyr Pro Asp Ala Thr Asp Glu Asp Ile Thr
                   165                 170                 175
   Ser His Met Glu Ser Glu Glu Leu Asn Gly Ala Tyr Lys Ala Ile Pro
               180                 185                 190
   Val Ala Gln Asp Leu Asn Ala Pro Ser Asp Trp Asp Ser Arg Gly Lys
           195                 200                 205
   Asp Ser Tyr Glu Thr Ser Gln Leu Asp Asp Gln Ser Ala Glu Thr His
       210                 215                 220
   Ser His Lys Gln Ser Arg Leu Tyr Lys Arg Lys Ala Asn Asp Glu Ser
   225                 230                 235                 240
   Asn Glu His Ser Asp Val Ile Asp Ser Gln Glu Leu Ser Lys Val Ser
                   245                 250                 255
   Arg Glu Phe His Ser His Glu Phe His Ser His Glu Asp Met Leu Val
               260                 265                 270
   Val Asp Pro Lys Ser Lys Glu Glu Asp Lys His Leu Lys Phe Arg Ile
           275                 280                 285
   Ser His Glu Leu Asp Ser Ala Ser Ser Glu Val Asn
       290                 295                 300
<210> 13
   <211> 1424
   <212> DNA
   <213> Homo sapiens
<220>
   <221> CDS
   <222> (68)..(1012)
<400> 13
   gaccagactc gtctcaggcc agttgcagcc ttctcagcca aacgccgacc aaggaaaact 60
   cactacc atg aga att gca gtg att tgc ttt tgc ctc cta ggc atc acc     109
           Met Arg Ile Ala Val Ile Cys Phe Cys Leu Leu Gly Ile Thr
             1               5                  10
   tgt gcc ata cca gtt aaa cag gct gat tct gga agt tct gag gaa aag     157
   Cys Ala Ile Pro Val Lys Gln Ala Asp Ser Gly Ser Ser Glu Glu Lys
    15                  20                  25                  30
   cag ctt tac aac aaa tac cca gat gct gtg gcc aca tgg cta aac cct     205
   Gln Leu Tyr Asn Lys Tyr Pro Asp Ala Val Ala Thr Trp Leu Asn Pro
                    35                  40                  45
   gac cca tct cag aag cag aat ctc cta gcc cca cag aat gct gtg tcc     253
   Asp Pro Ser Gln Lys Gln Asn Leu Leu Ala Pro Gln Asn Ala Val Ser
                50                  55                  60
   tct gaa gaa acc aat gac ttt aaa caa gag acc ctt cca agt aag tcc     301
   Ser Glu Glu Thr Asn Asp Phe Lys Gln Glu Thr Leu Pro Ser Lys Ser
            65                  70                  75
   aac gaa agc cat gac cac atg gat gat atg gat gat gaa gat gat gat     349
   Asn Glu Ser His Asp His Met Asp Asp Met Asp Asp Glu Asp Asp Asp
        80                  85                  90
   gac cat gtg gac agc cag gac tcc att gac tcg aac gac tct gat gat    397
   Asp His Val Asp Ser Gln Asp Ser Ile Asp Ser Asn Asp Ser Asp Asp
    95                 100                 105                 110
   gta gat gac act gat gat tct cac cag tct gat gag tct cac cat tct    445
   Val Asp Asp Thr Asp Asp Ser His Gln Ser Asp Glu Ser His His Ser
                   115                 120                 125
   gat gaa tct gat gaa ctg gtc act gat ttt ccc acg gac ctg cca gca    493
   Asp Glu Ser Asp Glu Leu Val Thr Asp Phe Pro Thr Asp Leu Pro Ala
               130                 135                 140
   acc gaa gtt ttc act cca gtt gtc ccc aca gta gac aca tat gat ggc    541
   Thr Glu Val Phe Thr Pro Val Val Pro Thr Val Asp Thr Tyr Asp Gly
           145                 150                 155
   cga ggt gat agt gtg gtt tat gga ctg agg tca aaa tct aag aag ttt    589
   Arg Gly Asp Ser Val Val Tyr Gly Leu Arg Ser Lys Ser Lys Lys Phe
       160                 165                 170
   cgc aga cct gac atc cag tac cct gat gct aca gac gag gac atc acc    637
   Arg Arg Pro Asp Ile Gln Tyr Pro Asp Ala Thr Asp Glu Asp Ile Thr
   175                 180                 185                 190
   tca cac atg gaa agc gag gag ttg aat ggt gca tac aag gcc atc ccc    685
   Ser His Met Glu Ser Glu Glu Leu Asn Gly Ala Tyr Lys Ala Ile Pro
                   195                 200                 205
   gtt gcc cag gac ctg aac gcg cct tct gat tgg gac agc cgt ggg aag    733
   Val Ala Gln Asp Leu Asn Ala Pro Ser Asp Trp Asp Ser Arg Gly Lys
               210                 215                 220
   gac agt tat gaa acg agt cag ctg gat gac cag agt gct gaa acc cac    781
   Asp Ser Tyr Glu Thr Ser Gln Leu Asp Asp Gln Ser Ala Glu Thr His
           225                 230                 235
   agc cac aag cag tcc aga tta tat aag cgg aaa gcc aat gat gag agc    829
   Ser His Lys Gln Ser Arg Leu Tyr Lys Arg Lys Ala Asn Asp Glu Ser
       240                 245                 250
   aat gag cat tcc gat gtg att gat agt cag gaa ctt tcc aaa gtc agc    877
   Asn Glu His Ser Asp Val Ile Asp Ser Gln Glu Leu Ser Lys Val Ser
   255                 260                 265                 270
   cgt gaa ttc cac agc cat gaa ttt cac agc cat gaa gat atg ctg gtt    925
   Arg Glu Phe His Ser His Glu Phe His Ser His Glu Asp Met Leu Val
                   275                 280                 285
   gta gac ccc aaa agt aag gaa gaa gat aaa cac ctg aaa ttt cgt att    973
   Val Asp Pro Lys Ser Lys Glu Glu Asp Lys His Leu Lys Phe Arg Ile
               290                 295                 300
   tct cat gaa tta gat agt gca tct tct gag gtc aat taa aaggagaaaa    1022
   Ser His Glu Leu Asp Ser Ala Ser Ser Glu Val Asn
           305                 310                 315
   aatacaattt ctcactttgc atttagtcaa aagaaaaaat gctttatagc aaaatgaaag 1082
   agaacatgaa atgcttcttt ctcagtttat tggttgaatg tgtatctatt tgagtctgga 1142
   aataactaat gtgtttgata attagtttag tttgtggctt catggaaact ccctgtaaac 1202
   taaaagcttc agggttatgt ctatgttcat tctatagaag aaatgcaaac tatcactgta 1262
   ttttaatatt tgttattctc tcatgaatag aaatttatgt agaagcaaac aaaatacttt 1322
   tacccactta aaaagagaat ataacatttt atgtcactat aatcttttgt tttttaagtt 1382
   agtgtatatt ttgttgtgat tatctttttg tggtgtgaat aa 1424
<210> 14
   <211> 314
   <212> PRT
   <213> Homo sapiens
<400> 14
   Met Arg Ile Ala Val Ile Cys Phe Cys Leu Leu Gly Ile Thr Cys Ala
    1               5                  10                  15
   Ile Pro Val Lys Gln Ala Asp Ser Gly Ser Ser Glu Glu Lys Gln Leu
               20                  25                  30
   Tyr Asn Lys Tyr Pro Asp Ala Val Ala Thr Trp Leu Asn Pro Asp Pro
           35                  40                  45
   Ser Gln Lys Gln Asn Leu Leu Ala Pro Gln Asn Ala Val Ser Ser Glu
       50                  55                  60
   Glu Thr Asn Asp Phe Lys Gln Glu Thr Leu Pro Ser Lys Ser Asn Glu
    65                  70                  75                  80
   Ser His Asp His Met Asp Asp Met Asp Asp Glu Asp Asp Asp Asp His
                   85                  90                  95
   Val Asp Ser Gln Asp Ser Ile Asp Ser Asn Asp Ser Asp Asp Val Asp
               100                 105                 110
   Asp Thr Asp Asp Ser His Gln Ser Asp Glu Ser His His Ser Asp Glu
           115                 120                 125
   Ser Asp Glu Leu Val Thr Asp Phe Pro Thr Asp Leu Pro Ala Thr Glu
       130                 135                 140
   Val Phe Thr Pro Val Val Pro Thr Val Asp Thr Tyr Asp Gly Arg Gly
   145                 150                 155                 160
   Asp Ser Val Val Tyr Gly Leu Arg Ser Lys Ser Lys Lys Phe Arg Arg
                   165                 170                 175
   Pro Asp Ile Gln Tyr Pro Asp Ala Thr Asp Glu Asp Ile Thr Ser His
               180                 185                 190
   Met Glu Ser Glu Glu Leu Asn Gly Ala Tyr Lys Ala Ile Pro Val Ala
           195                 200                 205
   Gln Asp Leu Asn Ala Pro Ser Asp Trp Asp Ser Arg Gly Lys Asp Ser
       210                 215                 220
   Tyr Glu Thr Ser Gln Leu Asp Asp Gln Ser Ala Glu Thr His Ser His
   225                 230                 235                 240
   Lys Gln Ser Arg Leu Tyr Lys Arg Lys Ala Asn Asp Glu Ser Asn Glu
                   245                 250                 255
   His Ser Asp Val Ile Asp Ser Gln Glu Leu Ser Lys Val Ser Arg Glu
               260                 265                 270
   Phe His Ser His Glu Phe His Ser His Glu Asp Met Leu Val Val Asp
           275                 280                 285
   Pro Lys Ser Lys Glu Glu Asp Lys His Leu Lys Phe Arg Ile Ser His
       290                 295                 300
   Glu Leu Asp Ser Ala Ser Ser Glu Val Asn
   305                 310
<210> 15
   <211> 2682
   <212> DNA
   <213> Homo sapiens
<220>
   <221> CDS
   <222> (100)..(1641)
<400> 15
   tgggcataga tttcctcttt gagaacatca acctgatttt tgagactttt tgaaaaaatt 60
   ctttgtgaac tacggagggt agaggtatca cacccaact atg aag atc agc atc    114
                                              Met Lys Ile Ser Ile
                                               1               5
   ctg ctc atg ttc ctt tgg gga tta tcc tgt gct ctc cca gta acc agg   162
   Leu Leu Met Phe Leu Trp Gly Leu Ser Cys Ala Leu Pro Val Thr Arg
                   10                  15                  20
   tat caa aat aat gaa tct gag gat tct gaa gaa tgg aag ggt cat ttg   210
   Tyr Gln Asn Asn Glu Ser Glu Asp Ser Glu Glu Trp Lys Gly His Leu
               25                  30                  35
   gct cag gca cca aca cca ccc ttg gag agc agt gag tca tca gaa ggc   258
   Ala Gln Ala Pro Thr Pro Pro Leu Glu Ser Ser Glu Ser Ser Glu Gly
           40                  45                  50
   agt aaa gtt agc tca gag gaa cag gca aat gaa gac ccc agt gac agc   306
   Ser Lys Val Ser Ser Glu Glu Gln Ala Asn Glu Asp Pro Ser Asp Ser
       55                  60                  65
   act cag tca gag gag ggc ctg ggc tct gat gat cat caa tac att tat   354
   Thr Gln Ser Glu Glu Gly Leu Gly Ser Asp Asp His Gln Tyr Ile Tyr
   70                  75                  80                  85
   agg cta gct ggt ggc ttc tcc agg agc aca gga aaa gga gga gat gat   402
   Arg Leu Ala Gly Gly Phe Ser Arg Ser Thr Gly Lys Gly Gly Asp Asp
                   90                  95                  100
   aaa gat gac gat gaa gat gac agt gga gat gac acc ttt ggt gac gat   450
   Lys Asp Asp Asp Glu Asp Asp Ser Gly Asp Asp Thr Phe Gly Asp Asp
               105                 110                 115
   gac agt ggc cca ggg ccc aaa gac aga caa gaa gga gga aac tcc aga   498
   Asp Ser Gly Pro Gly Pro Lys Asp Arg Gln Glu Gly Gly Asn Ser Arg
           120                 125                 130
   ctg gga agt gat gag gac tct gat gac acc ata caa gcc agt gaa gag   546
   Leu Gly Ser Asp Glu Asp Ser Asp Asp Thr Ile Gln Ala Ser Glu Glu
       135                 140                 145
   agt gcc cca caa ggg caa gac agt gcc caa gat acc acc agt gag agc   594
   Ser Ala Pro Gln Gly Gln Asp Ser Ala Gln Asp Thr Thr Ser Glu Ser
   150                 155                 160                 165
   agg gaa ctt gac aat gag gac cgg gtg gac agc aag cct gag gga ggt   642
   Arg Glu Leu Asp Asn Glu Asp Arg Val Asp Ser Lys Pro Glu Gly Gly
                   170                 175                 180
   gac tcc act caa gag agt gag agt gaa gag cac tgg gtg gga ggt ggc   690
   Asp Ser Thr Gln Glu Ser Glu Ser Glu Glu His Trp Val Gly Gly Gly
                   185                     190                     195
   agt gat ggg gag agc agc cat gga gac ggc tcc gag ttg gac gat gag      738
   Ser Asp Gly Glu Ser Ser His Gly Asp Gly Ser Glu Leu Asp Asp Glu
           200                 205                 210
   gga atg cag agt gat gac cca gag agc atc agg agt gaa agg gga aac      786
   Gly Met Gln Ser Asp Asp Pro Glu Ser Ile Arg Ser Glu Arg Gly Asn
       215                 220                 225
   tcc aga atg aac agt gca ggc atg aaa tca aaa gaa tct gga gaa aac      834
   Ser Arg Met Asn Ser Ala Gly Met Lys Ser Lys Glu Ser Gly Glu Asn
   230                 235                 240                 245
   agt gag caa gca aac act caa gat tca ggt ggc agc caa ttg ctg gag      882
   Ser Glu Gln Ala Asn Thr Gln Asp Ser Gly Gly Ser Gln Leu Leu Glu
                   250                 255                 260
   cat ccc agt agg aaa att ttt agg aag tct cgc atc tca gag gaa gat      930
   His Pro Ser Arg Lys Ile Phe Arg Lys Ser Arg Ile Ser Glu Glu Asp
               265                 270                 275
   gac aga agc gag ctt gat gac aac aac aca atg gaa gaa gtc aag agt      978
   Asp Arg Ser Glu Leu Asp Asp Asn Asn Thr Met Glu Glu Val Lys Ser
           280                 285                 290
   gac tct aca gaa aac agc aac tcc aga gac act ggc ctc agc caa ccc      1026
   Asp Ser Thr Glu Asn Ser Asn Ser Arg Asp Thr Gly Leu Ser Gln Pro
       295                 300                 305
   agg aga gac agc aag ggt gac tct caa gaa gac agc aag gag aat ctg      1074
   Arg Arg Asp Ser Lys Gly Asp Ser Gln Glu Asp Ser Lys Glu Asn Leu
   310                 315                 320                 325
   tcc cag gaa gag agc caa aac gta gat ggt ccc agc agt gag tcc agc      1122
   Ser Gln Glu Glu Ser Gln Asn Val Asp Gly Pro Ser Ser Glu Ser Ser
                   330                 335                 340
   caa gag gcc aac ctg tca tct caa gag aac agc agt gag tct cag gaa      1170
   Gln Glu Ala Asn Leu Ser Ser Gln Glu Asn Ser Ser Glu Ser Gln Glu
               345                 350                 355
   gag gtg gtg agt gag tcc agg gga gat aac ccc gac ccc aca act agt      1218
   Glu Val Val Ser Glu Ser Arg Gly Asp Asn Pro Asp Pro Thr Thr Ser
           360                 365                 370
   tat gta gaa gac cag gaa gac agt gac tcc agc gag gag gac agc tcg      1266
   Tyr Val Glu Asp Gln Glu Asp Ser Asp Ser Ser Glu Glu Asp Ser Ser
       375                 380                 385
   cac aca ctc tcc cac tca aaa agt gaa tcc aga gag gag caa gca gac      1314
   His Thr Leu Ser His Ser Lys Ser Glu Ser Arg Glu Glu Gln Ala Asp
   390                 395                 400                 405
   agc gaa tcc agt gag agc ctc aac ttc tca gag gaa agc ccg gag tcc      1362
   Ser Glu Ser Ser Glu Ser Leu Asn Phe Ser Glu Glu Ser Pro Glu Ser
                   410                 415                 420
   cct gag gat gag aac agc tcc agc cag gag ggc ctc cag tct cac agc      1410
   Pro Glu Asp Glu Asn Ser Ser Ser Gln Glu Gly Leu Gln Ser His Ser
               425                 430                 435
   agc tca gca gag agt cag agc gag gaa agc cat tct gag gaa gac gac    1458
   Ser Ser Ala Glu Ser Gln Ser Glu Glu Ser His Ser Glu Glu Asp Asp
           440                 445                 450
   agt gac tct caa gac agc agc aga tcc aaa gaa gat agc aac tcc acg    1506
   Ser Asp Ser Gln Asp Ser Ser Arg Ser Lys Glu Asp Ser Asn Ser Thr
       455                 460                 465
   gag agc aaa tca agc agt gag gaa gat ggc cag ttg aaa aac att gag    1554
   Glu Ser Lys Ser Ser Ser Glu Glu Asp Gly Gln Leu Lys Asn Ile Glu
   470                 475                 480                 485
   ata gag agc cgg aaa tta aca gtt gat gcc tat cac aac aaa ccc att    1602
   Ile Glu Ser Arg Lys Leu Thr Val Asp Ala Tyr His Asn Lys Pro Ile
                   490                 495                 500
   ggg gac caa gat gac aat gac tgc caa gac ggc tat tag catcagctgt     1651
   Gly Asp Gln Asp Asp Asn Asp Cys Gln Asp Gly Tyr
               505                 510
   cctaagaagc agttgtcaca taaaggagtc ttagggactt gaaaatgtat catgataact 1711
   ataatttatt gatgttttga tcaaaagaat aaccagatgc catatttttc ctgaaaggaa 1771
   ttgctggaca ttacacttgt ttttagggtg tcatcatttc acagaggttt aaatactgtg 1831
   gagtgacacc agtaacacag ccaaagaggc tagtaagcaa gaaaggatct gcatgataac 1891
   tttgcagctg agatagttcc taattcatca acgtaacaaa caaagctatt gggtgtccat 1951
   gtatatacca ggcactatgc taggtgttga gaatgtaaag caggttaaga cttgtttctt 2011
   gctcctgagg agcgtataga ggggacccac gtagctaaca gagttaacag aggggtatgt 2071
   ataagagaaa cgtgaatagt tacaacccag tatgctgagt gccaaggcca gtgaggctat 2131
   gaacacgata tcaatctggg gacactgaga aggataacca atgcgttgga gaacgaggga 2191
   ggacttcata gcagaagaat tggagttgga aggttgcata gggtttcctc attggagacc 2251
   ggggaattct attcttggga taaataatag taaattctat aggattcaat acttagtttg 2311
   caaaagcact ttgaaattat ggcacagtga gttgttttgg ataagaaaat ctttttctcc 2371
   caattaattt accaattcat catttttttt ttttgtagaa ctcccataaa catcacctta 2431
   ctaatcactg gtgatgataa gaaggattgg gtcaggaaga gtgggagaaa gaaattcctc 2491
   tttacgtaga tactttttag ctttattttt tctaaaatca gtttgcgtgc aatgctagaa 2551
   aaaaactgtt ctctgagtcc tttacagagc aaaattctgt atgtaagatt caattgattt 2611
   ttgacaaata ccatttgaaa tattacctca acataaaata cttgttttgt aataaagatt 2671
   ataataccca g                                                      2682
<210> 16
   <211> 513
   <212> PRT
   <213> Homo sapiens
<400> 16
   Met Lys Ile Ser Ile Leu Leu Met Phe Leu Trp Gly Leu Ser Cys Ala
    1               5                  10                  15
   Leu Pro Val Thr Arg Tyr Gln Asn Asn Glu Ser Glu Asp Ser Glu Glu
               20                  25                  30
   Trp Lys Gly His Leu Ala Gln Ala Pro Thr Pro Pro Leu Glu Ser Ser
           35                  40                  45
   Glu Ser Ser Glu Gly Ser Lys Val Ser Ser Glu Glu Gln Ala Asn Glu
       50                  55                  60
   Asp Pro Ser Asp Ser Thr Gln Ser Glu Glu Gly Leu Gly Ser Asp Asp
   65                  70                  75                  80
   His Gln Tyr Ile Tyr Arg Leu Ala Gly Gly Phe Ser Arg Ser Thr Gly
                   85                  90                  95
   Lys Gly Gly Asp Asp Lys Asp Asp Asp Glu Asp Asp Ser Gly Asp Asp
               100                 105                 110
   Thr Phe Gly Asp Asp Asp Ser Gly Pro Gly Pro Lys Asp Arg Gln Glu
           115                 120                 125
   Gly Gly Asn Ser Arg Leu Gly Ser Asp Glu Asp Ser Asp Asp Thr Ile
       130                 135                 140
   Gln Ala Ser Glu Glu Ser Ala Pro Gln Gly Gln Asp Ser Ala Gln Asp
   145                 150                 155                 160
   Thr Thr Ser Glu Ser Arg Glu Leu Asp Asn Glu Asp Arg Val Asp Ser
                   165                 170                 175
   Lys Pro Glu Gly Gly Asp Ser Thr Gln Glu Ser Glu Ser Glu Glu His
               180                 185                 190
   Trp Val Gly Gly Gly Ser Asp Gly Glu Ser Ser His Gly Asp Gly Ser
           195                 200                 205
   Glu Leu Asp Asp Glu Gly Met Gln Ser Asp Asp Pro Glu Ser Ile Arg
       210                 215                 220
   Ser Glu Arg Gly Asn Ser Arg Met Asn Ser Ala Gly Met Lys Ser Lys
   225                 230                 235                 240
   Glu Ser Gly Glu Asn Ser Glu Gln Ala Asn Thr Gln Asp Ser Gly Gly
                   245                 250                 255
   Ser Gln Leu Leu Glu His Pro Ser Arg Lys Ile Phe Arg Lys Ser Arg
               260                 265                 270
   Ile Ser Glu Glu Asp Asp Arg Ser Glu Leu Asp Asp Asn Asn Thr Met
           275                 280                 285
   Glu Glu Val Lys Ser Asp Ser Thr Glu Asn Ser Asn Ser Arg Asp Thr
       290                 295                 300
   Gly Leu Ser Gln Pro Arg Arg Asp Ser Lys Gly Asp Ser Gln Glu Asp
   305                 310                 315                 320
   Ser Lys Glu Asn Leu Ser Gln Glu Glu Ser Gln Asn Val Asp Gly Pro
                   325                 330                 335
   Ser Ser Glu Ser Ser Gln Glu Ala Asn Leu Ser Ser Gln Glu Asn Ser
               340                 345                 350
   Ser Glu Ser Gln Glu Glu Val Val Ser Glu Ser Arg Gly Asp Asn Pro
           355                 360                 365
   Asp Pro Thr Thr Ser Tyr Val Glu Asp Gln Glu Asp Ser Asp Ser Ser
       370                 375                 380
   Glu Glu Asp Ser Ser His Thr Leu Ser His Ser Lys Ser Glu Ser Arg
   385                 390                 395                 400
   Glu Glu Gln Ala Asp Ser Glu Ser Ser Glu Ser Leu Asn Phe Ser Glu
                   405                 410                 415
   Glu Ser Pro Glu Ser Pro Glu Asp Glu Asn Ser Ser Ser Gln Glu Gly
               420                 425                 430
   Leu Gln Ser His Ser Ser Ser Ala Glu Ser Gln Ser Glu Glu Ser His
           435                 440                 445
   Ser Glu Glu Asp Asp Ser Asp Ser Gln Asp Ser Ser Arg Ser Lys Glu
       450                 455                 460
   Asp Ser Asn Ser Thr Glu Ser Lys Ser Ser Ser Glu Glu Asp Gly Gln
   465                 470                 475                 480
   Leu Lys Asn Ile Glu Ile Glu Ser Arg Lys Leu Thr Val Asp Ala Tyr
                   485                 490                 495
   His Asn Lys Pro Ile Gly Asp Gln Asp Asp Asn Asp Cys Gln Asp Gly
               500                 505                 510
   Tyr
<210> 17
   <211> 8201
   <212> DNA
   <213> Homo sapiens
<220>
   <221> exon
   <222> (2387)..(2437)
<220>
   <221> exon
   <222> (3577)..(3660)
<220>
   <221> exon
   <222> (3794)..(4780)
<220>
   <221> exon
   <222> (5257)..(7896)
<400> 17
   attgtcatgc aaaagtccag gacagtgggc cactttcagt cttcaaagag aaagataaga 60
   aattctggat tttcaaaatc cttttgaagc cttttaaggt aagatgaaat atccttttta 120
   ctcagaacca actgattcat ttagaaagaa ctttgaattt caaagatgaa gccagtttga 180
   ttttaagaag cgagtacccc ttaatgatta gattgtatgc ttcctttttg acttgtcata 240
   ttgatagtat gtataaaaga taacggacga ttacgaccta aggaagagat agattgggaa 300
   gaagaaagac ctcgtactga aaaattggcc aactgaggtg gaaatttgac aattaactat 360
   ctgggcactt tgattagttt tgataaaaaa tgagataact cagatttcaa aaatccacct 420
   tgggctttca aacaaggctt caattaggct ttgcttttta gtattttatt acttactatt 480
   acttattatt tattgtccca catgaaatga aatttagcaa tcactaatga tgccaaatct 540
   aattgctaaa tgaaatgaag ctaaatctca tttcattagt aacaataaat gaaataatct 600
   gatggagctt cacaaattct gaagtctttg tttcatgctg aggtcacctg ggccattttt 660
   attgtagtct tcgaagtcat tcacctgcct tggaaacggt gataaccatc atggaattgt 720
   tcaggagtgg agctgaaaga gagatgtagt ggtcagattt ctgaactgta gctcagaaac 780
   tggacacgta tcactctggc cttggctgca ggtacctttc cagtatgctg aggctcttcc 840
   aaatcacagt gcagacgggc cttctgcaga gctatgtaat gattaggctt gggactgcaa 900
   agtacaggat aactgtggct tagtaaacag ctggccttca acatctgtgc cccagagctc 960
   tgcatgatac ttgtcctggt gtcacctcag cctcacttga atctatggca tttcagaagg 1020
   agctctagct gttcttggct ttctgttgaa cagctataag aatgagcact tttttccctc 1080
   tcagtagctc tggaactgtg tcatctctcc tgtgagaaaa cgccagtaat tctcatgaca 1140
   gttgatattc agtgaagttt tattatattt tcactaccac cattaaattc aatcaaagcc 1200
   attttatgac atgcagcatt ataatctata catctggtgg gagttcatga aataggagta 1260
   aaactctcct ttctatcatt acttcaagaa atccaacttg caatataaat taattttttt 1320
   actcacacag attataaaat gtctattcca acttatcaga aacatgtttt agaccatttc 1380
   tgaatttgaa ttctaacagg gatgaagaat catgatttta gaagtcccat aaaataattg 1440
   ctatcattta ttcaaaaatt gcaaagtgcc tgaagcaatg ctagatattg ctgatagtca 1500
   taaatattta tcaacaacat tcagaaaacg tttttttctg tgctttgcat tggaatacaa 1560
   taatcaccaa gacactctcc tgggcctcag gagcttacag gaaatcaggg caacacataa 1620
   gtaactaggc aattttaaac agtgcaatgc gttaccagtg agacgtgcaa acttccttgg 1680
   tataaaaagg aaagagatac caaataccct ttgaagtggc gtcagagagg gcgtctcaga 1740
   gataattcta ccaaacttca ggataatcct gaggtgcagg tgttgttatt attccaggtg 1800
   gagggataat aaacctactt aaatttctca agcttacaca gcaagtagca ggggtaacat 1860
   ttgaacccag gtctctgaat acaaaccccg tattctttcc actagcgtag gctccctcat 1920
   gttagtaatt tctttctctt aaagtctggt atagctcaat tctatagatt tggagtaagg 1980
   atgacaagtg ttttaccttt gaagcacaat ttcagcagaa ttagttagta cttgattaaa 2040
   gctattcaga agagaaatag atgtttttac acccaagaat tgcagaagaa caaagttaca 2100
   gctatgccct ttgtacctat tatggtgttt tccttcattg gcacaggcag aaaaaaatct 2160
   aggaagctac attagtgctg agcctggtga tgtccccata accacaccag gtatgttctg 2220
   gaccatcgta tgtcttctcg tgttagatac atgcttcttg tccaggaaaa gggcaaatgc 2280
   ttacacatca aaataatata gtactatgat tttcccttta ctttataagt aattttgtgc 2340
   tgttcctttt ttatacagcc attgattatt attattccta aagaaaatga agataattac 2400
   atatttttgc atttgggcag tagcatgggc cattccagta agtatgcctt tcttagaaaa 2460
   cctcttcact ttgttatctt ttttaaccta acattaatac aaaatgtagt gtgtgtgtgt 2520
   gtgtgtgtgt gtgtgtgtgt gtgcatgtac atgtgtgtat atatgtgtgt gtgtatatat 2580
   gtttccttaa ttttttttaa caggctgagt ctaaacattt agatttgcac taagggcttt 2640
   atgtgatatc tgtgaggttt caacaaaacc actccaattc atcgtctcat tcctctatag 2700
   aaactcatat ctcgtctgaa ggattattat tatttaaaac atttattcag attaatttac 2760
   acttaatgcc cagaagtcat ggagactttg tccatctttg cttcatactc tgtgaatttc 2820
   attctaatac gaacaaagtc tgtgctgttt aggaagtttc caagaaagaa taataagaaa 2880
   aagtagattt tttttcaaca tataggagac taatttttca ctcagagtta ttatttatgt 2940
   gctcactgtg gaaaatttgg aatatatgac gaaaaccaat aaaaaattga gaaaattcaa 3000
   ccatttataa ttttactagc cagccatcat gtttaacatt ttcatatgct ttcataatac 3060
   caaacatttg gtatttatgt agttgaaaat gttctcaagt atttcaaatg tgctcttgca 3120
   gagcacagaa gtatactagc gtaatacttg attttgcttc tgtgcaggct ctggtcacgc 3180
   ctcctgttct cttaagagtt ttcatcagga ttacacttag agcgggtttg tgctagtgca 3240
   agaggctttt tgtagagaaa caccagaggt ctatcccctc gtctttctac aagactcttt 3300
   ccttctacag ttgagataag tgggctgatc taacacgtcc ataaaattgg taataccaca 3360
   gtgaaaaata tccatgtacc cagtttaaat tctacacaag ccctgtaaga agccacttct 3420
   cttttctatc tgattagatc atactttggc ctttgtgtta aacctttctt cttcatggag 3480
   ggaagaatat ttgtgtgtgt gtgtgtgtgt gtgcacgctc acacacatat tcacaaataa 3540
   gaacctttcc aatagccagt attttctact tggcaggttc ctcaaagcaa accactggag 3600
   agacatgtcg aaaaatccat gaatttgcat ctcctagcaa gatcaaatgt gtcagtacag 3660
   gtataggatg taatatattt cattttattt cctatttctg agttgctaca ttccattaac 3720
   ttctccaaga ttgcaatttg ctttccttca agatcattga cactcataat tgattgaatt 3780
   gtttcttttt caggatgagt taaatgccag tggaaccatc aaagaaagtg gtgtcctggt 3840
   gcatgaaggt gatagaggaa ggcaagagaa tacccaagat ggtcacaagg gagaagggaa 3900
   tggctctaag tgggcagaag taggagggaa gagtttttct acatattcca cattagcaaa 3960
   cgaagagggg aatattgagg gctggaatgg ggacacagga aaagcagaaa catatggtca 4020
   tgatggaata catgggaaag aagaaaacat cacagcaaat ggcatccagg gacaagtaag 4080
   catcattgac aatgctggag ccacaaacag aagcaacact aatggaaata ctgataagaa 4140
   tacccaaaat ggggatgttg gcgatgcagg tcacaatgag gatgtcgctg ttgtccaaga 4200
   agatggacct caagtagctg gaagcaataa cagtacagac aatgaggatg aaataattga 4260
   gaattcctgt agaaacgagg gtaatacaag tgaaataaca cctcagatca acagcaagag 4320
   aaatgggact aaggaagctg aggtaacacc aggcactgga gaagatgctg gcctggataa 4380
   ttccgatggg agtcctagtg ggaatggagc agatgaggat gaagacgagg gttctggtga 4440
   tgatgaagat gaagaagcag ggaatggaaa agacagtagt aataacagca agggccagga 4500
   gggccaggac catgggaaag aagatgatca tgatagtagc ataggtcaaa attcggatag 4560
   taaagaatat tatgaccctg aaggcaaaga agatccccat aatgaagttg atggagacaa 4620
   gacctccaag agtgaggaga attctgctgg tattccagaa gacaatggca gccaaagaat 4680
   agaggacacc cagaagctca accatagaga aagcaaacgc gtagaaaata gaatcaccaa 4740
   agaatcagag acacatgctg ttgggaagag ccaagataag gttagtttgt aaagctgatt 4800
   tctttcaatg gcagtttaaa ttcttcccct ccatctattg atgctagcac aaaaataaac 4860
   catgacaagc atccatgtat ttttgtatcc atattacttg actatttaag gaaatctaga 4920
   gtccttacta gacttcgaga tagaacaact ttaaacatct tacatttctg ataacttagt 4980
   tataattcta gaaaagtctt atgtgaaatc atggatcccc atgtaattgt ttacaaaagt 5040
   tcctactggg taggaatgtg gatgaatttt taaggaatct aagcaccagg atgctttcaa 5100
   ttacagaata aagcacattt tcacaaataa ctgtgaagta ctagaaatgt aactcctatc 5160
   cctatggcaa cttttcccag ttattcttcc tcagatcaat gcaattttgc agcaaatatt 5220
   cactagttaa tcattctttc ctccatcctt ccatagggaa tagaaatcaa gggtcccagc 5280
   agtggcaaca gaaatattac caaagaagtt gggaaaggca acgaaggtaa agaggataaa 5340
   ggacaacatg gaatgatctt gggcaaaggc aatgtcaaga cacaaggaga ggttgtcaac 5400
   atagaaggac ctggccaaaa atcagaacca ggaaataaag ttggacacag caatacaggt 5460
   agtgacagca atagtgatgg atatgacagt tatgattttg atgataagtc catgcaagga 5520
   gatgatccca atagcagtga tgaatctaat ggcaatgatg atgctaattc agaaagtgac 5580
   aataacagca gtagccgagg agatgcttct tataactctg atgaatcaaa agataatggc 5640
   aatggcagtg actcaaaagg agcagaagat gatgacagtg atagcacatc agacactaat 5700
   aatagtgaca gtaatggcaa tggtaacaat gggaatgatg acaatgacaa atcagacagt 5760
   ggcaaaggta aatcagatag cagtgacagt gatagtagtg atagcagcaa tagcagtgat 5820
   agtagtgaca gcagtgacag tgacagcagt gatagcaaca gtagcagtga tagtgacagc 5880
   agtgacagtg acagcagtga tagcagtgac agtgatagta gtgatagcag caatagcagt 5940
   gacagtagtg acagcagtga tagcagtgac agtagtgata gtagtgacag cagtgacagc 6000
   aagtcagaca gcagcaaatc agagagcgac agcagtgata gtgacagtaa gtcagacagc 6060
   agtgacagca acagcagtga cagtagtgac aacagtgata gcagcgacag cagcaatagc 6120
   agtaacagca gtgatagtag tgacagcagt gatagcagtg acagcagcag tagcagtgac 6180
   agcagcagta gcagtgacag cagcaacagc agtgatagta gtgacagtag tgacagcagc 6240
   aatagcagtg agagcagtga tagtagtgac agcagtgata gtgacagcag tgatagtagt 6300
   gacagcagta atagtaacag cagcgatagt gacagcagca acagcagcga tagcagtgac 6360
   agcagtgata gcagtgacag cagcaacagc agtgacagta gcgatagcag tgacagcagc 6420
   aacagcagtg acagcagtga tagcagtgac agcagtgata gtagtgacag cagcaacagc 6480
   agtgatagca acgacagcag caatagcagt gacagcagtg atagcagcaa cagcagtgat 6540
   agcagcaaca gcagtgatag cagtgatagc agtgacagca gtgatagcga cagcagcaat 6600
   agcagtgaca gcagtaatag tagtgacagc agcgatagca gcaacagcag tgatagcagc 6660
   gacagcagcg atagcagtga cagcagtgat agcgacagca gcaatagaag tgacagtagt 6720
   aatagtagtg acagcagcga tagcagtgac agcagcaaca gcagtgacag cagtgatagt 6780
   agtgacagca gtgacagcaa cgaaagcagc aatagcagtg acagcagtga tagcagcaac 6840
   agcagtgata gtgacagcag tgatagcagc aacagcagtg acagcagtga tagcagcaac 6900
   agcagtgata gcagtgaaag cagtaatagt agtgacaaca gcaatagcag tgacagcagc 6960
   aacagcagtg acagcagtga tagcagtgac agcagtaata gtagtgacag cagcaatagc 7020
   ggtgacagca gcaacagcag tgacagcagt gatagcaata gcagcgacag cagtgacagc 7080
   agcaacagca gcgatagcag tgacagcagt gatagcagtg acagcagtga cagcagtgat 7140
   agcagcaaca gcagtgatag cagtgacagc agtgacagca gtgatagcag taatagtagt 7200
   gacagcagca acagcagtga cagcagcgat agcagtgaca gcagcgatag cagtgacagc 7260
   agtgacagca gcaatagcag tgacagcagt gacagcagcg acagcagtga tagcagtgac 7320
   agcagtggca gcagcgacag cagtgatagc agtgacagca gtgatagcag cgatagcagt 7380
   gacagcagcg acagcagtga cagcagtgac agcagtgaaa gcagcgacag cagcgatagc 7440
   agcgacagca gtgacagcag cgacagcagt gacagcagcg atagcagcga cagcagcgac 7500
   agcagcgata gcagtgacag cagcaatagc agtgatagca gcgacagcag tgatagcagt 7560
   gacagcagcg acagcagcga tagcagcgac agcagtgata gtagtgatag cagtgacagc 7620
   agtgacagca gcgacagcag tgacagcagc gacagcagtg acagcagcga cagcagtgac 7680
   agcaatgaaa gcagcgacag cagtgacagc agcgatagca gtgacagcag caacagcagt 7740
   gacagcagcg acagcagtga tagcagtgac agcacatctg acagcaatga tgagagtgac 7800
   agccagagca agtctggtaa cggtaacaac aatggaagtg acagtgacag tgacagtgaa 7860
   ggcagtgaca gtaaccactc aaccagtgat gattagaaca aaagaaaaac ccataagatt 7920
   ccttttgtga aaagtttggt aatgggatag gaaaaaaaga tttccaagaa agtaaagaaa 7980
   ggggagaaat aaacataaga cgtatgtaaa caaaaacaac tgggggaatc aaatcaaaca 8040
   gttggattca gaaccaagac ctaactcctg cagagacaga ctctgaatgc atgacctttg 8100
   gtacatgcct gttaatattc atgttctgaa aatattttgt taaaagtgta aatctaaaca 8160
   taaaagaaca attaaaatat tctttaatac ttcacacaga a                     8201
<210> 18
   <211> 1253
   <212> PRT
   <213> Homo sapiens
<400> 18
   Met Lys Ile Ile Thr Tyr Phe Cys Ile Trp Ala Val Ala Trp Ala Ile
    1               5                  10                  15
   Pro Val Pro Gln Ser Lys Pro Leu Glu Arg His Val Glu Lys Ser Met
               20                  25                  30
   Asn Leu His Leu Leu Ala Arg Ser Asn Val Ser Val Gln Asp Glu Leu
   35 40 45
   Asn Ala Ser Gly Thr Ile Lys Glu Ser Gly Val Leu Val His Glu Gly
   50 55 60
   Asp Arg Gly Arg Gln Glu Asn Thr Gln Asp Gly His Lys Gly Glu Gly
   65 70 75 80
   Asn Gly Ser Lys Trp Ala Glu Val Gly Gly Lys Ser Phe Ser Thr Tyr
   85 90 95
   Ser Thr Leu Ala Asn Glu Glu Gly Asn Ile Glu Gly Trp Asn Gly Asp
   100 105 110
   Thr Gly Lys Ala Glu Thr Tyr Gly His Asp Gly Ile His Gly Lys Glu
   115 120 125
   Glu Asn Ile Thr Ala Asn Gly Ile Gln Gly Gln Val Ser Ile Ile Asp
   130 135 140
   Asn Ala Gly Ala Thr Asn Arg Ser Asn Thr Asn Gly Asn Thr Asp Lys
   145 150 155 160
   Asn Thr Gln Asn Gly Asp Val Gly Asp Ala Gly His Asn Glu Asp Val
   165 170 175
   Ala Val Val Gln Glu Asp Gly Pro Gln Val Ala Gly Ser Asn Asn Ser
   180 185 190
   Thr Asp Asn Glu Asp Glu Ile Ile Glu Asn Ser Cys Arg Asn Glu Gly
   195 200 205
   Asn Thr Ser Glu Ile Thr Pro Gln Ile Asn Ser Lys Arg Asn Gly Thr
   210 215 220
   Lys Glu Ala Glu Val Thr Pro Gly Thr Gly Glu Asp Ala Gly Leu Asp
   225 230 235 240
   Asn Ser Asp Gly Ser Pro Ser Gly Asn Gly Ala Asp Glu Asp Glu Asp
   245 250 255
   Glu Gly Ser Gly Asp Asp Glu Asp Glu Glu Ala Gly Asn Gly Lys Asp
   260 265 270
   Ser Ser Asn Asn Ser Lys Gly Gln Glu Gly Gln Asp His Gly Lys Glu
   275 280 285
   Asp Asp His Asp Ser Ser Ile Gly Gln Asn Ser Asp Ser Lys Glu Tyr
   290 295 300
   Tyr Asp Pro Glu Gly Lys Glu Asp Pro His Asn Glu Val Asp Gly Asp
   305 310 315 320
   Lys Thr Ser Lys Ser Glu Glu Asn Ser Ala Gly Ile Pro Glu Asp Asn
   325 330 335
   Gly Ser Gln Arg Ile Glu Asp Thr Gln Lys Leu Asn His Arg Glu Ser
   340 345 350
   Lys Arg Val Glu Asn Arg Ile Thr Lys Glu Ser Glu Thr His Ala Val
   355 360 365
   Gly Lys Ser Gln Asp Lys Gly Ile Glu Ile Lys Gly Pro Ser Ser Gly
   370 375 380
   Asn Arg Asn Ile Thr Lys Glu Val Gly Lys Gly Asn Glu Gly Lys Glu
   385 390 395 400
   Asp Lys Gly Gln His Gly Met Ile Leu Gly Lys Gly Asn Val Lys Thr
   405 410 415
   Gln Gly Glu Val Val Asn Ile Glu Gly Pro Gly Gln Lys Ser Glu Pro
   420 425 430
   Gly Asn Lys Val Gly His Ser Asn Thr Gly Ser Asp Ser Asn Ser Asp
   435 440 445
   Gly Tyr Asp Ser Tyr Asp Phe Asp Asp Lys Ser Met Gln Gly Asp Asp
   450 455 460
   Pro Asn Ser Ser Asp Glu Ser Asn Gly Asn Asp Asp Ala Asn Ser Glu
   465 470 475 480
   Ser Asp Asn Asn Ser Ser Ser Arg Gly Asp Ala Ser Tyr Asn Ser Asp
   485 490 495
   Glu Ser Lys Asp Asn Gly Asn Gly Ser Asp Ser Lys Gly Ala Glu Asp
   500 505 510
   Asp Asp Ser Asp Ser Thr Ser Asp Thr Asn Asn Ser Asp Ser Asn Gly
   515 520 525
   Asn Gly Asn Asn Gly Asn Asp Asp Asn Asp Lys Ser Asp Ser Gly Lys
   530 535 540
   Gly Lys Ser Asp Ser Ser Asp Ser Asp Ser Ser Asp Ser Ser Asn Ser
   545 550 555 560
   Ser Asp Ser Ser Asp Ser Ser Asp Ser Asp Ser Ser Asp Ser Asn Ser
   565 570 575
   Ser Ser Asp Ser Asp Ser Ser Asp Ser Asp Ser Ser Asp Ser Ser Asp
   580 585 590
   Ser Asp Ser Ser Asp Ser Ser Asn Ser Ser Asp Ser Ser Asp Ser Ser
   595 600 605
   Asp Ser Ser Asp Ser Ser Asp Ser Ser Asp Ser Ser Asp Ser Lys Ser
   610 615 620
   Asp Ser Ser Lys Ser Glu Ser Asp Ser Ser Asp Ser Asp Ser Lys Ser
   625 630 635 640
   Asp Ser Ser Asp Ser Asn Ser Ser Asp Ser Ser Asp Asn Ser Asp Ser
   645 650 655
   Ser Asp Ser Ser Asn Ser Ser Asn Ser Ser Asp Ser Ser Asp Ser Ser
   660 665 670
   Asp Ser Ser Asp Ser Ser Ser Ser Ser Asp Ser Ser Ser Ser Ser Asp
   675 680 685
   Ser Ser Asn Ser Ser Asp Ser Ser Asp Ser Ser Asp Ser Ser Asn Ser
       690                 695                 700
   Ser Glu Ser Ser Asp Ser Ser Asp Ser Ser Asp Ser Asp Ser Ser Asp
   705                 710                 715                 720
   Ser Ser Asp Ser Ser Asn Ser Asn Ser Ser Asp Ser Asp Ser Ser Asn
                   725                 730                 735
   Ser Ser Asp Ser Ser Asp Ser Ser Asp Ser Ser Asp Ser Ser Asn Ser
               740                 745                 750
   Ser Asp Ser Ser Asp Ser Ser Asp Ser Ser Asn Ser Ser Asp Ser Ser
           755                 760                 765
   Asp Ser Ser Asp Ser Ser Asp Ser Ser Asp Ser Ser Asn Ser Ser Asp
       770                 775                 780
   Ser Asn Asp Ser Ser Asn Ser Ser Asp Ser Ser Asp Ser Ser Asn Ser
   785                 790                 795                 800
   Ser Asp Ser Ser Asn Ser Ser Asp Ser Ser Asp Ser Ser Asp Ser Ser
                   805                 810                 815
   Asp Ser Asp Ser Ser Asn Ser Ser Asp Ser Ser Asn Ser Ser Asp Ser
               820                 825                 830
   Ser Asp Ser Ser Asn Ser Ser Asp Ser Ser Asp Ser Ser Asp Ser Ser
           835                 840                 845
   Asp Ser Ser Asp Ser Asp Ser Ser Asn Arg Ser Asp Ser Ser Asn Ser
       850                 855                 860
   Ser Asp Ser Ser Asp Ser Ser Asp Ser Ser Asn Ser Ser Asp Ser Ser
   865                 870                 875                 880
   Asp Ser Ser Asp Ser Ser Asp Ser Asn Glu Ser Ser Asn Ser Ser Asp
                   885                 890                 895
   Ser Ser Asp Ser Ser Asn Ser Ser Asp Ser Asp Ser Ser Asp Ser Ser
               900                 905                 910
   Asn Ser Ser Asp Ser Ser Asp Ser Ser Asn Ser Ser Asp Ser Ser Glu
           915                 920                 925
   Ser Ser Asn Ser Ser Asp Asn Ser Asn Ser Ser Asp Ser Ser Asn Ser
       930                 935                 940
   Ser Asp Ser Ser Asp Ser Ser Asp Ser Ser Asn Ser Ser Asp Ser Ser
   945                 950                 955                 960
   Asn Ser Gly Asp Ser Ser Asn Ser Ser Asp Ser Ser Asp Ser Asn Ser
                   965                 970                 975
   Ser Asp Ser Ser Asp Ser Ser Asn Ser Ser Asp Ser Ser Asp Ser Ser
               980                 985                 990
   Asp Ser Ser Asp Ser Ser Asp Ser Ser Asp Ser Ser Asn Ser Ser Asp
           995                 1000                1005
   Ser Ser Asp Ser Ser Asp Ser Ser Asp Ser Ser Asn Ser Ser Asp Ser
      1010                1015                1020
   Ser Asn Ser Ser Asp Ser Ser Asp Ser Ser Asp Ser Ser Asp Ser Ser
   1025                1030                1035                1040
   Asp Ser Ser Asp Ser Ser Asn Ser Ser Asp Ser Ser Asp Ser Ser Asp
                  1045                1050                1055
   Ser Ser Asp Ser Ser Asp Ser Ser Gly Ser Ser Asp Ser Ser Asp Ser
              1060                1065                1070
   Ser Asp Ser Ser Asp Ser Ser Asp Ser Ser Asp Ser Ser Asp Ser Ser
          1075                1080                1085
   Asp Ser Ser Asp Ser Ser Glu Ser Ser Asp Ser Ser Asp Ser Ser Asp
      1090                1095                1100
   Ser Ser Asp Ser Ser Asp Ser Ser Asp Ser Ser Asp Ser Ser Asp Ser
   1105                1110                1115                1120
   Ser Asp Ser Ser Asp Ser Ser Asp Ser Ser Asn Ser Ser Asp Ser Ser
                  1125                1130                1135
   Asp Ser Ser Asp Ser Ser Asp Ser Ser Asp Ser Ser Asp Ser Ser Asp
              1140                1145                1150
   Ser Ser Asp Ser Ser Asp Ser Ser Asp Ser Ser Asp Ser Ser Asp Ser
          1155                1160                1165
   Ser Asp Ser Ser Asp Ser Ser Asp Ser Ser Asp Ser Ser Asp Ser Asn
      1170                1175                1180
   Glu Ser Ser Asp Ser Ser Asp Ser Ser Asp Ser Ser Asp Ser Ser Asn
   1185                1190                1195                1200
   Ser Ser Asp Ser Ser Asp Ser Ser Asp Ser Ser Asp Ser Thr Ser Asp
                  1205                1210                1215
   Ser Asn Asp Glu Ser Asp Ser Gln Ser Lys Ser Gly Asn Gly Asn Asn
              1220                1225                1230
   Asn Gly Ser Asp Ser Asp Ser Asp Ser Glu Gly Ser Asp Ser Asn His
          1235                1240                1245
   Ser Thr Ser Asp Asp
      1250